# EUROPEAN PATENT SPECIFICATION

(11) **EP 2 064 233 B1**
(45) Date of publication and mention of the grant of the patent: **18.04.2012**
(21) Application number: 07788331.2
(22) Date of filing: 09.08.2007
(51) Int. Cl.: C07K 14/415, C12N 15/82

(54) **METHOD OF INCREASING RESISTANCE AGAINST SOYBEAN RUST IN TRANSGENIC PLANTS**
VERFAHREN ZUM ERHÖHEN DER RESISTENZ GEGEN SOJABOHNENROST IN TRANSGENEN PFLANZEN
PROCÉDÉ DESTINÉ À ACCROÎTRE LA RÉSISTANCE CONTRE LA ROUILLE DU SOJA DANS DES PLANTES TRANSGÉNIQUES

(30) Priority: 10.08.2006 EP 06118753
(43) Date of publication of application: 03.06.2009
(73) Proprietor: BASF Plant Science GmbH, 67056 Ludwigshafen (DE)
(72) Inventor: FRANK, Markus, 67434 Neustadt (DE); SCHULTHEISS, Holger, 67459 Böhl-Igglheim (DE); HÖFLE, Caroline, 85395 Wolfersdorf (DE)
(86) International application number: PCT/EP2007/058268
(87) International publication number: WO 2008/017706

(56) References cited:
- WO-A-00/01722
- WO-A-00/36110
- WO-A-00/78799
- WO-A-98/04586
- BUESCHGES R ET AL: "THE BARLEY MLO GENE: A NOVEL CONTROL ELEMENT OF PLANT PATHOGEN RESISTANCE" CELL, CELL PRESS, CAMBRIDGE, NA, US, vol. 88, no. 5, 7 March 1997 (1997-03-07), pages 695-705, XP002035301 ISSN: 0092-8674
- DEVOTO ALESSANDRA ET AL: "Topology, subcellular localization, and sequence diversity of the Mlo family in plants" JOURNAL OF BIOLOGICAL CHEMISTRY, vol. 274, no. 49, 3 December 1999 (1999-12-03), pages 34993-35004, XP002459572 ISSN: 0021-9258

## Description

### BACKGROUND OF THE INVENTION

The present invention relates to a method of increasing resistance against soybean rust in transgenic plants and/or plant cells, as well as to the use of a nucleic acid molecule for the production of these plants and/or plant cells. In these plants, the content and/or the activity of at least one MLO protein is altered in comparison to the wild-type plants. Furthermore, the application relates to transgenic plants and/or plant cells having an increased resistance against soybean rust and to expression vectors comprising a sequence that is identical, homologous or complementary to a sequence encoding an functional or non-functional MLO or fragments thereof.

Plants are permanently confronted with pathogenic microbes. Plant diseases caused by various pathogens, such as viruses, bacteria and fungi, can lead to substantial crop losses in the growing of cultivated plants, with economic consequences on the one hand, but also pose a threat for the safety of human food on the other hand. Chemical fungicides have been used since the last century to control fungi diseases. Although the use of these agents led to a reduction in the extent of plant diseases, up to now it cannot be ruled out that these compounds may have harmful effects on humans, animals and the environment. In order to reduce the use of traditional pesticides to a minimum, it is therefore important to examine the natural pathogen defense of various plants to different pathogens, and to make - in addition to the classical breeding methods - systematic use of genetic engineering, such as by introducing external resistance genes, or by manipulating endogenous gene expression in plants for the production of pathogen resistant plants.

Resistance generally means the ability of a plant to prevent, or at least curtail the infestation and colonization by a harmful pathogen. Different mechanisms can be discerned in the naturally occurring resistance, with which the plants fend off colonization by phytopathogenic organisms. These specific interactions between the pathogen and the host determine the course of infection (Schopfer and Brennicke (1999) Pflanzenphysiologie, Springer Verlag, Berlin-Heidelberg, Germany).

With regard to the race specific resistance, also called host resistance, a differentiation is made between compatible and incompatible interactions. In the compatible interaction, an interaction occurs between a virulent pathogen and a susceptible plant. The pathogen survives, and may build up reproduction structures, while the host dies off. An incompatible interaction occurs on the other hand when the pathogen infects the plant but is inhibited in its growth before or after weak development of symptoms. In the latter case, the plant is resistant to the respective pathogen (Schopfer and Brennick, vide supra). In both compatible and incompatible interactions a defensive and specific reaction of the host to the pathogen occurs.

In nature, however, this host resistance is often overcome because of the rapid evolutionary development of pathogens (Neu et al. (2003) American Cytopathol. Society, MPMI 16 No. 7: 626-633). As against this, the non-host resistance offers strong, broad, and permanent protection from phytopathogens. Non-host resistance means the phenomenon that a pathogen can induce a disease in a certain plant species, but not in other plant species (Heath (2002) Can. J. Plant Pathol. 24: 259-264).

Despite this interesting characteristics, the genetic and molecular biological bases for the non-host resistance have up to now only been poorly understood. There are indications that the non-host resistance is induced by unspecific agents, and also that individual pathogen proteins induce the non-host resistance reaction (Heath (1981) Phytopathology 71: 1121-1123; Heath (2001) Physiol. Mol. Plant Pathol. 58: 53-54; Kamoun et al. (1998) Plant Cell 10: 1413-1425; Lauge et al. (2000) Plant J. 23: 735-745; Whalen et al. (1988) Proc. Natl. Acad. Sci. USA 85: 6743-6747). The phenomenon of non-host resistance might also be based on structural or chemical properties of the plant species, such as the thickness of the cuticle or the presence of inhibitory substances.

Besides resistance based on preformed physical barriers, the most effective non-host defense system of the plant is represented by the recognition of conserved molecular microbial structures, also termed PAMPs (pathogen associated molecular patterns). Recognition of PAMPs by a PAMP-receptor triggers a signaling cascade leading to the activation of a multitude of resistance mechanisms, including cell wall fortification, secretion of toxic compounds and programmed cell death. Those defense mechanisms suffice to effectively arrest the attempted assaults of most microbes. This innate immune response is thought to be an integral part of the genetically complex and durable set of non-host resistance defense mechanisms.

Only a few pathogen species appear to have evolved specific mechanisms to circumvent or to block the basic defense system of individual plant species, and, as a consequence, have become pathogens of these species. It is conceivable that targeting and manipulation of particular host proteins is a key step of this species-specific defense sabotage.

Powdery mildew is a common fungal disease of many monocotyledonous and dicotyledonous plants like beet, various cereals, cucumber, lettuce, carrot, pea, tomato, strawberry, apple, grapes etc. Powdery mildew fungi (Erysiphales) belong to the division of Ascomycota. Blumeria graminis is the fungus that causes powdery mildew in grasses. The barley powdery mildew fungus (Blumeria graminis f.sp. hordei, Bgh) is an obligate biotrophic pathogen that attacks epidermal cells of barley (*Hordeum vulgare* L.). After contact of the spore with the cuticle of the barley leaf, an appressorium is formed. The following and crucial step of fungal invasion is the penetration of the cell wall followed by the establishment of a specialized intracellular feeding structure called "haustorium" that does not destroy plasma membrane integrity.

In the monocotyledonous barley, the presence of isoforms of the family of heptahelical plasma membrane-localized MLO proteins is required for successful penetration of the host cell wall by the powdery mildew fungi. Absence of these MLO proteins, either caused by natural genetic variations or induced lesions in the respective *mlo* genes, results in failure of the fungal spores to penetrate the plant cell wall. All barley genotypes lacking a functional *mlo* gene are resistant to all known isolates of the barley powdery mildew. Additionally, the recessively inherited mlo resistance is extremely durable under field conditions. The mlo mutation has been used in most European spring barley varieties for the last 25 years.

The MLO protein is an integral plasma membrane-localized protein and contains seven hydrophobic transmembrane domains. The cytoplasmic C-terminus harbors an amphiphilic α-helix that serves as a calmodulin binding domain, which is necessary for full activity of the MLO protein. Calcium-dependent calmodulin binding to this peptide domain was shown both *in vitro* and *in vivo* and contributes about half of the susceptibility-conferring activity of mlo (Kim M.C. Nature. 2002 Mar 28;416(6879):447-51).

The gene *ror2* (*required for mlo resistance 2*) that, when mutated, suppressed mlo-mediated resistance, was found to encode a plasma membrane-resident syntaxin, a protein belonging to the superfamily of SNARE proteins. Lack of wild-type ROR2 partially compromises penetration resistance in mlo genotypes, suggesting that syntaxin activity is required for effective mlo resistance (Freialdenhoven A. Plant Cell. 1996 Jan;8(1):5-14). Both MLO and ROR2 seem to focally accumulate at sites of attempted fungal cell-wall penetration. Thus, it appears that MLO and ROR2 form a novel pathogen-triggered micro-domain at biotic stress sites (Bhat R.A. Proc. Natl. Acad. Sci. USA. 2005 Feb 22;102(8):3135-40). At these subcellular regions, interaction between MLO and the cytoplasmic calcium sensor calmodulin transiently increases during successful fungal host cell invasion (Bhat R.A., vide supra). Moreover, a direct physical interaction between MLO and ROR2 was suggested. The intensity of this interaction is drastically lowered between a subset of single amino acid substitution mlo mutant proteins and wild-type ROR2 as well as between wild-type MLO and the barley variant encoded by the barley ror2 mutant. MLO might modulate SNARE protein dependent and vesicle transport-associated processes at the plasma membrane. In conclusion, powdery mildew fungi appear to specifically corrupt MLO to modulate vesicle-associated processes at the plant cell periphery for successful pathogenesis.

This mechanism of MLO seems to be conserved in plants. The dicotyledonous plant *Arabidopsis thaliana* contains 15 homologues of barley MLO, called AtMlo1-AtMlo15. The knockout of AtMlo2 confers resistance to the powdery mildew fungi *Erysiphe chichoracearum* and *Golvinomyces orontii.* These data indicate that the powdery mildew infection mechanism is conserved between monocotyledonous and dicotyledonous plants.

It was considered to be plausible that each pathogen species evolved its own specific means to suppress and overcome general or specialized host defense mechanisms. The resistance phenotypes mediated by mlo appear to be highly specific for powdery mildew fungi (Ascomycota - Pezizomycotina - Leotiomycetes - Erysiphales). Up to present it is known from the literature that the mlo mutation does not confer resistance to any pathogen other than powdery mildew fungi.

For example, the mlo mutation does not confer any resistance to other fungi of the division of Ascomycota, which are closely related to powdery mildew fungi. No effect of mlo is observed after inoculation of barley with the take-all fungus (*Gaeumannomyces graminis:* Ascomycota - Pezizomycotina - Sordariomycetes - Sordariomycetes incertae sedis - Magnaporthaceae; Jorgensen J.H. Induced Mutations Against Plant Diseases, Proc. Symp. Wien, 1977. Wien: Int. Atomic Energy Agency, pp. 533-547). Compared with *mlo* wild-type plants, barley mlo mutants do not differ in the infection phenotype to a range of other phytopathogens, as for example barley leaf rust (*Puccinia striiformis;* Basidomycota - Urediniomycetes - Urediniomycetidae - Uredinales - Pucciniaceae) or stripe rust (*Puccinia hordei;* Basidomycota - Urediniomycetes - Urediniomycetidae - Uredinales - Pucciniaceae).

Moreover the mlo mutation is responsible for an enhanced susceptibility of barley to the hemibiotrophic rice blast fungus *Magnaporthe grisea* (Ascomycota-Pezizomycotina - Sordariomycetes - Sordariomycetes incertae sedis - Magnaporthaceae; Jarosch B. Mol. Plant-Microbe Interact. 12 (6): 508-514, 1999) and to the necrotrophic fungus *Bipolaris sorokiniana* (Ascomycota - Pezizomycotina - Dothideomycetes - Pleosporales - Pleosporaceae). In summary, beside powdery mildew fungi, no pathogen is known that is negatively influenced by the mlo mutation.

Soybean rust (SR), also known as Asian soybean rust (Basidiomycota - Urediniomycetes - Urediniomycetidae - Uredinales - Phakopsoraceae), is a disease that affects soybeans and other legumes. It is caused by two types of fungi, *Phakopsora pachyrhizi* and *Phakopsora meibomiae,* the latter being the weaker pathogen of the two.

The infection process of soybean rust starts when urediospores germinate to produce a single germ tube, form appressoria and infect always by direct, cuticular penetration. Penetration starts with the formation of an appressorial cone which is continuous with the cell wall of the penetration hypha. The penetration hypha enters the epidermal cell, transverses it and reaches the intercellular space of the mesophyll where the first septum is formed, separating the penetration hypha from the primary hypha. The first haustorium is visible between 24 and 48 hours after inoculation. Haustoria are formed in the mesophyll and epidermal cells.

Under optimal conditions it takes spores 6-7 days to mature. Then, after infection of healthy soybean plants, new spores are produced for about 10 days. These new spores can re-infect the same plant or be carried to other susceptible plants. Soybean rust causes lesions on cotyledons, stems, petioles, leaves, and pods of soybean and other host plants. The main effects on the soybean plant are destruction of photosynthetic tissue which in turn causes premature defoliation, early maturation, and severe yield reductions through reduction in the number of pods and seeds, and decreased seed weight.

Currently there is no resistance to soybean rust in any of the U.S. commercial soybean cultivars. Specific resistance to *P. pachyrhizi* is known, and four single dominant genes have been identified as Rpp1, Rpp2, Rpp3, and Rpp4. These four genes condition resistance to a limited set of rust isolates. Single gene resistance has not been durable, and the usefulness of the single genes was lost soon after the sources were identified.

The object of the present invention is to provide a method of increasing resistance against soybean rust in transgenic plants and/or plant cells. This object is achieved by the subject-matter of the main claim. The features of the other independent claims serve to solve this and further objects shown in the description. Preferred embodiments of the invention are defined by the features of the sub-claims.

### DETAILED DESCRIPTION OF THE INVENTION

Surprisingly, the inventors found an influence of an mlo mutation in the resistance reaction against soybean rust. This interaction phenotype is totally unexpected because of several reasons:
1. With the exception of the powdery mildew resistance, no other resistance phenotype has been described until now that is based on MLO influence, i.e. MLO overexpression or MLO underexpression. For the fungi *Magnaporthe grisea* and *Bipolaris sorokiniana,* even an enhanced susceptibility has been observed in mlo mutants.
2. The phenotype of mlo-barley is indistinguishable from wild type plants after infection with other rust fungi which are closely related to soybean rust. i.e. stripe rust (*Puccinia hordei,* Basidiomycota; Urediniomycetes; Urediniomycetidae; Uredinales; Pucciniaceae) and barley leaf rust (*Puccinia striiformis,* Basidiomycota; Urediniomycetes; Urediniomycetidae; Uredinales; Pucciniaceae).
3. Furthermore, MLO does not influence other pathogenic fungi which use infection processes similar to soybean rust, e.g. direct penetration of the epidermal cells and/or intercellular growth in the mesophyll. For example rice blast fungus *Magnaporthe oryzae* also penetrates directly the epidermis to grow within the leaf.
4. Finally, the powdery mildew fungus is from the division of Ascomycota, whereas the Asian soybean rust is from the division of Basidomycota.

Therefore, in a first aspect, the present invention relates to a method of increasing resistance against soybean rust in transgenic plants and/or plant cells, characterized in that the content and/or the activity of at least one MLO protein is decreased in comparison to wild type plants or plant cells, respectively; wherein the at least one endogenous MLO protein has an amino acid sequence with at least 75% homology with the amino acid sequence as depicted in SEQ ID NO: 4 or their functionally equivalent parts or fragments thereof, or wherein the at least one endogenous MLO protein has an amino acid sequence which is functionally equivalent to the amino acid sequence of a protein having at least 75% homology, with the amino acid sequence as depicted in SEQ ID NO: 4 or fragments thereof.

Within the scope of the present invention, "transgenic" plant cells (or plants) are cells into which a nucleic acid molecule has been introduced. This molecule can be a DNA / cDNA molecule or an RNA molecule, it can be double-stranded or single-stranded, and examples of such molecules are double-stranded RNA molecules or vectors, e.g. plasmids, cosmids, recombinant viruses or minichromosomes. The nucleic acid molecule can comprise sequences that derive from the species of the host cell or from another organism / species. Furthermore, those sequences can be natural or modified or synthetic.

According to the present invention, a "plant" can be any monocotyledonous or dicotyledonous plant. It is preferably a monocotyledonous or dicotyledonous agricultural, food or feed plant. Preferably, the monocotyledonous plant is selected from the group consisting of *Hordeum* (barley), *Avena* (oat), *Triticum* (wheat), *Secale* (rye), *Oryza* (rice), *Sorghum* (millet), *Zea* (com), *Panicum, Pennisetum, Setaria* and suchlike. Preferably, the dicotyledonous plant is selected from the group consisting of soybean, cotton, rapeseed, tomato, sugar beet, potato, sunflower, pea, ornamental plants, tobacco, clover (Trifolium spec.), Kudzu (Pueraria lobata), trees and legumes such as Alfalfa. Further agricultural plants can include fruit (in particular apples, pears, cherries, grapes, citrus fruits, pineapples and bananas), oil palms, tea, cocoa and coffee trees, tobacco, sisal, as well as medical plants such as rauwolfia and digitales. Particularly favored are the cereals wheat, rye, oat, barley, rice, maize and millet, sugar beet, rape, soybean, tomato, potato and tobacco. Other agricultural plants can be taken from US patent US 6,137,030. The most preferred plant is soybean.

The plant cells according to the invention include differentiated and undifferentiated plant cells including protoplasts which were produced by the method according to the invention and which have integrated the nucleic acid molecules described in the following into the plant genome, or have received these as autonomously replicating molecules.

In the scope of the present invention, the soybean rust pathogen is either *Phakopsora pachyrhizi* or *Phakopsora meibomiae.* Preferably, the pathogen is *Phakopsora pachyrhizi.*

Pathogen "resistance" means the lessening or weakening of a plant's pathogenic symptoms following an attack by a pathogen. The symptoms may be of various kinds, but preferably comprise those which directly or indirectly lead to an impairment of the quality of the plant, the size of the harvest, suitability for use as animal fodder or food for human consumption, or which hamper the sowing, cultivation, harvesting or processing of the crop.

According to the invention, the term "increased resistance" (against soybean rust) is understood to mean that the transgenic plants, or plant cells, according to the invention are less vigorously, and/or less frequently, affected by soybean rust than non-transformed wild type plants, or plant cells, which were otherwise treated in the same way (such as climate and cultivation conditions, pathogen type, etc.). According to the invention, the term "wild type" is to be understood as the respective non genetically modified parent organism. The penetration efficiency as well as the rate of papillae formation offer a possibility to quantify the reaction of the plant to the pathogen infestation (see examples). The term "increased resistance" also comprises what is known as transient pathogen resistance, i.e. the transgenic plants, or plant cells, according to the invention have an increased pathogen resistance as compared to the respective wild type only for a limited period of time.

Transient silencing or transient resistance can be advantageous because it is a valuable addition to other methods which allow the production of plants with increased soybean rust resistance, but which affect the phenotype. Plants which are produced by a method according to the invention and which show transient resistance during development of the infection do not exhibit any significant change of phenotype, and therefore methods according to the invention which give rise to transient resistance can help, along with other methods, to produce plants which are characterized by more enduring and more stable resistance, without having any negative effect upon the phenotype restriction caused by the methods.

The infestation with soybean rust is preferably reduced by at least 10 % or 20 %, more preferably by at least 30 % or 40 %, especially preferably by at least 50 % or 60 %, particularly preferably by at least 70 % or 80 %, and most preferably by at least 90 %, 95 % or 100 %, which is manifested in a reduction of the development of pathogenic symptoms.

According to the present invention, an "MLO protein" is a protein having an amino acid sequence as depicted in SEQ ID NOs: 2, 4, 7, 9, 11, 14, 16, 18, 20, 22, 24, 26, 28, 30, 32, 34, 36, 38, 40, 42, 44, 46 and 48 or a protein having a sequence which is essentially homologous to said sequences or a protein which is functionally equivalent to said MLO proteins.

The MLO proteins specified by the above-mentioned sequences originate from the following plant species: Hordeum vulgare, Glycine max, Oryza sativa, Linum usitatissimum, Triticum aestivum and Arabidopsis thaliana. However, many other species like Zea mays, Saccharum officinarum, Antirrhinum majus, Solanum tuberosum, Gossypium raimondii, Pinus taeda, Aquilegia Formosa, Aquilegia pubescens, Coffea canephora, Lactuca serriola, Lactuca sativa, Zingiber officinale, Fragaria vesca, Helianthus petiolaris, Brassica rapa, Lotus japonicus, Physcomitrella patens, Capsicum annuum, Lycopersicon esculentum and Nicotiana tabacum contain MLO proteins which are also comprised within the scope of the present invention. The following Table 1 will give an overview of the results of database researches on MLO proteins:

| Hit_ID | Organism | Accession Number | Patent Number / GI number |
|---|---|---|---|
| FastAlert_N\|EP1586645.43647 | Arabidopsis thaliana | | EP1586645 |
| FastAlert_N\|JP2005185101.15616 | Oryza sativa | | JP2005185101 |
| FastAlert_N\|US2004123343.11552 | Oryza sativa | | US2004123343 |
| FastAlert_N\|US2004214272.113197 | Zea mays | | US2004214272 |
| FastAlert_N\|US2006107345.16980 | | | US2006107345 |
| FastAlert_N\|US2006135758.8510 | | | US2006135758 |
| FastAtert_N\|US2006141495.4465 | | | US2006141495 |
| FastAlert_N\|US2006143729.3731 | | | US2006143729 |
| FastAlert_N\|US2006150283.10138 4 | | | US2006150283 |
| FastAlert_N\|US6680427.1 | | | US6680427 |
| GENBANK_EST2\|BX837230 | Arabidopsis thaliana | BX837230 | 42531313 |
| GENBANK_EST2\|CA084154 | Saccharum officinarum | CA084154 | 34937465 |
| GENBANK_EST2\|CB642264 | Oryza sativa (japonica cultivar-group) | CB642264 | 29637255 |
| GENBANK_EST3\|AJ803024 | Antirrhinum majus | AJ803024 | 51118352 |
| GENBANK_EST3\|CK276064 | Solanum tuberosum | CK276064 | 39833042 |
| GENBANK_EST31CO085008 | Gossypium raimondii | CO085008 | 48775642 |
| GENBANK_EST4\|DR092880 | Pinus taeda | DR092880 | 67551853 |
| GENBANK_EST4\|DR801464 | Zea mays | DR801464 | 71329486 |
| GENBANK_EST4\|DR917775 | Aquilegia formosa x Aquilegia pubescens | DR917775 | 71687138 |
| GENBANK_EST4\|DT755246 | Aquilegia formosa x Aquilegia pubescens | DT755246 | 74554469 |
| GENBANK_EST4\|DV705346 | Coffea canephora | DV705346 | 82485174 |
| GENBANK_EST4\|DV707424 | Coffea canephora | DV707424 | 82487252 |
| GENBANK_EST4\|DV710345 | Coffea canephora | DV710345 | 82490173 |
| GENBANK_EST4\|DW118461 | Lactuca serriola | DW118461 | 83916381 |
| GENBANK_EST5\|DY356131 | Zingiber officinale | DY356131 | 87089344 |
| GENBANK_EST5\|DY669424 | Fragaria vesca | DY669424 | 89545769 |
| GENBANK_ESTS\|DY943422 | Helianthus petiolaris | DY943422 | 90481564 |
| GENBANK_EST5\|DY945447 | Helianthus petiolaris | DY945447 | 90483589 |
| GENBANK_EST5\|DY950977 | Helianthus petiolaris | DY950977 | 90489119 |
| GENBANK_EST5\|DY963085 | Lactuca sativa | DY963085 | 90501227 |
| GENBANK_EST5\|EB425411 | Nicotiana tabacum | EB425411 | 92011825 |
| GENBANK_EST5\|EB441983 | Nicotiana tabacum | EB441983 | 92030278 |
| GENBANK_GSS2\|CL945757 | Oryza sativa (indica cultivar-group) | CL945757 | 52357766 |
| GENBANK_GSS2\|CL964365 | Oryza sativa (indica cultivar-group) | CL964365 | 52383436 |
| GENBANK_GSS2\|CL966901 | Oryza sativa (indica cultivar-group) | CL966901 | 52388451 |
| GENBANK_GSS2\|CL969027 | Oryza sativa (indica cultivar-group) | CL969027 | 52392684 |
| GENBANK_GSS2\|CL969943 | Oryza sativa (indica cultivar-group) | CL969945 | 52394507 |
| GENBANK_GSS2\|CL970460 | Oryza sativa (indica cultivar-group) | CL970460 | 52395529 |
| GENBANK_GSS2\|CL971387 | Oryza sativa (indica cultivar-group) | CL971387 | 52397377 |
| GENBANK_GSS2\|CL978285 | Oryza sativa (indica cultivar-group) | CL978285 | 52411073 |
| GENBANK_HTC\|AY103929 | Zea mays | AY103929 | 21207007 |
| GENBANK_HTC\|AY104078 | Zea mays | AY104078 | 21207156 |
| GENBANK_HTC\|AY105309 | Zea mays | AY105309 | 21208387 |
| GENBANK_HTC\|AY108340 | Zea mays | AY108340 | 21211418 |
| GENBANK_HTC\|BX819297 | Arabidopsis thaliana | BX819297 | 42469457 |
| GENBANK_HTC\|BX819596 | Arabidopsis thaliana | BX819596 | 42466898 |
| GENBANK_HTC\|BX820553 | Arabidopsis thaliana | BX820553 | 42469164 |
| GENBANK_HTC\|BX820977 | Arabidopsis thaliana | BX820977 | 42469308 |
| GENBANK_HTC\|BX826472 | Arabidopsis thaliana | BX826472 | 42459842 |
| GENBANK_HTC\|BX827067 | Arabidopsis thaliana | BX827067 | 42462173 |
| GENBANK_HTC\|BX831968 | Arabidopsis thaliana | BX831968 | 42458075 |
| GENBANK_HTC\|BX832580 | Arabidopsis thaliana | BX832580 | 2459139 |
| GENBANK_HTC\|BX841625 | Arabidopsis thaliana | BX841625 | 42406472 |
| GENBANK\|A92828 | Hordeum vulgare | A92828 | 6741365 |
| GENBANK\|A92837 | Oryza sativa | A92837 | 6741373 |
| GENBANK\|A92838 | Hordeum vulgare | A92838 | 6741374 |
| GENBANK\|AF361932 | Triticum aestivum | AF361932 | 15290588 |
| | | | 15290589 |
| GENBANK\|AF361933 | Triticum aestivum | AF361933 | 15290590 |
| | | | 15290591 |
| GENBANK\|AF369563 | Arabidopsis thaliana | AF369563 | 14091573 |
| | | | 14091574 |
| GENBANK\|AF369565 | Arabidopsis thaliana | AF369565 | 14091577 |
| | | | 14091578 |
| GENBANK\|AF369566 | Arabidopsis thaliana | AF369566 | 14091579 |
| | | | 14091580 |
| GENBANK\|AF369568 | Arabidopsis thaliana | AF369568 | 14091583 |
| | | | 14091584 |
| GENBANK\|AF369569 | Arabidopsis thaliana | AF369569 | 14091585 |
| | | | 14091586 |
| GENBANK\|AF369572 | Arabidopsis thaliana | AF369572 | 14091591 |
| | | | 14091592 |
| GENBANK\|AF369573 | Arabidopsis thaliana | AF369573 | 14091593 |
| | | | 14091594 |
| GENBANK\|AF369574 | Arabidopsis thaliana | AF369574 | 14091595 |
| | | | 14091596 |
| GENBANK\|AF384030 | Oryza sativa (indica | AF384030 | 15290604 |
| | cultivar-group) | | 15290605 |
| GENBANK\|AF384144 | Triticum aestivum | AF384144 | 14334166 |
| | | | 14334167 |
| GENBANK\|AF384145 | Triticum aestivum | AF384145 | 14334168 |
| | | | 14334169 |
| GENBANK\|AF388195 | Oryza sativa (indica | AF388195 | 14718603 |
| | cultivar-group) | | 14718604 |
| GENBANK\|AK066134 | Oryza sativa (japonica cultivar-group) | AK066134 | 32976152 |
| GENBANK\|AK072272 | Oryza sativa (japonica cultivar-group) | AK072272 | 32982295 |
| GENBANK\|AK072733 | Oryza sativa (japonica cultivar-group) | AK072733 | 32982756 |
| GENBANK\|AK098993 | Oryza sativa (japonica cultivar-group) | AK098993 | 32984202 |
| GENBANK\|AK111990 | Oryza sativa (japonica cultivar-group) | AK111990 | 37988653 |
| GENBANK\|AK121347 | Oryza sativa (japonica cultivar-group) | AK121347 | 37990970 |
| GENBANK\|AK121374 | Oryza sativa (japonica cultivar-group) | AK121374 | 37990997 |
| GENBANK\|AK221618 | Arabidopsis thaliana | AK221618 | 62320583 |
| | | | 62320584 |
| GENBANK\|AR172598 | Unknown. | AR172598 | 17912089 |
| GENBANK\|AR172601 | Unknown. | AR172601 | 17912092 |
| GENBANK\|AR172602 | Unknown. | AR172602 | 17912093 |
| GENBANK\|AR172603 | Unknown. | AR172603 | 17912094 |
| GENBANK\|AR454293 | Unknown. | AR454293 | 42687440 |
| GENBANK\|AR633457 | Unknown. | AR633457 | 59780849 |
| GENBANK\|AR633459 | Unknown. | AR633459 | 59780853 |
| GENBANK\|AR633462 | Unknown. | AR633462 | 59780859 |
| GENBANK\|AR633469 | Unknown. | AR633469 | 59780872 |
| GENBANK\|AX063294 | Triticum sp. | AX063294 | 12541084 |
| | | | 12541085 |
| GENBANK\|AX063296 | Triticum sp. | AX063296 | 12541086 |
| | | | 12541087 |
| GENBANK\|AX063298 | Triticum sp. | AX063298 | 12541088 |
| | | | 12541089 |
| GENBANK\|AX063300 | Arabidopsis thaliana | AX063300 | 12541090 |
| | | | 12541091 |
| GENBANK\|AX063302 | Arabidopsis thaliana | AX063302 | 12541092 |
| | | | 12541093 |
| GENBANK\|AX063304 | Arabidopsis thaliana | AX063304 | 12541094 |
| | | | 12541095 |
| GENBANK\|AX063306 | Arabidopsis thaliana | AX063306 | 12541096 |
| | | | 12541097 |
| GENBANK\|AX063308 | Arabidopsis thaliana | AX063308 | 12541098 |
| | | | 12541099 |
| GENBANK\|AX412295 | Arabidopsis thaliana | AX412295 | 21444753 |
| GENBANK\|AX506391 | Arabidopsis thaliana | AX506391 | 23387628 |
| GENBANK\|AX506652 | Arabidopsis thaliana | AX506652 | 23387889 |
| GENBANK\|AX506994 | Arabidopsis thaliana | AX506994 | 23388231 |
| GENBANK\|AX507333 | Arabidopsis thaliana | AX507353 | 23388590 |
| GENBANK\|AX507573 | Arabidopsis thaliana | AX507573 | 23388810 |
| GENBANK\|AX653006 | Oryza sativa | AX653006 | 29155820 |
| GENBANK\|AX653229 | Oryza sativa | AX653229 | 29156043 |
| GENBANK\|AX653497 | Oryza sativa | AX653497 | 29156311 |
| GENBANK\|AX653740 | Oryza sativa | AX653740 | 29156554 |
| GENBANK\|AX654786 | Oryza sativa | AX654786 | 29157600 |
| GENBANK\|AY029312 | Zea mays | AY029312 | 44458501 |
| | | | 44458502 |
| GENBANK\|AY029313 | Zea mays | AY029313 | 13784976 |
| | | | 13784977 |
| GENBANK\|AY029314 | Zea mays | AY029314 | 13784978 |
| | | | 13784979 |
| GENBANK\|AY029315 | Zea mays | AY029315 | 13784980 |
| | | | 13784981 |
| GENBANK\|AY029317 | Zea mays | AY029317 | 13784984 |
| | | | 13784985 |
| GENBANK\|AY029318 | Zea mays | AY029318 | 13784986 |
| | | | 13784987 |
| GENBANK\|AY029319 | Zea mays | AY029319 | 13784988 |
| | | | 13784989 |
| GENBANK\|AY029320 | Zea mays | AY029320 | 13784990 |
| | | | 13784991 |
| GENBANK\|AY054241 | Arabidopsis thaliana | AY054241 | 15809945 |
| | | | 15809946 |
| GENBANK\|AY057502 | Arabidopsis thaliana | AY057502 | 15982790 |
| | | | 15982791 |
| GENBANK\|AY072135 | Arabidopsis thaliana | AY072135 | 18175952 |
| | | | 18175953 |
| GENBANK\|AY086586 | Arabidopsis thaliana | AY086586 | 21405296 |
| | | | 21554658 |
| GENBANK\|AY113992 | Arabidopsis thaliana | AY113992 | 21280824 |
| | | | 21280825 |
| GENBANK\|AY581255 | Hordeum vulgare | AY581255 | 46405142 |
| | subsp. vulgare | | 46405143 |
| GENBANK\|AY584534 | Triticum aestivum | AY584534 | 46405854 |
| | | | 46405855 |
| GENBANK\|AY599871 | Physcomitrella | AY599871 | 47028562 |
| | patens | | 47028563 |
| GENBANY\|AY934528 | Capsicum annuum | AY934528 | 60617256 |
| | | | 60617257 |
| GENBANK\|AY967408 | Lycopersicon | AY967408 | 62208138 |
| | esculentum | | 62208139 |
| GENBANK\|AY967409 | Brassica rapa | AY967409 | 62208140 |
| | | | 62208141 |
| GENBANK\|AY967410 | Lotus japonicus | AY967410 | 62208142 |
| | | | 62208143 |
| GENBANK\|BT000434 | Arabidopsis thaliana | BT000434 | 23306367 |
| | | | 23306368 |
| GENBANK\|BT002581 | Arabidopsis thaliana | BT002581 | 27311950 |
| | | | 27311951 |
| GENBANK\|BT002918 | Arabidopsis thaliana | BT002918 | 27754573 |
| | | | 27754574 |
| GENBANK\|BT004356 | Arabidopsis thaliana | BT004356 | 28393884 |
| | | | 28393885 |
| GENBANK\|BT009442 | Triticum aestivum | BT009442 | 32128993 |
| GENBANK\|BT010322 | Arabidopsis thaliana | BT010322 | 33942040 |
| | | | 33942041 |
| GENBANK\|DW486556 | | | |
| GENBANK\|Z83834 | Hordeum vulgare subsp. vulgare | Z83834 | 1877220 1877221 |
| GENBANK\|Z95352 | Arabidopsis thaliana | Z95352 | 2765816 2765817 |
| GENESEQ_DNA\|AAA52708 | Triticum aestivum. | AAA52708 | WO200036110 |
| GENESEQ_DNA\|AAA52715 | Triticum aestivum. | AAA52715 | WO200036110 |
| GENESEQ_DNA\|AAA52718 | Triticum aestivum. | AAA52718 | W0200036110 |
| GENESEQ_DNA\|AAC44660 | Arabidopsis thaliana. | AAC44660 | EP1033405 |
| GENESEQ-DNA\|AAF24583 | Triticum sp. | AAF24583 | WO200078799 |
| GENESEQ_DNA\|AAF24584 | Triticum sp. | AAF24584 | WO200078799 |
| GENESEQ_DNA\|AAF24585 | Triticum sp. | AAF24585 | WO200078799 |
| GENESEQ_DNA\|AAF24586 | Arabidopsis thaliana. | AAF24586 | WO200078799 |
| GENESEQ_DNA\|AAF24587 | Arabidopsis thaliana. | AAF24587 | WO200078799 |
| GENESEQ-DNA\|AAF24588 | Arabidopsis thaliana. | AAF24588 | WO200078799 |
| GENESEQ_DNA\|AAF24589 | Arabidopsis thaliana. | AAF24589 | WO200078799 |
| GENESEQ_DNA\|AAF24590 | Arabidopsis thaliana. | AAF24590 | WO200078799 |
| GENESEQ_DNA\|AAS01109 | Zea mays. | AAS01109 | US6211433 |
| GENESEQ_DNA\|AAV35022 | Hordeum vulgare. | AAV35022 | WO9804586 |
| GENESEQ_DNA\|AAV35026 | Hordeum vulgare. | AAV35026 | WO980458 |
| GENESEQ_DNA\|AAV35028 | Oryza sativa. | AAV35028 | WO9804586 |
| GENESEQ_DNA\|AAV35030 | Hordeum vulgare. | AAV35030 | WO9804586 |
| GENESEQ_DNA\|AAV35031 | Arabidopsis thaliana. | AAV35031 | WO9804586 |
| GENESEQ_DNA\|AAX58270 | Zea mays. | AAX58270 | WO9923235 |
| GENESEQ_DNA\|AAX58273 | Zea mays. | AAX58273 | WO9923235 |
| GENESEQ_DNA\|AAX58274 | Zea mays. | AAX58274 | WO9923235 |
| GENESEQ_DNA\|AAX58275 | Zea mays. | AAX58275 | WO9923235 |
| GENESEQ_DNA\|AAZ30409 | Triticum sp. | AAZ30409 | WO9947552 |
| GENESEQ_DNA\|AAZ30410 | Triticum sp. | AAZ30410 | WO9947552 |
| GENESEQ_DNA\|AAZ30411 | Triticum sp. | AAZ30411 | WO9947552 |
| GENESEQ_DNA\|AAZ30412 | Arabidopsis thaliana. | AAZ30412 | WO9947552 |
| GENESEQ_DNA\|AAZ30413 | Arabidopsis thaliana. | AAZ30413 | WO9947552 |
| GENESEQ_DNA\|AAZ30414 | Arabidopsis thaliana. | AAZ30414 | WO9947552 |
| GENESEQ_DNA\|AAZ30415 | Arabidopsis thaliana. | AAZ30415 | WO9947552 |
| GENESEQ_DNA\|AAZ30416 | Arabidopsis thaliana. | AAZ30416 | WO9947552 |
| GENESEQ_DNA\|AAZ49561 | Zea mays. | AAZ49561 | WO200001722 |
| GENESEQ_DNA\|AAZ49562 | Zea mays. | AAZ49562 | WO200001722 |
| GENESEQ_DNA\|AAZ49564 | Zea mays. | AAZ49564 | WO200001722 |
| GENESEQ_DNA\|AAZ49565 | Zea mays. | AAZ49565 | WO200001722 |
| GENESEQ_DNA\|AAZ49566 | Zea mays. | AAZ49566 | WO20000172 |
| GENESEQ_DNA\|AAZ49567 | Zea mays. | AAZ49567 | WO200001722 |
| GENESEQ_DNA\|AAZ50126 | Zea mays. | AAZ50126 | WO200001721 |
| GENESEQ_DNA\|ABZ13281 | Arabidopsis thaliana. | ABZ13281 | WO200216655 |
| GENESEQ_DNA\|ABZ13542 | Arabidopsis thaliana. | ABZ13542 | WO20021665 |
| GENESEQ_DNA\|ABZ13875 | Arabidopsis thaliana. | ABZ13875 | WO200216655 |
| GENESEQ_DNA\|ABZ13884 | Arabidopsis thaliana. | ABZ13884 | WO200216655 |
| GENESEQ_DNA\|ABZ14243 | Arabidopsis thaliana. | ABZ14243 | WO200216655 |
| GENESEQ_DNA\|ABZ14463 | Arabidopsis thaliana. | ABZ14463 | WO200216655 |
| GENESEQ_DNA\|ADA67959 | Arabidopsis thaliana. | ADA67959 | WO2003000898 |
| GENESEQ_DNA\|ADA68054 | Arabidopsis thaliana. | ADA68054 | WO2003000898 |
| GENESEQ_DNA\|ADA69553 | Oryza sativa. | ADA69553 | WO2003000898 |
| GENESEQ_DNA\|ADA69776 | Oryza sativa. | ADA69776 | WO2003000898 |
| GENESEQ_DNA\|ADA70044 | Oryza sativa. | ADA70044 | WO2003000898 |
| GENESEQ_DNA\|ADA70287 | Oryza sativa. | ADA70287 | WO2003000898 |
| GENESEQ_DNA\|ADA71333 | Oryza sativa. | ADA71333 | WO2003000898 |
| GENESEQ_DNA\|ADG87617 | Arabidopsis thaliana. | ADG87617 | WO200222675 |
| GENESEQ_DNA\|ADG87618 | Arabidopsis thaliana. | ADG87618 | WO200222675 |
| GENESEQ_DNA\|ADT16339 | V iridiplantae. | ADT16339 | US2004216190 |
| GENESEQ_DNA\|ADT18635 | Viridiplantae. | ADT18635 | US2004216190 |
| GENESEQ_DNA\|ADX12455 | Unidentified. | ADX12455 | US2004034888 |
| GENESEQ-DNA\|ADX27198 | Unidentified. | ADX27198 | US2004034888 |
| GENESEQ_DNA\|ADX30090 | Unidentified. | ADX30090 | US2004034888 |
| GENESEQ_DNA\|ADX31306 | Unidentified. | ADX31306 | US2004034888 |
| GENESEQ_DNA\|ADX46115 | Unidentified. | ADX46115 | US2004034888 |
| GENESEQ_DNA\|ADX47477 | Unidentified. | ADX47477 | US2004034888 |
| GENESEQ_DNA\|ADX54605 | Unidentified. | ADX54605 | US2004034888 |
| GENESEQ_DNA\|ADX59361 | Unidentified. | ADX59361 | US2004034888 |
| GENESEQ_DNA\|ADX62039 | Unidentified. | ADX62039 | US2004034888 |
| GENESEQ_DNA\|ADX62042 | Unidentified. | ADX62042 | US2004034888 |
| GENESEQ_DNA\|ADX63313 | Unidentified. | ADX63313 | US2004034888 |
| GENESEQ_DNA\|AEH11765 | Hordeum vulgare. | AEH11765 | WO2006042145 |
| GENESEQ_DNA\|AEH11766 | Oryza sativa. | AEH11766 | WO2006042145 |
| Hordeum_vulgare_Barley_Apr03\|c 62774660hv270303 | | | |
| Hyseq_Canola_Oct02\|bn1106c258 67 | | | |
| Hyseq_Canola_Oct02\|bn1106c259 90 | | | |
| REFSEQ_NUCLEOTIDE\|NM_001 036501 | Arabidopsis thaliana | NM_001036501 | 79324986 |
| REFSEQ_NUCLEOTIDE\|NM_001 036993 | Arabidopsis thaliana | NM_00103699 3 | 79330794 |
| REFSEQ_NUCLEOTIDE\|NM_100 975 | Arabidopsis thaliana | NM_100975 | 30682023 |
| REFSEQ_NUCLEOTIDE\|NM_101 004 | Arabidopsis thaliana | NM_101004 | 18391262 |
| REFSEQ_NUCLEOTIDE\|NM_102 433 | thaliana | NM_102433 | 18396018 |
| REFSEQ_NUCLEOTIDE\|NM_ 103 440 | Arabidopsis thaliana | NM_103440 | 79358659 |
| REFSEQ_NUCLEOTIDE\|NM_104 836 | Arabidopsis thaliana | NM_104836 | 18407233 |
| REFSEQ_NUCLEOTIDE\|NM_114 398 | Arabidopsis thaliana | M_114398 | 18407954 |
| REFSEQ_NUCLEOTIDE\|NM_116 494 | thaliana | NM_116494 | 30679208 |
| REFSEQ_NUCLEOTIDE\|M_118 558 | Arabidopsis thaliana | NM_118558 | 42567096 |
| REFSEQ_NUCLEOTIDE\|NM_124 755 | Arabidopsis thaliana | NM_124755 | 30696372 |
| REFSEQ-NUCLEOTIDE\|NM_125 994 | thaliana | NM_125994 | 18425014 |
| REFSEQ_NUCLEOTIDE\|NM_127 298 | thaliana | NM_127298 | 42569101 |
| REFSEQ_NUCLEOTIDE\|NM_127 302 | thaliana | NM_127302 | 30679992 |
| REFSEQ_NUCLEOTIDE\|NM_128 925 | Arabidopsis thaliana | NM_128925 | 18403339 |
| REFSEQ_NUCLEOTIDE\|NM_129 478 | Arabidopsis thaliana | NM_129478 | 30687810 |
| REFSEQ_NUCLEOTIDE\|NM_129 974 | Arabidopsis thaliana | NM_129974 | 18406453 |
| REFSEQ_NUCLEOTIDE\|NM_190 204 | Oryza sativa (japonica cultivar-group) | NM_190204 | 34907491 |
| REFSEQ_NUCLEOTIDE\|NM_197 580 | Oryza sativa (japonica cultivar-group) | NM_197580 | 37536519 |
| REFSEQ_NUCLEOTIDE\|NM_201 957 | Arabidopsis thaliana | NM_201957 | 42571224 |
| REFSEQ_NUCLEOTIDE\|XM_464 475 | Oryza sativa (japonica cultivar-group) | XM_464475 | 50905972 |
| REFSEQ_NUCLEOTIDE\|XM_472 638 | Oryza sativa (japonica cultivar-group) | XM_472638 | 50924555 |
| REFSEQ_NUCLEOTIDE\|XM_474 381 | Oryza sativa (japonica cultivar-group) | XM_474381 | 50929706 |
| REFSEQ_NUCLEOTIDE\|XM_493 809 | Oryza sativa (japonica cultivar-group) | XM_493809 | 50948902 |
| Triticum_aestivum_Wheat_Apr03\|c 55126395 | | | |

In the scope of the present invention, the term "homologous" is used in reference to amino acid sequences or nucleic acid sequences, meaning that they share a certain degree of "homology", i.e. "identity" or "similarity", with another amino acid sequence or nucleic acid sequence, respectively.

Many algorithms exist to determine this degree of homology or similarity. Preferably the homology can be determined by means of the Lasergene software of the company DNA star Inc., Madison, Wisconsin (USA), using the CLUSTAL method (Higgins et al., 1989, Comput. Appl. Biosci., 5 (2), 151). Other programs that a skilled person can use for the comparison of sequences and that are based on algorithms are, e.g., the algorithms of Needleman and Wunsch or Smith and Waterman. Further useful programs are the Pile Aupa program (J. Mol. Evolution. (1987), 25, 351-360; Higgins et al., (1989), Cabgos, 5, 151-153) or the Gap and Best Fit program (Needleman and Wunsch, (1970), J. Mol. Biol., 48, 443-453, as well as Smith and Waterman (1981), Adv., Appl. Math., 2, 482-489) or the programs of the GCG software package of the Genetics Computer Group (575 Science Drive, Madison, Wisconsin, USA 53711). Sequence alignments can also be performed with the Clustal W program from the internet page http://www.ebi.ac.uk./clustalw or with the NCBI Blast Sequence alignment program from the internet page http://www.ncbi.nlm.nih.gov/BLAST/ or http://www.ncbi.nlm.nih.gov/blast/bl2seq/wblast2.cgi.

The skilled person can find adequate nucleic or amino acid sequences in databases which are available in the internet, e.g. http://www.ncbi.nlm.nih.gov/entrez or http://www.tigr.org. In addition to the known MLO sequences, which are disclosed in the present invention, further sequences can be found in those databases in the future and can be used in the context of the present invention. Also, the skilled person is aware of the techniques which allow him to isolate homologous sequences from other organisms. He can perform homology comparisons (via CLUSTAL, BLAST, NCBI) and then isolate the identified homologous nucleotide sequences by means of standard laboratory methods, e.g. primer design, PCR, hybridization or screening of cDNA libraries with adequate probes (cf. e.g. Sambrook and Russell (2001) Molecular Cloning: A Laboratory Manual, 3rd edition, Cold Spring Harbour Laboratory Press, Cold Spring Harbour, NY). The function of the identified proteins can then be determined.

An amino acid sequence which is "essentially homologous" (= essentially similar) to an MLO amino acid sequence means, in the scope of the present invention, that the sequence is at least 40 % or 50 %, preferably at least 55 % or 60 %, more preferably at least 65 % or 70 %, especially preferably at least 75 % or 80 %, particularly preferably at least 85 % or 90 %, and most preferably at least 92 %, 94 %, 95 %, 96 %, 97 %, 98 % or 99 % similar to the amino acid sequence of any of the MLO proteins depicted in SEQ ID NOs: 2, 4, 7, 9, 11, 14, 16, 18, 20, 22, 24, 26, 28, 30, 32, 34, 36, 38, 40, 42, 44, 46 and 48 or their functionally equivalent parts or fragments. Preferably, the homology is determined over the whole sequence length of those proteins. The same definition applies analogously to a nucleic acid sequence.

If some of the above-mentioned amino acid sequences are only partial sequences of the full length MLO protein (such as SEQ ID NO: 7), the term "essentially homologous amino acid sequence" also refers to the full length sequence or to new parts of the full length sequence which can be identified in the future.

According to the present invention, a protein which is "functionally equivalent" to an MLO protein is a protein which has the same cellular functions, the same binding properties and/or the same structural properties as any of the MLO proteins having an amino acid sequence as depicted in SEQ ID NOs: 2, 4, 7, 9, 11, 14, 16, 18, 20, 22, 24, 26, 28, 30, 32, 34, 36, 38, 40, 42, 44, 46 and 48. The cellular functions are especially meant to be the interactions of the protein with its pathogenic or physiologic binding partners, i.e. in the case of the MLO proteins their interactions with calmodulin and/or ROR2. Another possible physiologic binding partner of the MLO protein is the ATPase protein family. Any other possible interaction partner which will be discovered in the future is also meant to be included within the scope of the present invention. A functionally equivalent protein can also be a part (fragment) of said MLO proteins, e.g. a protein having amino acid deletions or additions at the N-terminus and/or at the C-terminus. It can also be a protein having one or more amino acid exchanges, insertions or deletions which do not lead to altered cellular functions, binding properties and/or structural properties.

Functional point mutations are, e.g., achieved by a conservative amino acid exchange, i.e. an amino acid is exchanged for another which has comparable physicochemical properties, such as hydrophobic, hydrophilic, positively charged, negatively charged amino acids etc. One example for a conservative amino acid exchange is the replacement of valine for alanine (or vice versa). The skilled person has to keep in mind the region where the exchange is being realized, i.e. if it is a region which is essential for the interaction of the MLO protein with its binding partners. For example, the MLO C-terminus comprises a calmodulin binding site. A sequence alignment with known MLO sequences can give an indication whether a region is essential for the binding behavior of the protein. In contrast to a conservative amino acid exchange, the skilled person will assume that the exchange of, e.g. a positively charged amino acid for a negatively charged amino acid (for example lysine - glutamic acid) will lead to a functional or structural change of the MLO protein. The same considerations apply to the generation of functional insertion or deletion mutants of mlo. The skilled person will pay attention to the issue whether the inserted or deleted amino acids or amino acid ranges are located within a region which is or is not essential for the binding properties of Mlo.

In the context of the present invention, a "fragment" of an MLO is a part of an MLO protein, wherein the original MLO protein has e.g. an amino acid sequence as depicted in SEQ ID NOs: 2, 4, 7, 9, 11, 14, 16, 18, 20, 22, 24, 26, 28, 30, 32, 34, 36, 38, 40, 42, 44, 46 and 48. Usually, the fragment lacks amino acids at the N-terminus or the C-terminus.

In the case that the MLO fragment needs to be functional, the fragment has preferably at least 40 % or 50 %, preferably at least 55 % or 60 %, more preferably at least 65 % or 70 %, especially preferably at least 75 % or 80 %, particularly preferably at least 85 % or 90 %, and most preferably at least 92 %, 94 %, 95 %, 96 %, 97 %, 98 % or 99 % of the length of the "whole" MLO protein.

Some of the methods described below, however, don't require that the nucleic acid sequence encoding a "fragment" of an MLO protein has to encode a functional protein. In those cases, the "fragment" or "part" of the nucleic acid can be as short as 20 nucleotides, in some cases even shorter. Details of the length of the (amino acid or nucleic acid) fragments will be described below.

Preferably, the MLO used for the present invention is a plant MLO selected from the group consisting of *Hordeum vulgare* (barley) MLO, *Oryza sativa* (rice) MLO, *Arabidopsis thaliana* MLO, especially preferably AtMlo1, AtMlo2, AtMlo3, AtMlo4, AtMlo5, AtMlo6, AtMlo7, AtMlo8, AtMlo9, AtMlo10, AtMlo11, AtMlo12, AtMlo13, AtMlo14 or AtMlo15, *Linum usitatissimum* (flax) MLO, *Triticum aestivum* (wheat) MLO, *Glycine max* (soy) Mlo, especially preferably GmMlol, GmMlo2, GmMlo3.1 or GmMlo3.2, or an MLO which is essentially functionally equivalent to any one of said MLO proteins.

Particularly preferably, the MLO used for the present invention is an MLO selected from the group consisting of an MLO having an amino acid sequence as depicted in any of SEQ ID NOs: 2, 4, 7, 9, 11, 14, 16, 18, 20, 22, 24, 26, 28, 30, 32, 34, 36, 38, 40, 42, 44, 46 and 48, or an MLO having an amino acid sequence which is essentially functionally equivalent to any of the MLO sequences depicted in SEQ ID NOs: 2, 4, 7, 9, 11, 14, 16, 18, 20, 22, 24, 26, 28, 30, 32, 34, 36, 38, 40, 42, 44, 46 and 48.

In the scope of the present invention, the "content" of an MLO protein is considered to be the amount of MLO protein as it can be determined for the wild type of a plant or plant cell with and/or without pathogen inoculation. Several methods which are appropriate to determine the amount of MLO in plant cells will be described in the following. All of those techniques are routine laboratory methods well-known to the skilled person. The exact protocols can be learned from any standard laboratory textbook.

The amount of MLO RNA (being an indirect indication for the protein amount) can be determined by means of a Reverse Transcriptase PCR (RT-PCR): 1. Isolation of total RNA. 2.Reverse transcription to cDNA using poly-T-Primer or random hexamer Primer. 3. PCR with Mlo specific primers using cDNA as Template. (Or One-step-RT-PCR using QuiagenKit).

Another possibility to quantify the amount of MLO RNA is the Northern Blot technique. This method is based on the transfer of electrophoretically separated RNA molecules from a gel onto an absorbent sheet, which is then immersed in a labeled probe that will hybridize to an RNA of interest to reveal its presence.

The amount of protein can be determined by means of the Western Blot (immunoblot) technique: This is a method to detect protein in a given sample of tissue homogenate or extract. It uses gel electrophoresis to separate denatured proteins by mass. The proteins are then transferred out of the gel and onto a membrane (typically nitrocellulose), where they are "probed" using antibodies specific to the protein.

Immunohistochemical staining is also a valuable tool for detecting specific antigens in tissues. In order to perform the standard staining procedure, first the tissue section has to be deparaffinized and then rehydrated before applying the primary antibody. Enzyme-conjugated secondary antibodies are then applied and the specific staining can be visualized after adding the enzyme-specific substrate. Occasionally, when weak or no staining is observed, an antigen "unmasking" by enzyme digestion, may be required.

The "activity" of an MLO protein means its capacity to perform its cellular function, especially the interaction with its physiologic or pathogenic binding partners, most especially with ROR2 and/or calmodulin. The interaction of MLO with Calmodulin and/or Ror2 can be detected using standard protein-protein interaction methods. Calmodulin was shown to interact with MLO in the yeast-two-hybrid (split-ubiquitin) system (see Kim M.C. Journal of Biological Chemistry. 277(22):19304-19314, 2002 May 31; and Kim M.C. Nature. 416(6879):447-450, 2002 Mar 28). Additionally the interaction was shown in a GST-pulldown experiment (see Kim M.C. Nature. 416(6879):447-450, 2002 Mar 28). The interaction of the ROR2 syntaxin and MLO was shown by FRET (see Bhat R.A. Proceedings of the National Academy of Sciences of the United States of America. 102(8):3135-3140, 2005 Feb 22).

Other methods to detect protein-protein interactions can also be applied to MLO and its binding partners. The split-ubiquitin system is an appropriate system to find new interaction partners (as described in Kim et al. 2002, vide supra), but also a transient transformation based (high throughput) FRET or Bi-Fluorescence Complementation (BiFC) screen can be used. In both cases the MLO bait is fused to a fluorescent protein (YFP f.l. for FRET and the N or C terminal half of YFP for BiFC). For the prey, a cDNA library is fused to CFP (FRET) or the complementary half of YFP (BiFC). Bait and prey are co-expressed transiently in epidermis cells or protoplasts. Flourecence is measured by CLSM or fluorescence photometer.

Also disclosed is that when the content and/or the activity of an MLO protein is altered within a plant or a plant cell, it can be either decreased or increased in comparison to the wild type. The increase of the content of MLO can be achieved by an increase of the endogenous MLO amount (i.e. the MLO that is or by the introduction of an additional amount of MLO into the plants or plant cells. The decrease of the MLO content in the plants or plant cells according to the invention is generally achieved by the decrease of the endogenous MLO amount. Accordingly, the increase of the MLO activity can be achieved by an increase of the endogenous MLO activity and/or the introduction of an additional amount of functional MLO. A decrease of the MLO activity can be achieved by the decrease of the activity of the endogenous MLO. Likewise, a decrease of the MLO activity can also mean that the activity of the endogenous MLO is unmodified, but its interaction with physiologic or pathogenic binding partners is inhibited, e.g. via the expression of a non-functional MLO or of an anti-MLO antibody or an MLO inhibitor. In other words, the interaction of an MLO protein with its binding partners is essentially suppressed and/or substantially prevented. Accordingly, a preferred embodiment of the present invention is directed to a method of increasing resistance against soybean rust in transgenic plants and/or plant cells, characterized in that the content and/or the activity of at least one endogenous MLO protein is decreased in comparison to the wild type.

The decrease of the Mlo content and/or activity in a transgenic plant or plant cell according to the invention is preferably at least 10 %, 15%, 20 % or 25 %, more preferably at least 30 %, 35 %, 40 % or 45 %, especially preferably at least 50 %, 55 %, 60 % or 65 %, particularly preferably at least 70 %, 75 %, 80 % or 85 %, and most preferably at least 90 %, 92 %, 94 %, 95 %, 96 %, 97 %, 98 %, 99 % or 100 %.

The decrease of the content and/or the activity of an MLO protein can be achieved by different means. One preferred method is the transfer of at least one nucleic acid molecule comprising at least one sequence which is identical, homologous or complementary to the sequence(s) encoding the endogenous MLO or fragments thereof to the plant cells.

According to the present invention, a "nucleic acid molecule" can be a DNA molecule, e.g. comprising a genomic sequence or a cDNA sequence, or an RNA molecule. The molecule can be double-stranded or single-stranded. Examples of such molecules are double-stranded RNA molecules or vectors, e.g. plasmids, cosmids, recombinant viruses or minichromosomes. The nucleic acid molecule can comprise sequences that derive from the species of the host cell or from another organism / species. Furthermore, those sequences can be natural or modified or synthetic.

The "transfer" of a nucleic acid molecule into a plant or plant cell can be performed by different methods. Preferably, the transfer occurs via transformation, transfection (stable or transient), injection, biolistic methods and/or electroporation, especially when the nucleic acid molecule is DNA. The DNA can e.g. be present in the form of a vector or a linear "promoter-gene-terminator construct" without a common vector backbone. When the molecule s a double stranded RNA, the transfer can be performed by means of biolistic methods. The skilled person is familiar with those methods and will be easily able to identify the best transfer method for his actual requirements. Some of the transfer methods will be described in detail (see below).

A nucleic acid sequence which is "identical" to a sequence encoding an MLO protein (or fragments thereof) is meant to be identical over a certain region, preferably over the whole region of one of the sequences.

A nucleic acid sequence which is "homologous" to a sequence encoding an MLO protein (or fragments thereof) means, in the scope of the present invention, that the sequence is at least 40 % or 50 %, preferably at least 55% or 60 %, more preferably at least 65 % or 70 %, especially preferably at least 75 % or 80 %, particularly preferably at least 85 % or 90 %, and most preferably at least 92 %, 94 %, 95 %, 96 %, 97 %, 98 % or 99 % similar to a nucleic acid sequence encoding any of the MLO proteins depicted in SEQ ID NOs: 2, 4, 7, 9, 11, 14, 16, 18, 20, 22, 24, 26, 28, 30, 32, 34, 36, 38, 40, 42, 44, 46 and 48 or their functionally equivalent parts or fragments. Preferably, the homology is determined over the whole sequence length of the nucleic acid molecules. The MLO encoding nucleic acid sequences can be easily deduced from said amino acid sequences by any skilled person. Some of the respective coding nucleic acid sequences are depicted in SEQ ID NOs: 1, 3, 5,6, 8, 10, 12, 13, 15, 17, 19, 21, 23, 25, 27, 29, 31, 33, 35, 37, 39, 41, 43, 45 and 47. Those sequences are of course not limiting. The skilled person will also adapt the nucleic acid sequences to the preferred codon usage of the host cell.

The following Table 2 gives an overview of the above-mentioned amino acid and nucleic acid sequences:

| **SEQ ID NO:** | **organism** | **name** | **nucleic acid / amino acid** | **comment** | **NCBI accession number** |
|---|---|---|---|---|---|
| **1** | Hordeum vulgare | HvMlo | na | wt, full length | |
| **2** | | HvMlo | aa | wt, full length | P93766 |
| **3** | Glycine max | GmMlo 1 | na | full length | |
| **4** | | GmlMlo 1 | aa | full length | |
| **5** | | GmMlo2 | na | genomic, partial | |
| **6** | | GmMlo2 | na | EST, partial | |
| **7** | | GmMlo2 | aa | EST | |
| **8** | | GmMlo3.1 | na | full length | |
| **9** | | GmMlo3.1 | aa | full length | |
| **10** | | GmMlo3.2 | na | EST | |
| **11** | | GmMlo3.2 | aa | predicted | |
| **12** | Oryza sativa | OsMlo | na | partial | |
| **13** | | OsMlo | na | genomic | |
| **14** | | OsMlo | aa | | 049914 |
| **15** | Linum | LuMlo | na | | |
| **16** | usitatissimum | LuMlo | aa | | CAA06487 |
| **17** | Triticum aestivum | TaMlo | na | | |
| **18** | | TaMlo | aa | | AAS93630 |
| **19** | Arabidopsis | AtMlo1 | na | | |
| **20** | thaliana | AtMlo1 | aa | | 049621 |
| **21** | | AtMlo2 | na | | |
| **22** | | AtMlo2 | aa | | Q9SXB6 |
| **23** | | AtMlo3 | na | | |
| **24** | | AtMlo3 | aa | | Q94KB9 |
| **25** | | AtMlo4 | na | | |
| **26** | | AtMlo4 | aa | | 023693 |
| **27** | | AtMlo5 | na | | |
| **28** | | AtMlo5 | aa | | 022815 |
| **29** | | AtMlo6 | na | | |
| **30** | | AtMlo6 | aa | | Q94KB7 |
| **31** | | AtMlo7 | na | | |
| **32** | | AtMlo7 | aa | | 022752 |
| **33** | | AtMlo8 | na | | |
| **34** | | AtMlo8 | aa | | 022757 |
| **35** | | AtMlo9 | na | | |
| **36** | | AtMlo9 | aa | | Q94KB4 |
| **37** | | AtMlo10 | na | | |
| **38** | | AtMlo10 | aa | | Q9FKY5 |
| **39** | | AtMlo11 | na | | |
| **40** | | AtMlo11 | aa | | Q9FI00 |
| **41** | | AtMlo12 | na | | |
| **42** | | AtMlo12 | aa | | 080961 |
| **43** | | AtMlo13 | na | | |
| **44** | | AtMlo13 | aa | | Q94KB2 |
| **45** | | AtMlo14 | na | | |
| **46** | | AtMlo14 | aa | | Q94KB1 |
| **47** | | AtMlo15 | na | | |
| **48** | | AtMlo15 | aa | | NP_973686 |

According to one preferred embodiment of the invention, a part of the transferred nucleic acid molecule is at least 50 %, more preferably at least 60 %, especially preferably at least 70 %, particularly preferably at least 80 %, also particularly preferably at least 90 %, and most preferably at least 95 % homologous to the sequence encoding the endogenous MLO or fragments thereof.

A nucleic acid sequence which is "complementary" to a sequence encoding an MLO protein (or fragments thereof) means, in the scope of the present invention, that the sequence can hybridize under stringent conditions with a nucleic acid sequence encoding an MLO protein (or fragments thereof) due to hydrogen bonds between complementary bases. This hybridization has to be specific. The person skilled in the art knows that two sequences do not need to have a 100 % complementarity in order to hybridize with one another. Herein, a "complementary" nucleic acid sequence is at least 40 % or 50 %, preferably at least 55 % or 60 %, more preferably at least 65 % or 70 %, especially preferably at least 75 % or 80 %, particularly preferably at least 85 % or 90 %, and most preferably at least 92 %, 94 %, 95 %, 96 %, 97 %, 98 % or 99 % complementary to a nucleic acid sequence encoding any of the MLO proteins depicted in SEQ ID NOs: 2, 4, 7, 9, 11, 14, 16, 18, 20, 22, 24, 26, 28, 30, 32, 34, 36, 38, 40, 42, 44, 46 and 48 or their functionally equivalent parts or fragments.

In the context of this invention the term "hybridization under stringent conditions" means that the hybridization is performed *in vitro* under conditions stringent enough to ensure a specific hybridization. Stringent *in vitro* hybridization conditions are known to the person skilled in the art, and can be found in the literature (e.g. Sambrook and Russell (2001) Molecular Cloning: A Laboratory Manual, 3rd edition, Cold Spring Harbour Laboratory Press, Cold Spring Harbour, NY). The term "specific hybridization" refers to the fact that a molecule preferably binds to a certain nucleic acid sequence, the target sequence, under stringent conditions, if the target sequence is part of a complex mixture of, for example, DNA or RNA molecules, but does not bind, or at least to a considerably lesser degree, to other sequences.

Stringent conditions depend on the conditions. Longer sequences hybridize specifically at higher temperatures. In general, stringent conditions are selected so that the hybridization temperature is approximately 5°C below the melting point (Tₘ) for the specific sequence at a defined ionic strength and a defined pH value. Tₘ is the temperature (at a defined pH value, a defined ionic strength and a defined nucleic acid concentration) at which 50% of the molecules complementary to the target sequence hybridize to the target sequence in the equilibrium state. Typically, stringent conditions are those in which the salt concentration is at least about 0.01 to 1.0 M of sodium ion concentration (or the concentration of another salt) at a pH of between 7.0 and 8.3 and the temperature is at least 30°C for short molecules (i.e. for example 10 to 50 nucleotides). Furthermore, stringent conditions can comprise the addition of agents, such as formamide, which destabilizes the hybrids. A preferred, non-limiting example for stringent hybridization conditions are hybridizations in 6 x sodium chloride/sodium citrate (SSC) at about 45°C, followed by one or more washing steps in 0.2 x SSC, 0.1 % SDS at 50 to 65°C. The temperature ranges, for example, under standard hybridization conditions depending on the type of nucleic acid, between 42°C and 58°C in an aqueous buffer at a concentration of 0.1 to 5 x SSC (pH 7.2).

If an organic solvent, e.g. 50% formamide, is present in the above-mentioned buffer, the temperature under standard conditions is about 42°C. Preferably, the hybridization conditions for DNA:DNA hybrids are for example 0.1 x SSC and 20°C to 45°C, preferably 30°C to 45°C. Preferably, the hybridization conditions for DNA:RNA hybrids are for example 0.1 x SSC and 30°C to 55°C, preferably between 45°C to 55°C. The hybridization temperatures mentioned above are determined for example for a nucleic acid having a length of about 100 base pairs and a G/C content of 50% in the absence of formamide. The person skilled in the art knows how the required hybridization conditions can be determined using the above mentioned, or the following, textbooks: Current Protocols in Molecular Biology, John Wiley & Sons, N.Y. (1989), Hames und Higgins (publisher) 1985, Nucleic Acids Hybridization: A Practical Approach, IRL Press at Oxford University Press, Oxford; Brown (publisher) 1991, Essential Molecular Biology: A Practical Approach, IRL Press at Oxford University Press, Oxford.

Typical hybridization and wash buffers have, e.g. the following composition (this example is not limiting):

| | |
|---|---|
| *Prehybridization solution:* | 0.5% SDS |
| | 5x SSC |
| | 50 mM NaPO₄, pH 6.8 |
| | 0.1 % Na pyrophosphate |
| | 5x Denhardt's solution |
| | 100 µg/ml salmon sperm |
| | |
| *Hybridisation solution:* | Prehybridization solution |
| | 1x10⁶ cpm/ml probe (5-10 min 95°C) |
| | |
| *20x SSC:* | 3 M NaCl |
| | 0,3 M sodium citrate |
| | ad pH 7 with HCI |
| 50*x Denhardt's reagent:* | 5 g Ficoll |
| | 5 g polyvinyl pyrrolidone |
| | 5g bovine serum albumine |
| | ad 500 ml A. dest. |

A typical method for hybridization is as follows (this example is not limiting):
- *Optional:*: wash blot 30 min in 1 x SSC/ 0.1% SDS at 65°C
- *Prehybridization:*: at least 2 hrs at 50-55°C
- *Hybridisation:*: over night at 55-60°C

| | | | | | | | |
|---|---|---|---|---|---|---|---|
| *Wash:* | 05 min | | 2x SSC/0.1% SDS | | | | hybridization |
| temp. | | | | | | | |
| | 30 min | | 2x SSC/ 0.1% SDS | | | hybridization temp. | |
| | 30 min | | 1x SSC/ 0.1% SDS | | | hybridization temp. | |
| | 45 min | | 0.2x SSC/ 0.1% SDS | | | 65°C | |
| | 5 min | 0.1x SSC | | room temperature | | | |

The person skilled in the art knows that the specified solutions and the protocol shown can or must be modified dependent upon the application.

Also disclosed is that a part of the transferred nucleic acid molecule is at least 50 %, preferably at least 60 %, more preferably at least 70 %, especially preferably at least 80 %, particularly preferably at least 90 %, and most preferably at least 95 % complementary to the sequence encoding the endogenous MLO or fragments thereof.

Also disclosed is that the part of the transferred nucleic acid molecule which is identical, homologous or complementary to the sequences encoding the endogenous MLO or fragments thereof comprises 20 to 1000 nucleotides, preferably 20 to 750 nucleotides, more preferably 20 to 500 nucleotides, especially preferably 20 to 250 nucleotides, particularly preferably 20 to 150 nucleotides, also particularly preferably 20 to 100 nucleotides and most preferably about 20 to 50 nucleotides.

The decrease of the content and/or the activity of the at least one endogenous MLO can be achieved by different methods, e.g. by RNA interference (RNAi), an antisense construct, a co-suppression construct, post-transcriptional gene silencing (PTGS), a ribonuclease P construct, homologous recombination, a ribozyme construct or virus induced gene silencing (VIGS). The methods will be explained in the following.

An increased resistance against soybean rust in transgenic plants or plant cells having a decreased MLO content / activity can be achieved, e.g., by the process of "silencing". During this process, a nucleic acid which encodes at least one MLO or fragments thereof and/or a nucleic acid which is complementary thereto is transferred to a plant cell. In order to ensure that the plant cell is transgenic for the transferred nucleic acid, usually the nucleic acid to be transferred is part of a vector, e.g. a plasmid, which is able to stably replicate within the cell or which assures the integration of the transferred nucleic acid into the plant genome.

Preferably the silencing of mlo is realized by means of the RNAi method. In this method, a vector is transferred to a plant cell which comprises the following elements in 5'-3' orientation: a promoter sequence which is functionally active in plants, operatively linked thereto an antisense sequence which is complementary to the sequence encoding the at least one MLO or fragments thereof (or a homologous of this antisense sequence), wherein the sequence has 3' exon sequences at its 3' end which are recognizable by the spliceosome, operatively linked thereto an intron, operatively linked thereto a sense sequence which is identical or homologous to the sequence encoding the at least one MLO or fragments thereof, wherein the sequence has 5' exon sequences at its 5' end which are recognizable by the spliceosome, and optionally, operatively linked thereto a termination sequence which is functionally active in plants. Of course, the position of the sense and the antisense sequence can be interchanged. It is obvious to the skilled person that in this case, the respective 3' splicing site and 5' splicing site need to be adapted.

When those vectors are stably transferred to plant cells, the transcription leads to the generation of a pre-mRNA containing a first exon comprising the antisense sequence, an intron, and a second exon comprising the sense sequence. The intron is then removed via the splicing process, which results in a continuous RNA molecule having regions which are complementary to each other. This molecule will develop a double stranded structure (Smith et al., 2000, Nature, 407:319-320).

Those double stranded RNS molecules are able to silence specifically the mlo mRNA via induction of the PTGS (post transcriptional gene silencing) system. As a consequence, the MLO protein cannot be expressed anymore. The choice of the antisense and sense sequences allows to determine which kind of mlo should be suppressed. The skilled person is able to identify the sequences which are characteristic for the protein. He further knows that a multitude of MLO proteins can be silenced when many corresponding characteristic sequences are used.

This RNAi method can comprise the following steps:
a) Construction of a vector comprising the following nucleic acid sequences in 5'-3' orientation:
   - a promoter sequence which is functionally active in plants,
   - operatively linked thereto an antisense sequence which is complementary to the sequence encoding the at least one MLO or fragments thereof, or a homologous of this antisense sequence, wherein the sequence has 3' exon sequences at its 3' end which are recognizable by the spliceosome,
   - operatively linked thereto an intron,
   - operatively linked thereto a sense sequence which is identical or homologous to the sequence encoding the at least one MLO or fragments thereof, wherein the sequence has 5' exon sequences at its 5' end which are recognizable by the spliceosome,
   - optionally, operatively linked thereto a termination sequence which is functionally active in plants,
b) transfer of the vector from step a) to a plant cell and optionally integration into the plant genome.

The skilled person knows which vectors to choose for the implementation of the RNAi method or the PTGS method. Those vectors can e.g. be constructed in a way to allow the sense and antisense sequences to be transcribed from any appropriate promoter, to hybridize within the cell and to induce the PTGS system (Tuschl, 2002, Nat. Biotechnol. 20, 446-448; Miyagishi et al., 2002, Nat. Biotechnol., 20, 497-500; Lee et al., 2002, Nat. Biotechnol., 20, 500-505). Other vectors combine the sense and antisense sequence by means of a "loop" sequence and are transcribed from any appropriate promoter. The back-folding of the loop allows the sense and antisense sequence to hybridize, to form double stranded RNA and to induce the PTGS system (Tuschl, 2002, vide supra; Paul et al., 2002, Nat. Biotechnol., 20, 505-508; Paddison P.J. Genes Dev. 2002 Apr 15;16(8):948-58; Brummelkamp et al., 2002, Science, 296, 550-553).

In another RNAi method, pre- synthesized double stranded RNA molecules comprising the above-mentioned sense and antisense sequences are transferred directly into the plant cells, e.g. by means of biolistic methods. Accordingly, this RNAi method can comprise the following steps:
a) Construction of a double stranded RNA molecule having a length of 15 to 100 nucleotides, preferably of 20 to 75 nucleotides, more preferably of 20 to 50 nucleotides, especially preferably of 20 to 40 nucleotides, particularly preferably of 20 to 30 nucleotides and most preferably of 20 to 25 or 21, 22 or 23 nucleotides, comprising a nucleic acid sequence having a sense strand which is identical or homologous to a fragment of the sequence(s) encoding the at least one endogenous MLO,
b) transfer of the molecule from step a) to a plant cell.

Also disclosed is that the vectors which are used for the transfer of nucleic acids comprise, in 5'-3' orientation: a promoter sequence, a sense sequence which is identical or homologous to the sequence encoding the at least one endogenous MLO or fragments thereof, wherein the sequence has self-complementary regions, and optionally a termination sequence. The transcription of those vectors in the plant cell results in the generation of RNA molecules which contain sequence regions being able to hybridize with themselves. This can lead to the formation of double stranded RNA molecules inside of the cell, which can induce the PTGS system and which results in the specific degradation of mlo mRNA. This method for the silencing of plant proteins, also called co-suppression, requires that the mRNA of the MLO to be suppressed contains regions which are complementary to each other. Such regions can be identified by the skilled person by a visual inspection of the respective coding DNA sequence or by means of sequence programs like DNAStar from DNAStar Inc., Madison, USA.

This co-suppression method can comprise the following steps:
a) Construction of a vector comprising the following nucleic acid sequences in 5'-3' orientation:
   - a promoter sequence which is functionally active in plants,
   - operatively linked thereto a sense sequence which is identical or homologous to the sequence encoding the at least one endogenous MLO or fragments thereof, wherein the sequence has self-complementary regions,
   - optionally, operatively linked thereto a termination sequence which is functionally active in plants,
b) transfer of the vector from step a) to a plant cell and optionally integration into the plant genome.

Also disclosed is that the vectors which are used for the transfer of the nucleic acids comprise, in 5'-3' orientation: a promoter sequence, operatively linked thereto an antisense sequence which is complementary to the sequence encoding the at least one endogenous MLO or fragments thereof (or a homologous of this antisense sequence), and optionally a termination sequence. The transcription of those vectors in plant cells results in the generation of an RNA molecule, the sequence of which is complementary to the mRNA encoding an MLO or parts thereof. Hybridization of the antisense sequence with the endogenous mRNA sequences of mlo *in vivo* can then lead to the suppression of the mlo expression in plant cells.

This antisense method can comprise the following steps:
a) construction of a vector comprising the following nucleic acid sequences in 5'-3' orientation:
   - a promoter sequence which is functionally active in plants,
   - operatively linked thereto an antisense sequence which is complementary to the sequence encoding the at least one endogenous MLO or fragments thereof, or a homologous of this antisense sequence,
   - optionally, operatively linked thereto a termination sequence which is functionally active in plants,
b) transfer of the vector from step a) to a plant cell and optionally integration into the plant genome.

Also disclosed is that the vectors which are used for the transfer of the nucleic acids comprise in 5'-3' orientation: a promoter sequence, operatively linked thereto a DNA sequence encoding a ribozyme which specifically recognizes the mRNA of the at least one mlo, and optionally a termination sequence. It is well known to the skilled person how to produce ribozymes which have an endonuclease activity which is directed against a specific mRNA. In detail, this method is e.g. described in Steinecke P et al. (EMBO J. 1992 Apr;11(4):1525-30). In the context of the present invention, the term "ribozyme" also comprises those RNA sequences which include in addition to the ribozyme itself leader sequences which are complementary to the mRNA of the mlo or fragments thereof and which are therefore able to guide the mRNA specific ribozyme even more efficiently to the mRNA substrate.

This ribozyme method can comprise the following steps:
a) construction of a vector comprising the following nucleic acid sequences in 5'-3' orientation:
   - a promoter sequence which is functionally active in plants,
   - operatively linked thereto a DNA sequence encoding a ribozyme which specifically recognizes the mRNA of the at least one endogenous mlo,
   - optionally, operatively linked thereto a termination sequence which is functionally active in plants,
b) transfer of the vector from step a) to a plant cell and optionally integration into the plant genome.

Another alternative for increasing the resistance against soybean rust in transgenic plants or plant cells is the transfer of nucleic acids via vectors which comprise in 5'-3' orientation: a promoter sequence, operatively linked thereto a DNA antisense sequence which is complementary to the sequence encoding the mRNA of the at least one mlo or fragments thereof, operatively linked thereto a sequence encoding a ribonuclease P (RNAse P), and optionally, operatively liked thereto a termination sequence. The transcription of these vectors in the cell results in RNA molecules which include a leading sequence (the antisense sequence), which guides the RNAse P to the mlo mRNA, whereupon the degradation of the mRNA by the RNAse P occurs (see US Patent 5,168,053). Preferably, the leading sequence comprises 10 to 15 nucleotides which are complementary to the DNA sequence of the mlo, and one 3'-NCCA nucleotide sequence, wherein the N is preferably a purine. The transcripts of the external leading sequence bind to the target mRNA via formation of base pairs, which allows the degradation of the mRNA by the RNAse P at the 5' nucleotide of the paired region. This degraded mRNA cannot be translated into a functional protein.

This RNAse P method can comprise the following steps:
a) construction of a vector comprising the following nucleic acid sequences in 5'-3' orientation:
   - a promoter sequence which is functionally active in plants,
   - operatively linked thereto a DNA sequence which is complementary to the sequence encoding the mRNA of the at least one MLO or fragments thereof,
   - operatively linked thereto a sequence encoding a ribonuclease P,
   - optionally, operatively linked thereto a termination sequence which is "functionally active in plants,
b) transfer of the vector from step a) to a plant cell and optionally integration into the plant genome.

Furthermore, vectors can be used for the method according to the invention, which comprise the following sequence in 5'-3' orientation: a DNA sequence which is identical or homologous to the sequence encoding the 5' end of the at least one endogenous MLO, a promoter sequence, operatively linked thereto a DNA sequence encoding a resistance or reporter gene, optionally a termination sequence, and a DNA sequence which is identical or homologous to the sequence encoding the 3' end of the at least one endogenous MLO. Those vectors can be used in order to induce a specific knock-out of the mlo of interest by means of homologous recombination. The sequence of the resistance or reporter gene is inserted in those plant cells in which the homologous recombination has occurred, so that no functional mlo mRNA can be produced in the cell. The plant cells in which the recombination has occurred can be identified by selection of the resistance or reporter gene. The skilled person knows how to produce those vectors for genetic knock-out via homologous recombination, which elements they have to comprise (promoters, enhancers, flanking sequences) and how to identify the respective plant cells. Usually, antibiotic resistance genes are used as resistance genes (Amp, Kan etc.). Of course, all other possible resistance genes can be used which allow the selection of the cells in which the recombination has occurred. In addition to the classical resistance genes, other reporter genes can be used for the detection and/or selection of the plants and plant cells in which the homologous recombination has occurred, such as GUS, GFP etc.

This homologous recombination method can comprise the following steps:
a) construction of a vector comprising the following nucleic acid sequences in 5'-3' orientation:
   - a DNA sequence which is identical or homologous to the sequence encoding the 5' end of the at least one endogenous MLO,
   - a promoter sequence which is functionally active in plants,
   - operatively linked thereto a DNA sequence encoding a resistance or reporter gene,
   - optionally, operatively linked thereto a termination sequence which is functionally active in plants,
   - a DNA sequence which is identical or homologous to the sequence encoding the 3' end of the at least one endogenous MLO,
b) transfer of the vector from step a) to a plant cell and optionally integration into the plant genome.

According to the present invention, nucleic acid sequences encoding an MLO or fragments thereof can be the complete coding DNA sequence for MLO or the complete mRNA sequence or fragments thereof. Since some of the above-mentioned methods for the production of transgenic plants, being directed to a significant reduction of mlo expression, are based on a specific hybridization of an endogenous mlo mRNA and the sequences which are generated during the transcription of the above-mentioned vectors (e.g. the antisense strategy), the skilled person knows that the transferred nucleic acids do not necessarily have to contain the complete sequence encoding the MLO, irrespective if it is a sense or an antisense sequence. In fact, relatively short regions of the sequences encoding the MLO are sufficient for a specific hybridization and an efficient silencing.

Those sequences of the vectors which correspond to the sequence regions of the mlo mRNA and which are transcribed to generate double stranded RNA molecules can have a length of about 25 nucleotides, preferably 21, 22 or 23 nucleotides. The sequences which are transferred for the antisense strategy usually comprise between 20 and 1000 nucleotides, preferably between 20 and 800 nucleotides, more preferably between 400 and 800 nucleotides, especially preferably between 500 and 750 nucleotides. But it is also possible to use sequences comprising between 20 and 500, between 20 and 300, between 20 and 150, between 20 and 100 or between 20 and 50 nucleotides. The skilled person knows that for the RNAi or the PTGS method, the sense and antisense RNAs which are used for the generation of double stranded RNA molecules can also comprise about 21, 22 or 23 nucleotides with a characteristic 3' overhang (Tuschl, 2002, Nat. Biotechnol. 20, 446-448).

When nucleic acids are transferred to plant cells, and the transcription of those sequences in the cell results in sequences which are complementary to the mlo mRNA (e.g. using the antisense strategy), those transferred sequences do not need to be 100 % complementary to the mRNA. It will be sufficient if the sequences are at least 50 %, preferably at least 60 %, more preferably at least 70 %, especially preferably at least 80 %, particularly at least 90 % and most preferably at least 95 % complementary. The differences can be the result of insertions, deletions and/or substitutions, preferably substitutions. The skilled person knows however that with decreasing complementarity, the probability to silence several mlo mRNAs will increase.

In general, only those complementary sequences can be used for the present invention which are able to specifically hybridize with regions of the mlo mRNA. Sequences which hybridize *in vivo* with RNA regions of proteins other than MLO and which cause their silencing are not adequate for the present invention. Depending on the selected sequence and on the degree of complementarity, a multitude of MLO proteins or only a few MLO proteins will be silenced. It is also possible that the expression of only one specific mlo is inhibited. The length of complementary sequences is preferably between 20 and 1000 nucleotides, more preferably between 20 and 750 nucleotides, especially between 20 and 500 nucleotides, particularly preferably between 20 and 300 nucleotides and most preferably between 20 and 150,20 and 75 or 20 and 50 nucleotides. It is also possible that the sequences only comprise about 20 or 25 nucleotides.

Some of the above-mentioned methods can also be performed with sequences which are not part of the coding region of the mlo mRNA or which are not complementary thereto. It can e.g. be sufficient that those sequences derive from the 5' or 3' untranslated region, if those regulatory sequences are characteristic for the mRNA of the respective MLO. Those sequences can be used especially when the silencing is induced via double stranded RNA constructs or when the translation of an mRNA is inhibited by antisense constructs. Therefore, in the context of the invention, the term "mRNA" not only comprises coding regions , but also regulatory regions which occur in the pre-mRNA or in the mature mRNA and which are characteristic for the mlo mRNA. The same applies for the DNA sequence, e.g. for untranscribed sequences, promoter sequences, upstream activating sequences, introns etc.

If vectors are used whose transcription results in the generation of RNA molecules which have a leading sequence and an RNAse P, the leading sequence has to be sufficiently complementary in order to specifically recognize the mlo mRNA. The conditions allow the skilled person to choose which part of the mlo mRNA is recognized by the leading sequence. Preferably the leading sequences comprise about 20 nucleotides, they should however not be shorter than about 15 nucleotides. Having a 100 % complementarity of the leading sequence, 12 nucleotides can be sufficient. Of course, the leading sequence can comprise up to about 100 nucleotides or even more, because this will increase the specificity for the respective mRNA.

In the context of the present invention, "sense sequences" (or sense strands) are those sequences which correspond to the coding strand of the *mlo* gene(s) or fragments thereof. Those sequences do not necessarily need to be 100 % identical with the sequences encoding the Mlo of interest. It will be sufficient that the sequences are similar (homologous) enough to the sequences encoding the MLO(s) that their expression in plant cells results in an efficient and specific silencing of the mlo(s), e.g. via RNA interference or co-suppression. It will be sufficient if those sequences are at least 50 %, preferably at least 60 %, more preferably at least 70 %, especially preferably at least 80 %, particularly at least 90 % and most preferably at least 95 % homologous. The differences can be the result of insertions, deletions, additions and/or substitutions. When sequences have those degrees of identity, they are usually called to be homologous (see above). The skilled person knows however that with decreasing identity or homology, the probability to silence several mlo mRNAs will increase. Sequences having a very low degree of similarity or homology, i.e. sequences that will also silence other proteins than MLO, are not sufficiently specific and therefore not suitable for the present invention.

Accordingly, "antisense sequences" (or antisense strands) are those sequences which correspond to the non-coding DNA strand of the genes of the MLO of interest. Those sequences do not necessarily need to be 100% identical with the sequence of the non-coding DNA strands of the genes of interest, but can have the above mentioned degrees of homology. For example, the antisense sequence can be at least 40 % or 50 %, preferably at least 55 % or 60 %, more preferably at least 65 % or 70 %, especially preferably at least 75 % or 80 %, particularly preferably at least 85 % or 90 %, and most preferably at least 92 %, 94 %, 95 %, 96 %, 97 %, 98 % or 99 % homologous to the non-coding *mlo* strand (or complementary to the coding strand). As mentioned above, it is sufficient that those antisense sequences are able to hybridize specifically with the respective mlo mRNA. The hybridization can take place either *in vivo* under cellular conditions or *in vitro.* The hybridization of an antisense sequence with an endogenous mRNA sequence usually takes place *in vivo* under cellular conditions.

The terms "sense" and "antisense" are well known to the person skilled in the art. The person skilled in the art of silencing genes in plants knows from the literature how long the nucleic acid molecules, which are used for silencing, have to be and what degree of homology or complementarity they have to exhibit in relation to the sequences of interest. In the context of the present invention, an antisense sequence which does not specifically hybridize with the coding sense sequences of mlo, i.e. which also hybridize with the coding sense sequences of other proteins, cannot be used.

The antisense strategy can be coupled with a ribozyme method. Ribozymes are catalytically active RNA sequences which, being coupled to the antisense sequences, catalytically cleave their target sequences (Tanner et al., (1999) FEMS Microbiol Rev. 23 (3), 257-75). This can increase the efficiency of an antisense strategy.

Other methods of reducing the expression of mlo particularly in plants comprise the over-expression of mlo nucleic acid sequences or their homologues, resulting in co-suppression (Jorgensen et al., (1996) Plant Mol. Biol. 31 (5), 957-973) or the induction of the specific RNA degradation by means of a viral expression system (amplicon) (Angell et al., (1999) Plant J. 20 (3), 357-362). Those methods are also referred to as PTGS (see above).

Other methods are the introduction of nonsense mutations in the endogenous gene via transfer of RNA/DNA oligonucleotides into the plant (Zhu et al., (2000) Nat. Biotechnol. 18 (5), 555-558) or the generation of knockout mutants by means of T-DNA mutagenesis (Koncz et al., (1992) Plant Mol. Biol. 20 (5) 963-976) or homologous recombination (Hohn et al., (1999) Proc. Natl. Acad. Sci. USA. 96, 8321-8323).

Furthermore a gene repression (but also the gene overexpression) can also be performed by means of specific DNA binding factors, e.g. factors of the type of zinc finger transcription factors. Also, factors can be introduced into a cell which inhibit the target protein. The protein binding factors can be for example aptamers (Famulok et al., (1999) Curr Top Microbiol Immunol. 243, 123-36). They are expressed via vector-based overexpression, and their design and selection can be easily performed by the skilled person.

An overview over the above-mentioned methods can be found, e.g., in Waterhouse et al., (2001), Nature 411, 834-842; Tuschl (2002), Nat. Biotechnol. 20, 446-448 ; Paddison et al., (2002), Genes Dev., 16, 948-958; Brummelkamp et al., (2002), Science 296, 550-553.

Another aspect of the present invention is a method of increasing resistance against soybean rust in transgenic plants and/or plant cells, wherein the content and/or the activity of at least one endogenous MLO is decreased by the expression of at least one non-functional MLO or a fragment thereof which has at least one point mutation, deletion and/or insertion. The non-functional MLO proteins have lost completely or to a very important degree their capacity to interact with the common physiological or pathogenic binding partners. Those non-functional mutants can comprise one or more amino acid insertions, deletions or point mutations. They are useful for the production of transgenic plants or plant cells in which the content of endogenous MLO protein is not altered, but the activity of the endogenous MLO is blocked by means of the overexpression of said non-functional MLO mutants. Furthermore, those resistant plants have the advantage to exhibit an essentially normal phenotype.

Non-functional MLO proteins or mutants have essentially the same nucleic acid and amino acid sequences as their functional counterparts. However, they comprise one or more insertions, deletions or point mutations of nucleotides or amino acids, which cause a dramatic decrease of the capacity of the mutated MLO protein to interact with its binding partners. The skilled person has a series of methods at hand which allow him to insert point mutations, deletions or insertions into the nucleic acid sequences encoding the functional or non-functional MLO proteins (Sambrook (2001), Molecular Cloning: A Laboratory Manual, 3rd edition, Coldspring Harbour Laboratory Press; "PCR technology: Principle and Applications for DNA Amplification", H. Ehrlich, id, Stockton Press). The reduced binding efficiency of those MLO mutants to the physiological and/or pathogenic binding partners in comparison to the wild type (non mutated) MLO proteins is preferably in the range of over 1 % to 90 %, more preferably over 1 % to 70 %, especially preferably over 1 % to 50 %, particularly preferably over 1 % to 30 %, and most preferably over 1 % to 10 %.

Although the non-functional MLO mutants show one or more point mutations, deletions and/or insertions, the term "non-functional" MLO (also called inactive MLO) does not comprise proteins which have no essential sequence homology to the functional MLO proteins as depicted in SEQ ID NOs: 2, 4, 7, 9, 11, 14, 16, 18, 20, 22, 24, 26, 28, 30, 32, 34, 36, 38, 40, 42, 44, 46 and 48.

Preferably, the at least one point mutation, deletion and/or insertion of the non-functional MLO prevent the cellular function of MLO, and especially inhibit the interaction of MLO with its pathogenic or physiologic binding partners, especially with ROR2 and/or calmodulin.

The non-functional MLO mutant being expressed or overexpressed in the transgenic plants according to the invention does not necessarily have to be the same MLO than the endogenous MLO of the host cell, but can also derive from another organism / species. The important characteristic of the non-functional MLO mutant is their competition with the activity of the endogenous MLO. Of course, a high degree of sequence homology between those two proteins will favor a high competitive activity of the non-functional MLO.

Also disclosed is that the non-functional MLO is a dominant negative MLO. The "dominant negative method" can comprise the following steps:
a) Construction of a vector comprising the following nucleic acid sequences in 5'-3' orientation:
   - a promoter sequence which is functionally active in plants,
   - operatively linked thereto a DNA sequence encoding a dominant negative mutant of the at least one endogenous Mlo,
   - optionally, operatively linked thereto a termination sequence which is functionally active in plants,
b) transfer of the vector from step a) to a plant cell and optionally integration into the plant genome.

The person skilled in the art can identify dominant negative mutants by means of routine methods. He can, e.g., introduce mutations into a wild type MLO sequence and perform *in vitro* binding assays of the obtained mutants with binding partners such as ROR2 or calmodulin. In the same way, the skilled person can test whether the MLO non-functional mutants compete with their wild type counterparts in terms of interactions with known binding partners.

A "dominant negative mutant", in the scope of the present invention, is every mutant (insertion, deletion, point mutation) which is capable of inhibiting the interaction of an MLO with its pathogenic or physiologic binding partners, such as ROR2 and/or calmodulin.

Transgenic plants or plant cells having an increased resistance against soybean rust can also be produced by a method which is characterized in that the content and/or the activity of at least one endogenous Mlo is decreased by the expression of at least one recombinant antibody which is specific for at least one endogenous Mlo and which prevents the cellular function of the Mlo, and which especially inhibits the interaction of the Mlo with its pathogenic or physiologic binding partners, especially with Ror2 and/or calmodulin.

The skilled person knows from the literature how those antibodies, which are e.g. specific for a certain MLO domain, can be produced, isolated and identified. According to the present invention, the term "recombinant antibody" comprises all of the different forms and types of antibodies, such as described in Skerra A. (Curr Opin Immunol. 1993 Apr;5(2):256-62). Examples are Fab fragments, Fv fragments, scFv antibodies, scFv homodimers, VH chains etc. A review is given by Conrad U. and Fiedler U. (Plant Mol Biol. 1998 Sep;38(1-2):101-9). Standard protocols for the production of monoclonal, polyclonal or recombinant antibodies can be found in: "Guide to Protein Purification", Meth. Enzymol. 182, pp. 663-679 (1990), M. P. Deutscher, ed. The expression of antibodies is also described in Fiedler et al., (1997) Immunotechnology 3, 205-216 and Maynard and Georgiou (2000) Annu. Rev. Biomed. Eng. 2, 339-76.

Preferred in the present invention are scFv antibodies which consist of the variable region of a light chain and the variable region of a heavy chain, being fused with one another by a flexible linker peptide (see, e.g. Breitling et al. (1999) Recombinant Antibodies, John Wiley & Sons, New York). ScFv antibodies have the same antigen specificity and activity as "normal" antibodies, but do not need to be assembled from single chains.

Usually, the production of a recombinant antibody starts with hybridoma cell lines expressing monoclonal antibodies. The cDNAs encoding the light and the heavy chain are isolated, and in a next step the coding regions for the variable regions of the light and the heavy chain are fused to one molecule. Another method of obtaining recombinant antibodies is based on the screening of recombinant antibody libraries, so-called phage display libraries (see Hoogenboom et al. (2000) Immunology Today 21, 371-378; Winter et al. (1994) Annu. Rev. Immunol. 12, 433-455; De Wildt et al. (2000) Nat. Biotechnol. 18, 989-994). This method allows the enrichment, selection and isolation of the desired antibody against an MLO protein.

One method of expression of an anti-MLO antibody can comprise the following steps:
a) construction of a vector comprising the following nucleic acid sequences in 5'-3' orientation:
   - a promoter sequence which is functionally active in plants,
   - operatively linked thereto a DNA sequence encoding a recombinant antibody which is specific for the at least one endogenous Mlo and which prevents the cellular function of Mlo,
   - optionally, operatively linked thereto a termination sequence which is functionally active in plants,
b) transfer of the vector from step a) to a plant cell and optionally integration into the plant genome.

Transgenic plants or plant cells having an increased resistance against soybean rust can also be produced by a method which is characterized in that the content and/or the activity of at least one endogenous MLO is decreased by the expression of at least one MLO inhibitor which prevents the cellular function of at least one MLO, and which especially inhibits the interaction of the MLO with its pathogenic or physiologic binding partners, especially with ROR2 and/or calmodulin. These inhibitors can be e.g. peptides which bind in the respective binding pockets of the MLO proteins for the interaction with the physiologic binding components or factors. This method resembles to the antibody strategy, in that an inhibitor of an MLO protein, being expressed or overexpressed in the plant cell, will block the MLO activity (e.g. sterically) by binding to the MLO. One method of expression of an MLO inhibitor can comprise the following steps:
a) construction of a vector comprising the following nucleic acid sequences in 5'-3' orientation:
   - a promoter sequence which is functionally active in plants,
   - operatively linked thereto a DNA sequence encoding an MLO inhibitor which prevents the cellular function of MLO,
   - optionally, operatively linked thereto a termination sequence which is functionally active in plants,
b) transfer of the vector from step a) to a plant cell and optionally integration into the plant genome.

In addition to the transfer of a nucleic acid molecule, other methods can be used for increasing the resistance of against soybean rust in transgenic plants or plant cells which have a decreased content and/or activity of at least one endogenous MLO. For example, the MLO content and/or activity can be decreased by mutagenesis, preferably by chemical mutagenesis or radiation induced mutagenesis. The mutagenesis can, e.g. be performed by means of ethyl methane sulfonate (EMS), gamma irradiation and/or fast neutron irradiation.

Induced mutations can also be caused by other chemicals like Nitrosoguanidine (NTG), base analogues (e.g. BrdU), simple chemicals (e.g. acids), alkylating agents (e.g. N-ethyl-N-nitrosourea, ENU), methylating agents (EMS), polycyclic hydrocarbons (e.g. benzpyrenes), DNA intercalating agents (e.g. ethidium bromide), DNA crosslinkers (e.g. platinum), oxygen radicals, or by UV irradiation (non-ionizing) or ionizing radiation. Alkylating agents can mutate both replicating and non-replicating DNA. In contrast, a base analog can only mutate the DNA when the analog is incorporated in replicating the DNA. Each of these classes of chemical mutagens has certain effects that then lead to transitions, transversions, or deletions. UV radiation excites electrons to a higher energy level. DNA absorbs one form, ultraviolet light. Two nucleotide bases in DNA - cytosine and thymine-are most vulnerable to excitation that can change base-pairing properties. UV light can induce adjacent thymine bases in a DNA strand to pair with each other, as a bulky dimer.

The mlo knockout in barley was found by the inventors to confer increased resistance against soybean rust. However, it is conceivable that also a positive resistance effect occurs via MLO. This means that an MLO overexpression, the MLO being derived e.g. from barley or Arabidopsis, could also lead to an increased resistance, especially of soybean. There are examples of proteins which confer resistance in a non-host interaction (as in the present case: barley - soybean rust), whereas in the case of a host interaction (soybean - soybean rust), those proteins confer susceptibility. For example, in susceptible interactions most fungal Avr proteins are pathogenicity factors, but in host-resistant plants the Avr protein is recognized by plant R-proteins. That means Avr proteins have different "functions" in resistant and compatible interactions.

Hence, an MLO overexpression could also confer resistance in soybean. Therefore, another aspect of the present application is directed to a method of increasing resistance against soybean rust in transgenic plants and/or plant cells, characterized in that the content and/or the activity of the at least one MLO is increased in comparison to the wild type.

The increase is preferably at least 10 % or 20 %, also preferably at least 30 % or 40 %, more preferably at least 50 % or 60 %, also more preferably at least 70 % or 80 %, especially preferably at least 90 %, 95% or 100 %, particularly preferably at least by a factor of 2 or 5, also particularly preferably at least by a factor of 10 or 50, and most preferably at least by a factor of 100 or 1000.

In a preferred embodiment of the application, this increase of the content and/or the activity of at least one MLO in comparison to the wild type can be performed by the transfer of at least one nucleic acid molecule encoding at least one MLO and/or a functionally equivalent fragment thereof and/or a functionally equivalent derivative thereof to the plants or plant cells.

In principle, the nucleic acid molecule can encode any known MLO from any organism (as well as functionally equivalent fragments and/or derivatives thereof). In case that the mlo sequence is of genomic origin from a eukaryotic cell and comprises introns, and in case that the host plant or plant cell is not able or cannot be enabled to splice those introns, it is preferable to use the corresponding cDNA sequence.

The following definitions of the terms "functionally equivalent fragment" and "functionally equivalent derivative" refer to the method of increasing the content and/or the activity of at least one MLO in comparison to the wild type. Therefore the fragments and mutants have to be functional, in contrast to the method of decreasing the content and/or the activity of at least one MLO, where the MLO fragments or mutants can also be non-functional.

In the context of the present invention, a nucleic acid molecule encoding a "functionally equivalent fragment" of an MLO is a fragment or part of a nucleic acid which encodes an MLO protein, having for example an amino acid sequence as depicted in SEQ ID NOs: 2, 4, 7, 9, 11, 14, 16, 18, 20, 22, 24, 26, 28, 30, 32, 34, 36, 38, 40, 42, 44, 46 and 48. The MLO fragment encoded by this nucleic acid has the same cellular functions, the same binding properties and/or the same structural properties as any of the MLO proteins having an amino acid sequence as depicted in SEQ ID NOs: 2, 4, 7, 9, 11, 14, 16, 18, 20, 22, 24, 26, 28, 30, 32, 34, 36, 38, 40, 42, 44, 46 and 48 (see above, definition of "functionally equivalent"). Usually, the fragment lacks amino acids at the N-terminus and/or at the C-terminus.

Preferably, the fragment has at least 40 % or 50 %, preferably at least 55 % or 60 %, more preferably at least 65 % or 70 %, especially preferably at least 75 % or 80 %, particularly preferably at least 85 % or 90 %, and most preferably at least 92 %, 94 %, 95 %, 96 %, 97 %, 98 % or 99 % of the length of the "whole" MLO protein.

In the context of the present invention, a nucleic acid molecule encoding a "functionally equivalent derivative" of an MLO protein is a derivative or "homologous" or "mutant" of a nucleic acid which encodes an MLO protein, wherein the MLO protein has e.g. an amino acid sequence as depicted in SEQ ID NOs: 2, 4, 7, 9, 11, 14, 16, 18, 20, 22, 24, 26, 28, 30, 32, 34, 36, 38, 40, 42, 44, 46 and 48. The MLO derivative encoded by this nucleic acid has the same cellular functions, the same binding properties and/or the same structural properties as any of the MLO proteins having an amino acid sequence as depicted in S SEQ ID NOs: 2, 4, 7, 9, 11, 14, 16, 18, 20, 22, 24, 26, 28, 30, 32, 34, 36, 38, 40, 42, 44, 46 and 48. Usually, the derivative has one or more amino acid exchanges, insertions or deletions which do not lead to altered cellular functions, binding properties and/or structural properties.

Preferably, the derivative is at least 40 % or 50 %, preferably at least 55 % or 60 %, more preferably at least 65 % or 70 %, especially preferably at least 75 % or 80 %, particularly preferably at least 85 % or 90 %, and most preferably at least 92 %, 94 %, 95 %, 96 %, 97 %, 98 % or 99 % similar of the "whole" MLO protein.

In a preferred embodiment of the application, a nucleic acid sequence encoding at least one MLO protein and/or a functionally equivalent fragment thereof and/or a functionally equivalent derivative thereof is transferred to a plant or plant cell. This transfer leads to an increase of the expression or the activity of MLO in comparison to the wild type and therefore to an increase of the resistance against soybean rust in the transgenic cells. The use of vectors comprising those nucleic acid sequences as well as promoter and optional termination sequences are well known to the skilled person. Such a method typically comprises the following steps:
a) Construction of a vector comprising the following nucleic acid sequences in 5'-3' orientation:
   - a promoter sequence which is functionally active in plants,
   - operatively linked thereto a DNA sequence encoding at least one MLO and/or a functionally equivalent fragment thereof and/or a functionally equivalent derivative thereof,
   - optionally, operatively linked thereto a termination sequence which is functionally active in plants,
b) transfer of the vector from step a) to a plant cell and optionally integration into the plant genome.

The skilled person knows how to transfer a vector from step a) to the plant cells and which characteristics the vector needs in order to be integrated into the plant genome. If the MLO content in transgenic plants or plant cells is increased by means of the transfer of a nucleic acid molecule encoding an Mlo from a different organism, it is preferable that the amino acid sequence is re-translated according to the genetic code into a nucleic acid sequence which mainly comprises codons which are preferably used by the host organism due to its "codon usage". The codon usage can be determined by means of computer based analyses of other genes known from the respective host organism.

The increase of the content and/or the activity of at least one endogenous Mlo can also be performed by influencing the transcription, the translation and/or the posttranslational modifications of the endogenous Mlo. This means for example that the gene expression of the endogenous Mlo is increased or that inhibiting regulatory mechanisms on the level of transcription, translation or proteins (e.g. posttranslational modifications) are turned off.

An increase of the gene expression can e.g. be achieved by influencing the promoter sequence of the endogenous Mlo gene. Such a modification, which preferably leads to an enhancement of the endogenous Mlo expression, can be achieved by deletion or insertion of DNA sequences. The modification of the promoter sequence usually leads to a modification of the amount of expressed Mlo and consequently to a modification of the MLO activity which can be determined in a plant cell.

Furthermore, a modified or increased expression of at least one endogenous Mlo gene can be achieved when a regulatory protein which does not occur in the transformed cell or plant interacts with the promoter of the endogenous Mlo gene. Such a regulator can be a chimeric protein containing a DNA binding domain and a transcription activation domain, as described e.g. in WO 96/06166.

Another possibility for increasing the content and/or the activity of an endogenous Mlo is based on the upregulation of transcription factors which are involved in the transcription of endogenous Mlo genes, e.g. by means of overexpression of those transcription factors. Methods of upregulating transcription factors are well known to the skilled person.

An increase of endogenous MLO can also be achieved when the post-transcriptional modifications of Mlo are influenced. For example, the activity of enzymes like kinases or phosphatases which are involved in this process can be regulated by means of procedures like overexpression or "gene silencing".

Finally the expression of endogenous Mlo can be regulated via the expression of aptamers which specifically bind to the promoter sequences of Mlo. Depending on whether the aptamers bind to stimulating or repressing promoter regions, the content and therefore also the activity of endogenous Mlo is increased.

Also disclosed is that the vector which is transferred to a plant or plant cell comprises further regulatory and/or functional sequences in addition to the promoter sequence and the optional termination sequence. More preferably, those regulatory and/or functional sequences are sequences which allow a propagation of the vector in bacteria and/or allow a transient and/or permanent replication in plant cells and/or are selected from the group consisting of enhancers, replication signals and selection markers.

The disclosed vectors can also include other e.g. enhancer elements as regulatory elements. In addition they can contain resistance genes, replication signals and other DNA regions which allow the propagation of the vectors in bacteria such as *E.coli.* The regulatory elements also include sequences which bring about stabilization of the vectors in the host cells. In particular, these regulatory elements include sequences which allow stable integration of the vector into the plant's host genome or an autonomous replication of the vector in the plant cells. The person skilled in the art is acquainted with this type of regulatory elements.

With the so-called termination sequences one means sequences which ensure that the transcription or the translation is properly terminated. If the transferred nucleic acids are to be translated, they are typically stop codons and corresponding regulatory sequences; if the transferred nucleic acids are only to be transcribed, they are generally poly-A sequences.

Preferably, the vector is selected from the group consisting of plasmids, cosmids, (recombinant) viruses and other current vectors known in the field of gene technology, with which nucleic acid molecules can be transferred to plants or plant cells. The term "vector" also comprises so-called minochromosomes which are linear or circular DNA fragments which contain centromer sequences of the respective plant in addition to the transgene. Minichromosomes are stable in the nucleus and are passed on to the daughter cells during cell division. They are transferred by standard methods of transformation. Most preferably, the vector is selected from the group consisting of pBR322, pUC vectors, M13mp vectors or vectors being derived from the Ti plasmid or the Ri plasmid of agrobacteria.

In order to prepare the introduction of foreign genes into higher plants or the cells of the same, a large number of cloning vectors are available which contain a replication signal for *E.coli* and a marker gene for the selection of transformed bacterial cells. Examples of such vectors are pBR322, pUC series, M13mp series, pACYC184, etc. The required sequence can be introduced into the vector at an appropriate restriction site. The plasmid obtained is used for the transformation of *E.coli* cells. Transformed *E.coli* cells are cultivated in an appropriate medium, and finally harvested and lysed. The plasmid is recovered. As an analysis method for characterizing the plasmid DNA obtained, methods such as restriction analyses, gel electrophoreses and other biochemical/molecular biological methods are generally used. Following each manipulation the plasmid DNA can be cleaved and the DNA fragments obtained can be combined with other DNA sequences. Each plasmid DNA sequence can be cloned into the same or other plasmids. Standard cloning methods can be taken from Sambrook et al., 2001 (Molecular cloning: A laboratory manual, 3rd edition, Cold Spring Harbor Laboratory Press).

The nucleic acid sequences to be transferred are preferably under the control of promoters which are functional in plants. In a preferred embodiment of the present invention, the promoter sequences are selected from the group consisting of constitutive promoters, preferably the 35S promoter, the actin promoter or the ubiquitin promoter, tissue specific promoters, preferably the phosphoenolpyruvate promoter or the fructose-1,6-bisphosphatase promoter, leaf specific promoters, epidermis specific promoters, development specific promoters, light specific promoters, lesion specific promoters or pathogen induced promoters, especially fungus induced promoters.

The promoters can be constitutive, induceable, tissue- or development-specific promoters. Moreover, they can also be pathogen-specific promoters. In this way e.g. transgenic plants can be produced which, under normal circumstances, express the MLO proteins, but if attacked by a pathogen, silence the genes for MLO proteins by means of the pathogen-specific promoter in the cells first affected.

Typically, the constitutive 35S promoter will be used as a promoter for vectors. Moreover, other promoters can, of course, be used, which are obtained from different sources, such as plants or plant viruses or fungi, and which are suitable for the expression of genes in plants. The choice of promoter and of other regulatory sequences determines the local and temporal expression pattern and also the silencing of the MLO proteins in transgenic plants.

Besides additional constitutive promoters, such as the actin promoter (McElroy et al., 1990, Plant Cell, 2:163) and the ubiquitin promoter (Binet et al., 1991, Plant Science, 79:87), the tissue-specific promoters of the phosphoenol pyruvate carboxylase from corn (Hudspeth et al., 1989, Plant Mol. Biol., 12:579) or of the fructose 1,6-bisphosphatase from potato (W0 98/18940), which determine the leaf-specific expression, can also be considered. Lesion induced, light induced or pathogen induced (especially fungus induced) promoters, leaf specific, epidermis specific and development-dependent promoters or control sequences can also be used (Xu et al., 1993, Plant Mol. Biol. 22:573; Logemann et al., 1989, Plant Cell, 1:151; Stockhaus et al., 1989, Plant Cell, 1:805; Puente et al., 1996, EMBO J., 15:3732; Gough et al., 1995, Mol. Gen. Genet., 247:323). A summary of useable control sequences can be found, e.g. in Zuo et al., 2000, Curr. Opin. Biotech., 11:146.

Appropriate promoters also include promoters which guarantee an expression solely in photosynthetically active tissues, e.g. the ST-LS1 promoter (Stockhaus et al. (1987) Proc. Natl. Acad. Sci. USA 84: 7943-7947; Stockhaus et al. (1989) EMBO J. 8:2445-2451). Promoters can also be used which are active during the plant transformation, the plant regeneration or specific stages of these processes, such as cell division-specific promoters such as the Histon H3 promoter (Kapros et al. (1993) InVitro Cell Cev. Biol. Plant 29:27-32) or the chemically induceable Tet-repressor system (Gatz et al. (1991) Mol. Gen. Genet. 227:229-237). Other suitable promoters can be taken from the literature, e.g. Ward (1993, Plant Mol. Biol. 22:361-366). The same applies for induceable and cell- or tissue-specific promoters, such as meristem-specific promoters, which have also been described in the literature and are suitable within the framework of the invention.

Other induceable promoters include pathogen-inducible promoters such as the ACMV virion sense promoter (Hong et al., 1996, Virology, 220:119-227) which is induced by the gene product AC2. Fungus induced promoters are also especially suitable for the present invention. Moreover, all promoters of such proteins which are induced in pathogen-infested tissues, such as phenylalanine ammonium lyase, chalcone synthase, hydroxyproline-rich glycoprotein, extensin, pathogenesis-related proteins (e.g. PR-1a) and wound-induceable protease inhibitors (US 6,013,864), are suitable. Furthermore, leaf specific promoters, such as promoters from photosynthetic tissue (e.g. CAP promoter, RBCS promoter, GAPA promoter, GAPB promoter, ST-LS1 promoter etc.) are especially suitable for the present invention.

Moreover, the average person skilled in the art is able to isolate additional suitable promoters by means of routine methods. The person skilled in the art, with the help of established molecular biology methods, e.g. hybridization experiments or DNA-protein-binding studies, can thus identify leaf-specific regulatory nucleic acid elements. In so doing, e.g. in a first step the whole poly(A) ⁺-RNA is isolated from the leaf tissue of the required organism from which the regulatory sequences are to be isolated, and a cDNA library is generated. In a second step, and with the help of cDNA clones which are based on poly(A) ⁺-RNA molecules from a non-leaf tissue, those clones, the corresponding poly(A) ⁺-RNA molecules of which only accumulate in the tissue of the leaf, are identified from the first library by means of hybridization. Finally, with the help of these cDNAs identified in this way, promoters are isolated which are equipped with leaf-specific regulatory elements. Other methods based on PCR are available to the person skilled in the art for the isolation of appropriate leaf-specific promoters.

Another embodiment uses the promoter of the class I patatin gene B33 from potato. Other favoured promoters are those which are particularly active in fruits. These include, for example, the promoter of a polygalacturonase gene, e.g. from tomato, which mediates expression during the maturation of tomato fruits (Nicholass et al.) (1995) Plant Mol. Biol. 28:423-435; this state of the art describes the analysis of promoter/GUS fusion constructs), the promoter of an ACC oxidase, e.g. from apple, which mediates maturity and fruit specificity in transgenic tomatoes (Atkinson et al. (1998) Plant Mol. Biol. 38:449-460; this state of the art also discloses promoter/GUS expression analyses), or the 2A11 promoter from tomato (van Haaren et al. (1991) Plant Mol. Biol. 17:615-630, also describes promoter/GUS fusions).

Also in the case of fruit-specific promoters, the person skilled in the art can take other suitable promoters from the literature, or as described above for leaf-specific promoters, isolate them by means of routine methods.

The person skilled in the art knows that the use of inducible promoters allows the production of plants and plant cells which only transiently express, and so only transiently silence the sequences according to the invention. Such a transient expression allows the production of plants which only show transient pathogen resistance. Such a transient resistance can e.g. be desirable if there is the risk of pathogen contamination and the plants therefore need to be resistant to the pathogen only for a particular length of time. The person skilled in the art is aware of other situations in which transient resistance is desirable. The person skilled in the art is also aware that, by the use of vectors which do not stably replicate in plant cells and which carry the respective sequences for the silencing of MLO proteins, he can achieve transient expression and therefore also transient silencing and transient resistance.

For the introduction of DNA into a plant host cell, there are a number of well-known techniques available, whereby the person skilled in the art can determine the appropriate method in each case without any problem. These techniques include the transformation of plant cells with T-DNA by using *Agrobacterium tumefaciens* or *Agrobacterium rhizogenes* as a transformation agent, the fusion of protoplasts, the direct gene transfer of isolated DNA into protoplasts, the electroporation of DNA, the introduction of DNA by means of the biolistic method, as well as other possibilities. In so doing, both stable and transient transformants can be generated.

With the injection and electroporation of DNA into plant-cells there are no special requirements per se for the plasmids used. The same applies for direct gene transfer. Simple plasmids, such as pUC derivates can be used. If, however, whole plants are to be regenerated from such transformed cells, the presence of a selectable marker gene is necessary. The person skilled in the art is acquainted with the current selection markers, and he will have no problem in selecting an appropriate marker. Standard selection markers are those which mediate resistance to a biocide or an antibiotic such as kanamycin, G418, bleomycin, hygromycin, methotrexat, glyphosat, streptomycin, sulfonyl urea, gentamycin or phosphinotricin and suchlike, to the transformed plant cell.

Dependent upon the method of introduction of the desired gene into the plant cell, other DNA sequences may be required. For example, if the Ti or Ri plasmid is used for the transformation of the plant cell, at least the right flanking region, often however the right and the left flanking region of the T-DNA contained in the Ti or Ri plasmid must be linked as a flanking region with the gene to be introduced.

If agrobacteria are used for the transformations, the DNA to be introduced must be cloned in special plasmids, either in an intermediary or in a binary vector. Based on sequences which are homologous to sequences in the T-DNA, the intermediary vectors can be integrated into the Ti or Ri plasmid of the agrobacteria by homologous recombination. This plasmid also contains the vir region necessary for the transfer of the T-DNA. Intermediary vectors cannot replicate in agrobacteria. By means of a helper plasmid, the intermediary vector can be transferred to *Agrobacterium tumefaciens* (conjugation). Binary vectors can replicate in *E.coli* as well as in agrobacteria. They contain a selection marker gene and a linker or polylinker which are framed by the right and left T-DNA border regions. They can be transformed directly into the agrobacteria (Holsters et al. (1978), Molecular and General Genetics 163, 181-187). The agrobacterium serving as a host cell should contain a plasmid which carries a vir region. The vir region is necessary for the transfer of the T-DNA into the plant cell. T-DNA can also be present. This type of transformed agrobacterium is used for the transformation of plant cells.

The use of T-DNA for the transformation of plant cells has been intensively investigated and is described sufficiently in EP 120 515.

For the transfer of DNA into the plant cell, plant explants can be cultivated specifically for this purpose with *Agrobacterium tumefaciens* or *Agrobacterium rhizogenes.* From the infected plant material (for example, pieces of leaf, stem segments, roots, but also protoplasts or suspension-cultivated plant cells) whole plants can be regenerated in an appropriate medium which can contain antibiotics or biocides for the selection of transformed cells. The regeneration of the plants takes place according to standard regeneration methods and using the common nutrient solutions. The plants and plant cells obtained in this way can by examined for the presence of the DNA introduced.

The person skilled in the art is acquainted with other possibilities for the introduction of foreign DNA using the biolistic method or by protoplast transformation (see L. Willmitzer (1993) Transgenic Plants in: Biotechnology, A Multi-Volume Comprehensive Treatise (publisher: H.J. Rehm et al.), volume 2, 627-659, VCH Weinheim, Germany).

Whereas the transformation of dicotyledenous plants or their cells by means ofTi plasmid vector systems with the help of *Agrobacterium tumefaciens* is well established, new work points to the fact that monocotyledonous plants or their cells are also very accessible to transformation by means of vectors based on agrobacteria (see e.g. Chan et al. (1993), Plant Mol. Biol. 22, 491-506).

Alternative systems for the transformation of monocotyledonous plants or their cells are transformation by means of the biolistic approach (Wan and Lemaux (1994) Plant Physiol. 104, 37-48; Vasil et al. (1993) Bio/Technology 11, 1553-1558; Ritala et al. (1994) Plant Mol. Bio. 24, 317-325; Spencer et al. (1990), Theor. Appl. Genet. 79, 625-631), protoplast transformation, electroporation of partially permeabilised cells as well as the introduction of DNA by means of glass tissues.

The transformed cells grow within the plant in the normal way (see also McCormick et al. (1986), Plant Cell Reports 5, 81-84). The resulting plants can be raised in the normal way and be crossed with plants which have the same transformed genetic disposition or other genetic dispositions. The resulting hybrid individuals have the respective phenotypical properties.

Two or more generations should be raised in order to ensure that the phenotypical feature remains stable and is inherited. Seeds should be harvested as well so as to ensure that the respective phenotype or other characteristics are maintained.

Similarly, by using the standard methods, transgenic lines can be determined which are homozygous for the new nucleic acid molecules and their phenotypical characteristics with regard to a present or non-present pathogen responsiveness is investigated and compared with that from hemizygous lines.

Of course, plant cells which contain the nucleic acid molecules according to the invention and plant cells (including protoplasts, calli, suspension cultures and suchlike) can further be cultivated.

The vectors described above can be transferred to plant cells in various ways. Whether the vectors are in linear or circular form depends upon the application in question. The person skilled in the art knows whether and when he can use respective linearized vectors or not. For example, the person skilled in the art knows that, for the production of specific knockouts of genes for MLO proteins by homologous recombination, it can suffice to linearize the corresponding vectors and inject them into the plants or plant cells.

Furthermore, also disclosed is a transgenic plant or plant cell having an increased resistance against soybean rust, characterized in that the content and/or the activity of at least one MLO protein is altered in comparison to wild type plants or plant cells, respectively, This plant can, e.g., be produced by any one of the methods which have been described above. "Transgenic plants" and the transgenic plant cells can signify any monocotyledonous or dicotyledonous plant or plant cell, preferably agricultural plants or cells from agricultural plants. The invention is further directed to transgenic parts of this plant such as leaves and blossoms, transgenic propagation material such as protoplasts, calli, fruit, seeds, tubers, rootstocks, germs, pollen, cuttings, and transgenic progeny of the plant.

According to one preferred embodiment, the content and/or the activity of at least one MLO protein is decreased in comparison to wild type plants or plant cells.

Also disclosed is that the content and/or the activity of at least one MLO protein is increased in comparison to wild type plants or plant cells.

Further disclosed are plant cells and plants in which the endogenous genes of MLO proteins have mutations, i.e. substitutions, insertions and/or deletions, which lead to the fact that the endogenous MLO proteins expressed are no longer able, or only able under certain circumstances, to interact with their endogenous cellular or pathologic binding partners. Plants or plant cells, which contain these endogenous gene copies for MLO proteins showing mutations, can be distinguished by increased transient or permanent resistance against soybean rust. Such plants and plant cells which, unlike the plants and plant cells specified above, are not transgenic can be produced by conventional mutagenesis.

Modulation of the expression of the endogenous plant MLO proteins can e.g. mean that by means of mutations in regulatory DNA elements of the genes of the MLO proteins, such as promoters, enhancers or generally so-called "upstream activating sequences", the expression of endogenous MLO proteins is downregulated.

Within the framework of the present invention, the modulation of the binding characteristics of the MLO proteins means that the above-specified types of mutation lead to a change of the binding characteristics of the endogenous MLO proteins their endogenous cellular or pathologic binding partners. A combination of the modulation of the expression and the binding characteristics of the MLO proteins is also possible.

For example, plants or plant cells can have mutations in the gene sequences for MLO proteins which lead to the reduction of the expression of these proteins. Other plants or plant cells have mutations which lead to the dominant negative mutants described above. In both cases, plants with increased resistance against soybean rust are obtained.

The person skilled in the art is aware that e.g. plants and plant cells can also be produced by mutagenesis which, because of mutations in enhancer and/or promoter sequences of the genes for plant MLO proteins, show a reduction of the expression of these proteins, and at the same time show mutations in the coding regions of the genes encoding MLO proteins, which give rise to the fact that the remaining expressed MLO proteins can no longer, or only to a limited extent, interact with the fungal and/or other cellular binding partners. On the other hand, respective mutations in enhancer and/or promoter sequences and in the coding sequences can have the effect that a dominant negative mutant of plant MLO proteins, as described above, which is no longer, or only to a very limited extent, able to interact with fungal and/or normal cellular interaction partners, is overexpressed, and so the competition reaction described above comes about.

Preferably, the non-transgenic plants and plant cells according to the invention, which are distinguished by a modulation of the expression and/or the binding characteristics of the endogenous MLO proteins and have permanent or transient resistance against soybean rust, are produced by means of the so-called "TILLING" method (Targeting Induced Local Lesion in Genomes). This method has been described in detail in Colbert et al. (2001, Plant Physiology, 126, 480 - 484), McCallum et al. (2000, Nat. Biotechnol., 18, 455 - 457) and McCallum et al. (2000, Plant Physiology, 123, 439 - 442). The above-specified references are introduced here as explicit disclosure with regard to the "TILLING" method.

The TILLING method is a strategy of the so-called reverse genetics which combines the production of high frequencies of point mutations in mutagenized plant collections, e.g. by means of chemical mutagenesis with ethyl methane sulphonate (EMS), with the fast systematic identification of mutations in target sequences. First of all, the target sequence is amplified via PCR in DNA pools of mutagenized M2 populations. Denaturation and annealing reactions of the heteroallelic PCR products allow the formation of heteroduplexes, wherein one DNA strand originates from the mutated and the other from the "wild-type" PCR product. A so-called mismatch then takes place at the site of the point mutation, which can be identified either by means of denaturating HPLC (DHPLC, McCallum et al., 2000, Plant Physiol., 123, 439 - 442) or with the *Cel*I mismatch detection system (Oleykowsky et al., 1998, Nucl. Acids Res. 26, 4597-4602). *Cel*I is an endonuclease which recognizes the mismatches in heteroduplex DNA and specifically cleaves the DNA at these sites. The cleavage products can then be separated and detected by means of automated sequencing gel electrophoresis (Colbert et al., 2001, vide supra). Following identification of target gene-specific mutations in a pool, individual DNA samples are analyzed accordingly in order to isolate the plant with the mutation. In this way, the identification of the mutagenized plant cells or plants can be made with the plants and plants cells according to the invention after the production of the mutagenized plant populations by the use of primer sequences targeted at MLO proteins. The TILLING method is generally applicable for all plants and so the cultivated and agricultural plants specified above are suitable for the method according to the invention.

Therefore also disclosed is a soybean rust resistant plant or plant cell, characterized in that it has been produced by the TILLING method and that it contains mutations in the coding and/or regulatory sequences of at least one gene encoding an MLO protein which cause an alteration in the content and/or the activity of the at least one MLO protein in comparison to wild type plants or plant cells.

Further disclosed is the use of at least one nucleic acid molecule, comprising:
a) at least one sequence which is identical, homologous or complementary to a sequence encoding an endogenous Mlo or fragments thereof,
b) at least one sequence encoding a non-functional Mlo or a fragment thereof which has at least one point mutation, deletion and/or insertion,
c) at least one sequence encoding a recombinant antibody which is specific for an endogenous Mlo and which prevents the cellular function of the Mlo,
d) at least one sequence encoding an Mlo inhibitor which prevents the cellular function an Mlo, and/or
e) at least one sequence encoding an Mlo and/or a functionally equivalent fragment thereof and/or a functionally equivalent derivative thereof
for increasing the resistance against soybean rust in transgenic plants and/or plant cells.

Also disclosed is an expression vector, comprising the following nucleic acid sequences in 5'-3' orientation:
a) a promoter sequence which is functionally active in plants,
b) operatively linked thereto a sequence
   - being identical, homologous or complementary to a sequence encoding an endogenous Mlo or fragments thereof,
   - encoding a non-functional Mlo or a fragment thereof which has at least one point mutation, deletion and/or insertion,
   - encoding a recombinant antibody which is specific for an endogenous Mlo and which prevents the cellular function of the Mlo,
   - encoding an Mlo inhibitor which prevents the cellular function an Mlo, and/or
   - encoding an Mlo and/or a functionally equivalent fragment thereof and/or a functionally equivalent derivative thereof,
c) optionally, operatively linked thereto a termination sequence which is functionally active in plants.

Finally, also disclosed is an isolated nucleic acid molecule comprising at least one nucleic acid sequence, selected from the group consisting of:
a) a nucleotide sequence according to SEQ ID NOs: 3, 5, 6, 8 or 10 or fragments thereof,
b) a nucleotide sequence which encodes a polypeptide having an amino acid sequence according to any one of SEQ ID NOs: 4, 7, 9 or 11 or fragments thereof,
c) a nucleotide sequence which is essentially homologous to any one of the nucleotide sequences of a) or b),
d) a nucleotide sequence which can hybridize under stringent conditions with any one of the nucleotide sequences of a), b) or c),
wherein the nucleic acid sequence encodes an MLO protein.

The skilled person is aware of the fact that the term "nucleotide sequence" of item a) preferably refers to the coding parts of SEQ ID NOs: 3, 5, 6, 8 or 10, i.e. the parts which encode an MLO protein, and not the regulatory parts which are usually located upstream and downstream of the coding region. However, the 5' and 3' untranslated regions of SEQ ID NOs: 3, 5, 6, 8 or 10 can also be included in the nucleic acid molecule.

An "isolated nucleic acid molecule" means a molecule which is separated from the other nucleic acid molecule which are present in the natural source of the nucleic acid. Preferably, an isolated nucleic acid molecule comprises no sequences which are naturally flanking the genomic DNA of the organism from which the molecule originates. In some embodiments, the isolated nucleic acid molecule can include, e.g., less than about 5 kb, 4 kb, 3 kb, 2 kb, 1 kb, 0,5 kb or 0,1 kb of nucleic acid sequences which are naturally flanking the genomic DNA of the cell from which the molecule originates.

An "MLO protein" has the biologic activity that the alteration of its content and/or activity within a plant or plant cell leads to in increased resistance of the plant or plant cell against soybean rust. The biological activity (or "cellular function") is especially meant to be the interactions of the MLO protein with its pathogenic or physiologic binding partners, i.e. preferably its interactions with calmodulin and/or ROR2. Therefore, a "fragment" of a nucleotide sequence which is part of the above-mentioned isolated nucleic acid molecule is limited to those fragments which encode an MLO protein having this biologic activity. Usually, the fragments lack nucleotides at the 5' end or at the 3' end. Preferably the fragment has at least 40 % or 50 %, preferably at least 55 % or 60 %, more preferably at least 65 % or 70 %, especially preferably at least 75 % or 80 %, particularly preferably at least 85 % or 90 %, and most preferably at least 92 %, 94 %, 95 %, 96 %, 97 %, 98 % or 99 % of the length of the "whole" MLO encoding sequence.

A nucleotide sequence which is "essentially homologous" to another nucleotide sequence means, in the scope of the present invention, that the sequence is at least 40 % or 50 %, preferably at least 55 % or 60 %, more preferably at least 65 % or 70 %, especially preferably at least 75 % or 80 %, particularly preferably at least 85 % or 90 %, and most preferably at least 92 %, 94 %, 95 %, 96 %, 97 %, 98 % or 99 % similar to the other sequence or fragments. Preferably, this homology is determined over the whole sequence length of the nucleotide sequence.

Of course, the nucleotide sequences which are "essentially homologous" or which can "hybridize under stringent conditions" (see items c) and d) above) also need to encode a functional MLO protein according to the invention, i.e. an MLO protein having a biologic activity as described above.

### EXAMPLES

Barley mlo mutation mlo5 was used in the present invention. Mlo5 is a complete null mutant which was obtained by the exchange of Met-1 (ATG) to Ile. The sequence of the wild-type MLO protein is depicted in SEQ ID NO: 2.

Barley cultivar "Ingrid" wild type and mlo5 mutant were used. The seeds were provided by the division APR/HS of the BASF AG (Agrarzentrum, Limburgerhof). The breeding was performed for 7 days in climatic exposure test cabinets at controlled conditions, i.e. a temperature of 22°C and a day/night rhythm of 12 hours. The plants were inoculated with *P.pachyrhizi* 7 days after the sowings.

The soybean rust fungus was a wild isolate from Brazil.

In order to obtain appropriate spore material for the inoculation, soybean leaves which had been infected with soybean rust 15-20 days ago, were taken 2-3 days before the inoculation and transferred to agar plates (1 % agar in H₂O). The leaves were placed with their upper side onto the agar, which allows the fungus to grow through the tissue and to produce very young spores. For the inoculation solution, the spores were knocked off the leaves and were added to a Tween-H₂O solution. The counting of spores was performed under a light microscope by means of a Thoma counting chamber. For the inoculation of the plants, the spore suspension was added into a compressed-air operated spray flask and applied uniformly onto the plants or the leaves until the leaf surface was well moisturized. For the microscopy, a density of 10x10⁵ spores / ml was used. The inoculated plants were placed for 24 hours in a greenhouse chamber with an average of 22°C and >90% of air humidity. The inoculated leaves were incubated under the same conditions in a closed Petri dish on 0,5% plant agar. The following cultivation was performed in a chamber with an average of 25°C and 70% of air humidity.

For the evaluation of the pathogen development, the inoculated leaves of barley "Ingrid" wild-type and barley "Ingrid" mlo5 were stained with aniline blue. The same protocol can also be used for soybean.

The aniline blue staining serves for the detection of fluorescent substances. During the defense reactions in host interactions and non-host interactions, substances such as phenols, callose or lignin accumulate or are produced and are incorporated at the cell wall either locally in papillae or in the whole cell (hypersensitive reaction, HR). Complexes are formed in association with aniline blue, which lead e.g. in the case of callose to yellow fluorescence. The leaf material was transferred to falcon tubes or dishes containing destaining solution II (ethanol / acetic acid 6/1) and was incubated in a water bath at 90°C for 10-15 minutes. The destaining solution II was removed immediately thereafter, and the leaves were washed 2x with water. For the staining, the leaves were incubated for 1,5-2 hours in staining solution II (0.05 % aniline blue = methyl blue, 0.067 M di-potassium hydrogen phosphate) and analyzed by microscopy immediately thereafter.

The different interaction types were evaluated (counted) by microscopy. An Olympus UV microscope BX61 (incident light) and a UV Longpath filter (excitation: 375/15, Beam splitter: 405 LP) were used. After aniline blue staining, the spores appear blue under UV light. The papillae can be recognized beneath the fungal appressorium by a green/yellow staining. The hypersensitive reaction (HR) is characterized by a whole cell fluorescence.

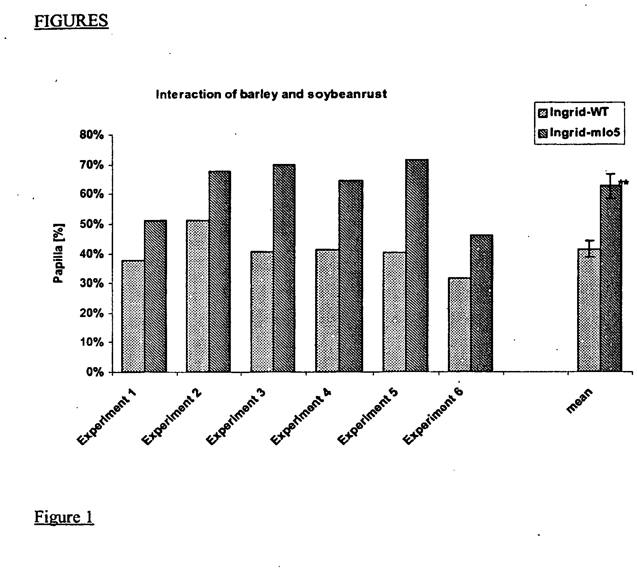

The results are shown in Table 3 as well as in Figure 1.

Two main stages of plant resistance against fungal growth can be discriminated. 1. The formation of a papilla as a mechanical barrier on the inner side of the cell wall under the appressorium prevents the infection of the cell and the further growth of the fungus. 2. The death of the infected cell, called hypersensitive reaction (HR), is another means of resistance against the fungus.

Figure 1 shows a significant increase of the rate of papillae formation in the barley "Ingrid" mlo5 compared to the wild-type.

**Table 3: Barley-lines infected with soybean rust fungus**

| exp.# | barley Ingrid | spores | spores with appressorium | appressorium with papillae | HR reaction | appressorium formation [%] | papillae formation [%] | HR [%] |
|---|---|---|---|---|---|---|---|---|
| 1 | wt | 570 | 479 | 181 | 142 | 84,0 | 37,8 | 29,6 |
| | mlo5 | 606 | 536 | 274 | 92 | 88,4 | 51,1 | 17,2 |
| | | | | | | | | |
| 2 | wt | 763 | 688 | 352 | 123 | 90,2 | 51,2 | 17,9 |
| | mlo5 | 795 | 657 | 445 | 127 | 82,6 | 67,7 | 19,3 |
| | | | | | | | | |
| 3 | wt | 638 | 523 | 212 | 74 | 82,0 | 40,5 | 14,1 |
| | mlo5 | 534 | 470 | 328 | 37 | 88,0 | 69,8 | 7,9 |
| | | | | | | | | |
| 4 | wt | 933 | 870 | 358 | 60 | 93,2 | 41,1 | 6,9 |
| | mlo5 | 894 | 793 | 510 | 26 | 88,7 | 64,3 | 3,3 |
| | | | | | | | | |
| 5 | wt | 692 | 643 | 258 | 44 | 92,9 | 40,1 | 6,8 |
| | mlo5 | 745 | 667 | 476 | 60 | 89,5 | 71,4 | 9,0 |
| | | | | | | | | |
| 6 | wt | 491 | 355 | 112 | 162 | 72,3 | 31,5 | 45,6 |
| | mlo5 | 707 | 500 | 230 | 148 | 70,7 | 46,0 | 29,6 |
| | | | | | | | | |
| total | wt | 4087 | 3558 | 1473 | 605 | 87,1 | 41,4 | 17,0 |
| | mlo5 | 4281 | 3623 | 2263 | 490 | 84,6 | 62,5 | 13,5 |

The standard error for the papillae formation percentage is 0,02592647 in wt barley and 0,04322237 in mlo5 barley. The value for the T test is 0,001735842.

### DESCRIPTION OF THE FIGURES

- Figure 1: Rate of papillae formation (%) of barley "Ingrid" wild type and barley "Ingrid" mlo5 mutant after infection with the soybean rust fungus.

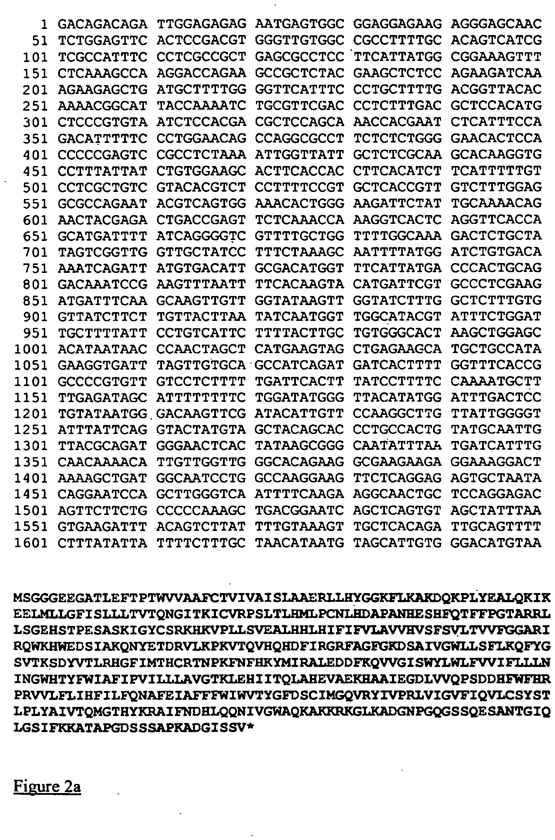

- Figure 2a: GmMlo1 - Soy (full-length) nucleic acid sequence (SEQ ID NO: 3)
GmMlo1 - Soy (full-length) amino acid sequence (SEQ ID NO: 4)

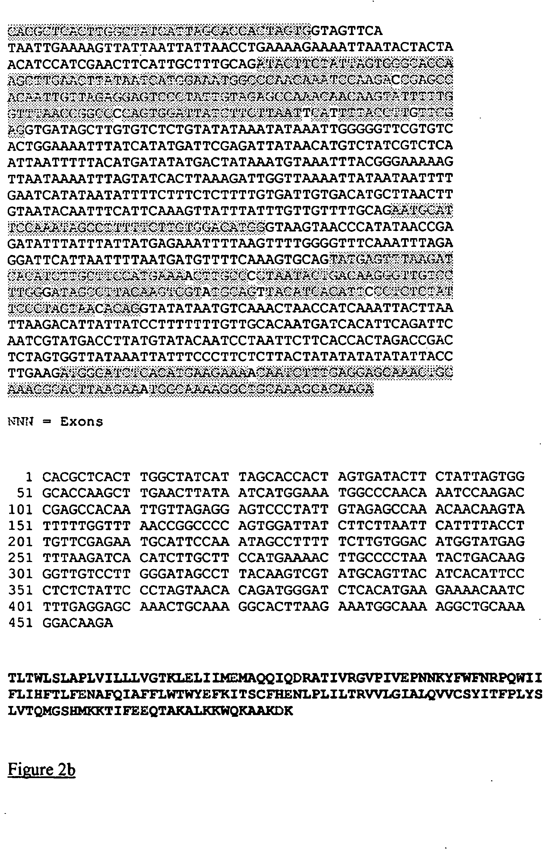

- Figure 2b: GmMlo2 (genomic) - Soy partial nucleic acid sequence (SEQ ID NO: 5)
GmMlo2 (EST) - Soy partial nucleic acid sequence (SEQ ID NO: 6)
GmMlo2 (EST) - Soy partial amino acid sequence (SEQ ID NO: 7)

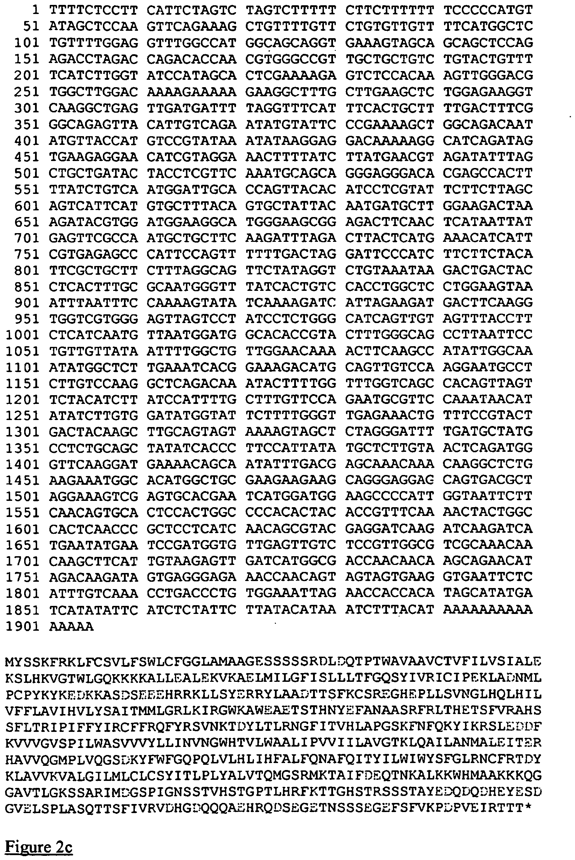

- Figure 2c: GmMlo3.1 - Soy (full length) nucleic acid sequence (SEQ ID NO: 8)
GmMlo23.1 - Soy (full length) amino acid sequence (SEQ ID NO: 9)

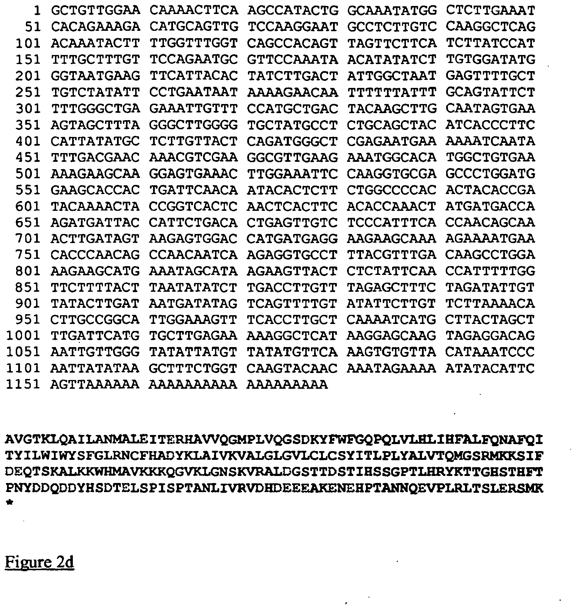

- Figure 2d: GmMlo3.2 (EST) - Soy nucleic acid sequence (SEQ ID NO: 10)
GmMlo3.2 - Soy theoretical amino acid sequence (SEQ ID NO: 11)

## Claims

1. Method of increasing resistance against soybean rust in transgenic plants and/or plant cells,
**characterized in that** the content and/or the activity of at least one endogenous MLO protein is decreased in comparison to wild type plants or plant cells, respectively; wherein the at least one endogenous MLO protein has an amino acid sequence with at least 75% homology with the amino acid sequence as depicted in SEQ ID NO: 4 or their functionally equivalent parts or fragments thereof, or wherein the at least one endogenous MLO protein has an amino acid sequence which is functionally equivalent to the amino acid sequence of a protein having at least 75% homology with the amino acid sequence as depicted in SEQ ID NO: 4 or fragments thereof.

2. Method according to claim 1,
**characterized in that** the content and/or the activity of at least one endogenous MLO is decreased by the transfer of at least one nucleic acid molecule comprising at least one sequence which is identical, homologous or complementary to the sequence(s) encoding the endogenous MLO or fragments thereof to the plant cells.

3. Method according to claim 2,
**characterized in that** a part of the transferred nucleic acid molecule is at least 50 %, preferably at least 60 %, more preferably at least 70 %, especially preferably at least 80 %, particularly preferably at least 90 %, and most preferably at least 95 % homologous to the sequence encoding the endogenous MLO or fragments thereof.

4. Method according to any one of claims 2 or 3,
**characterized in that** the decrease of the content and/or the activity of the at least one endogenous MLO is achieved by RNA interference (RNAi), an antisense construct, a co-suppression construct, post-transcriptional gene silencing (PTGS), a ribonuclease P construct, homologous recombination, a ribozyme construct or virus induced gene silencing (VIGS).

5. Method according to claim 1,
**characterized in that** the content and/or the activity of at least one endogenous MLO is decreased by the expression of at least one non-functional MLO or a fragment thereof which has at least one point mutation, deletion and/or insertion.

6. Method according to claim 5,
**characterized in that** the at least one point mutation, deletion and/or insertion of the non-functional MLO prevent the cellular function of MLO, and especially inhibit the interaction of MLO with its pathogenic or physiologic binding partners, especially with Ror2 and/or calmodulin.

7. Method according to claim 1,
**characterized in that** the content and/or the activity of at least one endogenous MLO is decreased by the expression of at least one recombinant antibody which is specific for at least one endogenous MLO and which prevents the cellular function of the MLO, and which especially inhibits the interaction of the MLO with its pathogenic or physiologic binding partners, especially with Ror2 and/or calmodulin.

8. Method according to claim 1,
**characterized in that** the content and/or the activity of at least one endogenous MLO is decreased by the expression of at least one MLO inhibitor which prevents the cellular function of at least one MLO, and which especially inhibits the interaction of the MLO with its pathogenic or physiologic binding partners, especially with Ror2 and/or calmodulin.

9. Method according to any of the preceding claims,
**characterized in that** the MLO is a plant MLO selected from the group consisting of *Hordeum vulgare* (barley) MLO, *Oryza sativa* (rice) MLO, *Arabidopsis thaliana* MLO, preferably AtMlo1, AtMlo2, AtMlo3, AtMlo4, AtMlo5, AtMlo6, AtMlo7, AtMlo8, AtMlo9, AtMlo10, AtMlo11, AtMlo12, AtMlo13, AtMlo14 or AtMlo15, *Linum usitatissimum* (flax) MLO, *Triticum aestivum* (wheat) MLO, *Glycine max* (soy) MLO, preferably GmMlol, GmMlo2, GmMlo3.1 or GmMlo3.2, or an MLO which is essentially functionally equivalent to any one of said MLO proteins.

10. Method according to any of the preceding claims,
**characterized in that** the MLO is selected from the group consisting of an MLO having an amino acid sequence as depicted in any of SEQ ID NOs: 2, 4, 7, 9, 11, 14, 16, 18, 20, 22, 24, 26, 28, 30, 32, 34, 36, 38, 40, 42, 44, 46 and 48, or an MLO having an amino acid sequence which is essentially functionally equivalent to any of the MLO sequences depicted in SEQ ID NOs: 2, 4, 7, 9, 11, 14, 16, 18, 20, 22, 24, 26, 28, 30, 32, 34, 36, 38, 40, 42, 44, 46 and 48.

11. Method according to any one of the preceding claims,
**characterized in that** the plants are dicotyledonous plants such as soybean, cotton, rapeseed, tomato, sugar beet, potato, sunflower, pea, ornamental plants, tobacco, clover (Trifolium spec.), Kudzu (Pueraria lobata), trees and legumes such as Alfalfa, and especially soybean.

12. Method according to any one of claims 1 to 10,
**characterized in that** the plants are monocotyledonous plants such as *Hordeum* (barley), *Avena* (oat), *Triticum* (wheat), *Secale* (rye), *Oryza* (rice), *Sorghum* (millet), *Zea* (corn), *Panicum*, *Pennisetum*, *Setaria* and suchlike.

## Patentansprüche

1. Verfahren zum Erhöhen der Resistenz gegen Sojabohnenrost in transgenen Pflanzen und/oder Pflanzenzellen,
**dadurch gekennzeichnet, daß** der Gehalt und/oder die Aktivität von mindestens einem endogenen MLO-Protein im Vergleich zu Wildtyppflanzen bzw. -pflanzenzellen verringert ist; wobei das mindestens eine endogene MLO-Protein eine Aminosäuresequenz mit mindestens 75% Homologie zu der Aminosäuresequenz gemäß SEQ ID NO: 4 oder ihren funktionell äquivalenten Teilen oder Fragmenten aufweist, oder wobei das mindestens eine endogene MLO-Protein eine Aminosäuresequenz aufweist, die zu der Aminosäuresequenz eines Proteins mit mindestens 75% Homologie zu der Aminosäuresequenz gemäß SEQ ID NO: 4 oder Fragmenten davon funktionell äquivalent ist.

2. Verfahren nach Anspruch 1,
**dadurch gekennzeichnet, daß** der Gehalt und/oder die Aktivität des mindestens einen endogenen MLO durch den Transfer von mindestens einem Nukleinsäuremolekül, umfassend mindestens eine Sequenz, die zu der Sequenz/den Sequenzen, die für das endogene MLO oder Fragmente davon codiert/codieren, identisch, homolog oder komplementär ist, in die Pflanzenzellen verringert wird.

3. Verfahren nach Anspruch 2,
**dadurch gekennzeichnet, daß** ein Teil des transferierten Nukleinsäuremoleküls zu mindestens 50%, vorzugsweise zu mindestens 60%, stärker bevorzugt zu mindestens 70%, speziell bevorzugt zu mindestens 80%, insbesondere bevorzugt zu mindestens 90% und am stärksten bevorzugt zu mindestens 95% zu der Sequenz, die für das endogene MLO oder Fragmente davon codiert, homolog ist.

4. Verfahren nach einem der Ansprüche 2 oder 3,
**dadurch gekennzeichnet, daß** die Verringerung des Gehalts und/oder der Aktivität von dem mindestens einen endogenen MLO durch RNA-Interferenz (RNAi), durch ein Antisense-Konstrukt, durch ein Cosuppressionskonstrukt, durch posttranskriptionelles Gen-Silencing (PTGS), durch ein Ribonuklease-P-Konstrukt, durch homologe Rekombination, durch ein Ribozymkonstrukt oder durch virusinduziertes Gen-Silencing (VIGS) erzielt wird.

5. Verfahren nach Anspruch 1,
**dadurch gekennzeichnet, daß** der Gehalt und/oder die Aktivität von mindestens einem endogenen MLO durch die Expression von mindestens einem nichtfunktionellen MLO oder einem Fragment davon, das zumindest eine Punktmutation, Deletion und/oder Insertion aufweist, verringert wird.

6. Verfahren nach Anspruch 5,
**dadurch gekennzeichnet, daß** die mindestens eine Punktmutation, Deletion und/oder Insertion des nichtfunktionellen MLO die Zellfunktion von MLO verhindert und insbesondere die Interaktion von MLO mit seinen pathogenen oder physiologischen Bindungspartnern, insbesondere mit Ror2 und/oder Calmodulin, hemmt.

7. Verfahren nach Anspruch 1,
**dadurch gekennzeichnet, daß** der Gehalt und/oder die Aktivität von dem mindestens einen endogenen MLO durch die Expression von mindestens einem rekombinanten Antikörper, der für mindestens ein endogenes MLO spezifisch ist und der die Zellfunktion des MLO verhindert und der speziell die Interaktion des MLO mit seinen pathogenen oder physiologischen Bindungspartnern, insbesondere mit Ror2 und/oder Calmodulin, inhibiert, verringert wird.

8. Verfahren nach Anspruch 1,
**dadurch gekennzeichnet, daß** der Gehalt und/oder die Aktivität von dem mindestens einen endogenen MLO durch die Expression des mindestens einen MLO-Inhibitors, der die Zellfunktion des mindestens einen MLO verhindert und der speziell die Interaktion des MLO mit seinen pathogenen oder physiologischen Bindungspartnern, insbesondere mit Ror2 und/oder Calmodulin, hemmt, verringert wird.

9. Verfahren nach einem der vorhergehenden Ansprüche, **dadurch gekennzeichnet, daß** es sich bei dem MLO um ein pflanzliches MLO, ausgewählt aus der Gruppe bestehend aus einem MLO aus *Hordeum vulgare* (Gerste), MLO aus *Oryza sativa* (Reis), MLO *Arabidopsis thaliana,* vorzugsweise AtMlo1, AtMlo2, AtMlo3, AtMlo4, AtMlo5, AtMlo6, AtMlo7, AtMlo8, AtMlo9, AtMlo10, Atmlo11, AtMlo12, AtMlo13, AtMlo14 oder AtMlo15, MLO aus *Linum usitatissimum* (Flachs), MLO aus *Triticum aestivum* (Weizen) , MLO aus *Glycin max* (Soja), vorzugsweise GmMlo1, GmMlo2, GmMlo3.1 oder GmMlo3.2 oder einem MLO, das im wesentlichen zu einem beliebigen dieser MLO-Proteine funktionell äquivalent ist, handelt.

10. Verfahren nach einem der vorhergehenden Ansprüche,
**dadurch gekennzeichnet, daß** das MLO aus der Gruppe bestehend aus einem MLO mit einer Aminosäuresequenz gemäß einer der SEQ ID NOs: 2, 4, 7, 9, 11, 14, 16, 18, 20, 22, 24, 26, 28, 30, 32, 34, 36, 38, 40, 42, 44, 46 und 48 oder einem MLO mit einer Aminosäuresequenz, die im wesentlichen zu einer beliebigen der MLO-Sequenzen gemäß SEQ ID NO: 2, 4, 7, 9, 11, 14, 16, 18, 20, 22, 24, 26, 28, 30, 32, 34, 36, 38, 40, 42, 44, 46 und 48 funktionell äquivalent ist, ausgewählt ist.

11. Verfahren nach einem der vorhergehenden Ansprüche,
**dadurch gekennzeichnet, daß** es sich bei den Pflanzen um dikotyle Pflanzen wie Sojabohne, Baumwolle, Raps, Tomate, Zuckerrübe, Kartoffel, Sonnenblume, Erbse, Zierpflanzen, Tabak, Klee (Trifolium spec.), Kudzu (Pueraria lobata), Bäume und Leguminosen wie Luzerne und insbesondere um Sojabohne handelt.

12. Verfahren nach einem der Ansprüche 1 bis 10,
**dadurch gekennzeichnet, daß** es sich bei den Pflanzen um monokotyle Pflanzen wie *Hordeum* (Gerste), *Avena* (Hafer), *Triticum* (Weizen), *Secale* (Roggen), *Oryza* (Reis), *Sorghum* (Hirse), *Zea* (Mais), *Panicum, Pennisetum, Setaria* und dergleichen handelt.

## Revendications

1. Procédé d'augmentation de la résistance à la rouille du soja dans des plantes et/ou des cellules de plante transgéniques,
**caractérisé en ce que** la teneur et/ou l'activité d'au moins une protéine MLO endogène est diminuée par rapport à des plantes ou des cellules de plante de type sauvage, respectivement ; dans lequel l'au moins une protéine MLO endogène a une séquence d'acides aminés avec au moins 75 % d'homologie avec la séquence d'acides aminés telle que décrite dans SEQ ID NO: 4 ou ses parties fonctionnellement équivalentes ou des fragments de celle-ci, ou dans lequel l'au moins une protéine MLO endogène a une séquence d'acides aminés qui est fonctionnellement équivalente à la séquence d'acides aminés d'une protéine ayant au moins 75 % d'homologie avec la séquence d'acides aminés telle que décrite dans SEQ ID NO: 4 ou des fragments de celle-ci.

2. Procédé selon la revendication 1,
**caractérisé en ce que** la teneur et/ou l'activité d'au moins un MLO endogène est diminuée par le transfert d'au moins une molécule d'acide nucléique comprenant au moins une séquence qui est identique, homologue ou complémentaire de la/les séquence(s) codant pour le MLO endogène ou des fragments de celui-ci vers les cellules de plante.

3. Procédé selon la revendication 2,
**caractérisé en ce qu'**une partie de la molécule d'acide nucléique transférée est d'au moins 50 %, de préférence au moins 60 %, plus préférablement au moins 70 %, de façon particulièrement préférable au moins 80 %, de façon particulièrement préférable au moins 90 %, et de manière préférée entre toutes au moins 95 % homologue à la séquence codant pour le MLO endogène ou des fragments de celui-ci.

4. Procédé selon l'une quelconque des revendications 2 ou 3,
**caractérisé en ce que** la diminution de la teneur et/ou l'activité de l'au moins un MLO endogène est obtenu par ARN interférence (ARNi), une construction antisens, une construction de cosuppression, le silençage de gène post-transcriptionnel (PTGS), une construction de ribonucléase P, la recombinaison homologue, une construction de ribozyme ou le silençage de gène induit par un virus (VIGS).

5. Procédé selon la revendication 1,
**caractérisé en ce que** la teneur et/ou l'activité d'au moins un MLO endogène est diminuée par l'expression d'au moins un MLO non fonctionnel ou un fragment de celui-ci qui a au moins une mutation, délétion et/ou insertion ponctuelle.

6. Procédé selon la revendication 5,
**caractérisé en ce que** l'au moins une mutation, délétion et/ou insertion ponctuelle du MLO non fonctionnel prévient la fonction cellulaire de MLO, et en particulier inhibe l'interaction de MLO avec ses partenaires de liaison pathogènes ou physiologiques, en particulier avec Ror2 et/ou la calmoduline.

7. Procédé selon la revendication 1,
**caractérisé en ce que** la teneur et/ou l'activité d'au moins un MLO endogène est diminuée par l'expression d'au moins un anticorps recombinant qui est spécifique pour au moins un MLO endogène et qui prévient la fonction cellulaire du MLO, et qui inhibe en particulier l'interaction du MLO avec ses partenaires de liaison pathogènes ou physiologiques, en particulier avec Ror2 et/ou la calmoduline.

8. Procédé selon la revendication 1,
**caractérisé en ce que** la teneur et/ou l'activité d'au moins un MLO endogène est diminuée par l'expression d'au moins un inhibiteur de MLO qui prévient la fonction cellulaire d'au moins un MLO, et qui inhibe en particulier l'interaction du MLO avec ses partenaires de liaison pathogènes ou physiologiques, en particulier avec Ror2 et/ou la calmoduline.

9. Procédé selon l'une quelconque des revendications précédentes,
**caractérisé en ce que** le MLO est un MLO de plante choisi dans le groupe constitué de MLO de *Hordeum vulgare* (orge), MLO d'*Oryza saliva* (riz), MLO d'*Arabidopsis thaliana*, de préférence AtMlo1, AtMlo2, AtMlo3, AtMlo4, AtMlo5, AtMlo6, AtMlo7, AtMlo8, AtMlo9, AtMlo10, AtMlo11, AtMlo12, AtMlo13, AtMlo14 ou AtMlo15, MLO de *Zinum usitatissimum* (lin), MLO de *Triticum aestivum* (blé), MLO de *Glycine max* (soja), de préférence GmMlo1, GmMlo2, GmMlo3.1 ou GmMlo3.2, ou un MLO qui est pratiquement équivalent fonctionnellement à l'une quelconque desdites protéines MLO.

10. Procédé selon l'une quelconque des revendications précédentes,
**caractérisé en ce que** le MLO est choisi dans le groupe constitué d'un MLO ayant une séquence d'acides aminés telle que décrite dans l'un quelconque des SEQ ID NO: 2, 4, 7, 9, 11, 14, 16, 18, 20, 22, 24, 26, 28, 30, 32, 34, 36, 38, 40, 42, 44, 46 et 48, ou un MLO ayant une séquence d'acides aminés qui est pratiquement équivalent fonctionnellement à l'une quelconque des séquences MLO décrites dans SEQ ID NO: 2, 4, 7, 9, 11, 14, 16, 18, 20, 22, 24, 26, 28, 30, 32, 34, 36, 38, 40, 42, 44, 46 et 48.

11. Procédé selon l'une quelconque des revendications précédentes,
**caractérisé en ce que** les plantes sont des plantes dicotylédones telles que le soja, le coton, le colza, la tomate, la betterave sucrière, la pomme de terre, le tournesol, le pois, les plantes d'ornement, le tabac, le trèfle (*Trifolium sp*.), le kudzu (*Pueraria lobata*), les arbres et les légumes tels que la luzerne, et en particulier le soja.

12. Procédé selon l'une quelconque des revendications 1 à 10,
**caractérisé en ce que** les plantes sont des plantes monocotylédones telles que *Hordeum* (orge), *Avena* (avoine), *Triticum* (blé), *Secale* (seigle), *Oryza* (riz), *Sorghum* (millet), Zea (maïs), *Panicum*, *Pennisetum*, *Setaria* et similaire.


### SEQUENCE LISTING

<110> BASF Plant Science GmbH
<120> Method of increasing resistance against soybean rust in transgenic plants
<130> B 8373/RN
<150> EP 06 118 753.0
   <151> 2006-08-10
<160> 48
<170> PatentIn version 3.3
<210> 1
   <211> 1602
   <212> DNA
   <213> Hordeum vulgare
<400> 1
   atgtcggaca aaaaaggggt gccggcgcgg gagctgccgg agacgccgtc gtgggcggtg      60
   gcggtggtct tcgccgccat ggtgctcgtg tccgtcctca tggaacacgg cctccacaag     120
   ctcggccatt ggttccagca ccggcacaag aaggccctgt gggaggcgct ggagaagatg     180
   aaggcggagc tcatgctggt gggcttcata tccctgctcc tcatcgtcac gcaggacccc     240
   atcatcgcca agatatgcat ctccgaggat gccgccgacg tcatgtggcc ctgcaagcgc     300
   ggcaccgagg gccgcaagcc cagcaagtac gttgactact gcccggaggg caaggtggcg     360
   ctcatgtcca cgggcagctt gcaccagctg cacgtcttca tcttcgtgct cgcggtcttc     420
   catgtcacct acagcgtcat caccatagct ctaagccgtc tcaaaatgag aacatggaag     480
   aaatgggaga cagagaccac ctccttggaa taccagttcg caaatgatcc tgcacggttc     540
   cggttcacgc accagacgtc gttcgtgaag cgccacctgg gcctctccag cacccctggc     600
   atcagatggg tggtggcctt cttcaggcag ttcttcaggt cagtcaccaa ggtggactac     660
   ctgaccttga gggcaggctt catcaacgcg catttgtcgc aaaacagcaa gttcgacttc     720
   cacaagtaca tcaagaggtc gatggaggac gacttcaagg tcgtcgtcgg catcagcctc     780
   ccgctgtggg gtgtggcgat cctcaccctc ttccttgaca tcaatggggt tggcacgctc     840
   atctggattt ctttcatccc tctcgtgatc ctcttgtgtg ttggaaccaa gctggagatg     900
   atcatcatgg agatggccct ggagatccag gaccgggcga gcgtcatcaa gggggccccc     960
   gtggtcgagc ccagcaacaa gttcttctgg ttccaccgcc ccgactgggt cctcttcttc    1020
   atacacctga cgttgttcca gaacgcgttt cagatggcgc attttgtgtg gacagtggcc    1080
   acgcccggct tgaagaaatg ctaccacacg cagatcgggc tgagcatcat gaaggtggtg    1140
   gtggggctag ctctccagtt cctctgcagc tatatgacct tcccccctca cgcgctcgtc    1200
   acacagatgg gatcaaacat gaagaggtcc atcttcgacg agcagacgtc caaggcgctc    1260
   accaactggc ggaacacggc caaggagaag aagaaagtcc gagacacgga catgctgatg    1320
   gctcagatga tcggcgacgc aacaccgagc cgaggctcgt cgccgatgcc gagccggggc    1380
   tcatcacccg tgcacctgct tcacaagggc atggggcggt cggacgaccc ccagagcgcg    1440
   cccacctcgc caaggaccca gcaggaggct agggacatgt acccggttgt ggtggcgcac    1500
   ccggtgcaca gactaaatcc taacgacagg aggaggtccg cctcgtcgtc ggccctcgaa    1560
   gccgacatcc ccagtgcaga tttttccttc agccagggat ga                       1602
<210> 2
   <211> 533
   <212> PRT
   <213> Hordeum vulgare
<400> 2
   Met Ser Asp Lys Lys Gly Val Pro Ala Arg Glu Leu Pro Glu Thr Pro
   1               5                   10                  15
   Ser Trp Ala Val Ala Val Val Phe Ala Ala Met Val Leu Val Ser Val
               20                  25                  30
   Leu Met Glu His Gly Leu His Lys Leu Gly His Trp Phe Gln His Arg
           35                  40                  45
   His Lys Lys Ala Leu Trp Glu Ala Leu Glu Lys Met Lys Ala Glu Leu
       50                  55                  60
   Met Leu Val Gly Phe Ile Ser Leu Leu Leu Ile Val Thr Gln Asp Pro
   65                  70                  75                  80
   Ile Ile Ala Lys Ile Cys Ile Ser Glu Asp Ala Ala Asp Val Met Trp
                   85                  90                  95
   Pro Cys Lys Arg Gly Thr Glu Gly Arg Lys Pro Ser Lys Tyr Val Asp
               100                 105                 110
   Tyr Cys Pro Glu Gly Lys Val Ala Leu Met Ser Thr Gly Ser Leu His
           115                 120                 125
   Gln Leu His Val Phe Ile Phe Val Leu Ala Val Phe His Val Thr Tyr
       130                 135                 140
   Ser Val Ile Thr Ile Ala Leu Ser Arg Leu Lys Met Arg Thr Trp Lys
   145                 150                 155                 160
   Lys Trp Glu Thr Glu Thr Thr Ser Leu Glu Tyr Gln Phe Ala Asn Asp
                   165                 170                 175
   Pro Ala Arg Phe Arg Phe Thr His Gln Thr Ser Phe Val Lys Arg His
               180                 185                 190
   Leu Gly Leu Ser Ser Thr Pro Gly Ile Arg Trp Val Val Ala Phe Phe
           195                 200                 205
   Arg Gln Phe Phe Arg Ser Val Thr Lys Val Asp Tyr Leu Thr Leu Arg
       210                 215                 220
   Ala Gly Phe Ile Asn Ala His Leu Ser Gln Asn Ser Lys Phe Asp Phe
   225 230 235 240
   His Lys Tyr Ile Lys Arg Ser Met Glu Asp Asp Phe Lys Val Val Val
   245 250 255
   Gly Ile Ser Leu Pro Leu Trp Gly Val Ala Ile Leu Thr Leu Phe Leu
   260 265 270
   Asp Ile Asn Gly Val Gly Thr Leu Ile Trp Ile Ser Phe Ile Pro Leu
   275 280 285
   Val Ile Leu Leu Cys Val Gly Thr Lys Leu Glu Met Ile Ile Met Glu
   290 295 300
   Met Ala Leu Glu Ile Gln Asp Arg Ala Ser Val Ile Lys Gly Ala Pro
   305 310 315 320
   Val Val Glu Pro Ser Asn Lys Phe Phe Trp Phe His Arg Pro Asp Trp
   325 330 335
   Val Leu Phe Phe Ile His Leu Thr Leu Phe Gln Asn Ala Phe Gln Met
   340 345 350
   Ala His Phe Val Trp Thr Val Ala Thr Pro Gly Leu Lys Lys Cys Tyr
   355 360 365
   His Thr Gln Ile Gly Leu Ser Ile Met Lys Val Val Val Gly Leu Ala
   370 375 380
   Leu Gln Phe Leu Cys Ser Tyr Met Thr Phe Pro Leu Tyr Ala Leu Val
   385 390 395 400
   Thr Gln Met Gly Ser Asn Met Lys Arg Ser Ile Phe Asp Glu Gln Thr
   405 410 415
   Ser Lys Ala Leu Thr Asn Trp Arg Asn Thr Ala Lys Glu Lys Lys Lys
   420 425 430
   Val Arg Asp Thr Asp Met Leu Met Ala Gln Met Ile Gly Asp Ala Thr
   435 440 445
   Pro Ser Arg Gly Ser Ser Pro Met Pro Ser Arg Gly Ser Ser Pro Val
   450 455 460
   His Leu Leu His Lys Gly Met Gly Arg Ser Asp Asp Pro Gln Ser Ala
   465 470 475 480
   Pro Thr Ser Pro Arg Thr Gln Gln Glu Ala Arg Asp Met Tyr Pro Val
   485 490 495
   Val Val Ala His Pro Val His Arg Leu Asn Pro Asn Asp Arg Arg Arg
   500 505 510
   Ser Ala Ser Ser Ser Ala Leu Glu Ala Asp Ile Pro Ser Ala Asp Phe
   515 520 525
   Ser Phe Ser Gln Gly
   530
<210> 3
   <211> 1650
   <212> DNA
   <213> Glycine max
<400> 3
   gacagacaga ttggagagag aatgagtggc ggaggagaag agggagcaac tctggagttc 60
   actccgacgt gggttgtggc cgccttttgc acagtcatcg tcgccatttc cctcgccgct 120
   gagcgcctcc ttcattatgg cggaaagttt ctcaaagcca aggaccagaa gccgctctac 180
   gaagctctcc agaagatcaa agaagagctg atgcttttgg ggttcatttc cctgcttttg 240
   acggttacac aaaacggcat taccaaaatc tgcgttcgac cctctttgac gctccacatg 300
   ctcccgtgta atctccacga cgctccagca aaccacgaat ctcatttcca gacatttttc 360
   cctggaacag ccaggcgcct tctctctggg gaacactcca cccccgagtc cgcctctaaa 420
   attggttatt gctctcgcaa gcacaaggtg cctttattat ctgtggaagc acttcaccac 480
   cttcacatct tcatttttgt cctcgctgtc gtacacgtct ccttttccgt gctcaccgtt 540
   gtctttggag gcgccagaat acgtcagtgg aaacactggg aagattctat tgcaaaacag 600
   aactacgaga ctgaccgagt tctcaaacca aaggtcactc aggttcacca gcatgatttt 660
   atcaggggtc gttttgctgg ttttggcaaa gactctgcta tagtcggttg gttgctatcc 720
   tttctaaagc aattttatgg atctgtgaca aaatcagatt atgtgacatt gcgacatggt 780
   ttcattatga cccactgcag gacaaatccg aagtttaatt ttcacaagta catgattcgt 840
   gccctcgaag atgatttcaa gcaagttgtt ggtataagtt ggtatctttg gctctttgtg 900
   gttatcttct tgttacttaa tatcaatggt tggcatacgt atttctggat tgcttttatt 960
   cctgtcattc ttttacttgc tgtgggcact aagctggagc acataataac ccaactagct 1020
   catgaagtag ctgagaagca tgctgccata gaaggtgatt tagttgtgca gccatcagat 1080
   gatcactttt ggtttcaccg gccccgtgtt gtcctctttt tgattcactt tatccttttc 1140
   caaaatgctt ttgagatagc attttttttc tggatatggg ttacatatgg atttgactcc 1200
   tgtataatgg gacaagttcg atacattgtt ccaaggcttg ttattggggt atttattcag 1260
   gtactatgta gctacagcac cctgccactg tatgcaattg ttacgcagat gggaactcac 1320
   tataagcggg caatatttaa tgatcatttg caacaaaaca ttgttggttg ggcacagaag 1380
   gcgaagaaga ggaaaggact aaaagctgat ggcaatcctg gccaaggaag ttctcaggag 1440
   agtgctaata caggaatcca gcttgggtca attttcaaga aggcaactgc tccaggagac 1500
   agttcttctg cccccaaagc tgacggaatc agctcagtgt agctatttaa gtgaagattt 1560
   acagtcttat tttgtaaagt tgctcacaga ttgcagtttt ctttatatta ttttctttgc 1620
   taacataatg tagcattgtg ggacatgtaa 1650
<210> 4
   <211> 506
   <212> PRT
   <213> Glycine max
<400> 4
   Met Ser Gly Gly Gly Glu Glu Gly Ala Thr Leu Glu Phe Thr Pro Thr
   1 5 10 15
   Trp Val Val Ala Ala Phe Cys Thr Val Ile Val Ala Ile Ser Leu Ala
   20 25 30
   Ala Glu Arg Leu Leu His Tyr Gly Gly Lys Phe Leu Lys Ala Lys Asp
   35 40 45
   Gln Lys Pro Leu Tyr Glu Ala Leu Gln Lys Ile Lys Glu Glu Leu Met
   50 55 60
   Leu Leu Gly Phe Ile Ser Leu Leu Leu Thr Val Thr Gln Asn Gly Ile
   65 70 75 80
   Thr Lys Ile Cys Val Arg Pro Ser Leu Thr Leu His Met Leu Pro Cys
   85 90 95
   Asn Leu His Asp Ala Pro Ala Asn His Glu Ser His Phe Gln Thr Phe
   100 105 110
   Phe Pro Gly Thr Ala Arg Arg Leu Leu Ser Gly Glu His Ser Thr Pro
   115 120 125
   Glu Ser Ala Ser Lys Ile Gly Tyr Cys Ser Arg Lys His Lys Val Pro
   130 135 140
   Leu Leu Ser Val Glu Ala Leu His His Leu His Ile Phe Ile Phe Val
   145 150 155 160
   Leu Ala Val Val His Val Ser Phe Ser Val Leu Thr Val Val Phe Gly
   165 170 175
   Gly Ala Arg Ile Arg Gln Trp Lys His Trp Glu Asp Ser Ile Ala Lys
   180 185 190
   Gln Asn Tyr Glu Thr Asp Arg Val Leu Lys Pro Lys Val Thr Gln Val
   195 200 205
   His Gln His Asp Phe Ile Arg Gly Arg Phe Ala Gly Phe Gly Lys Asp
   210 215 220
   Ser Ala Ile Val Gly Trp Leu Leu Ser Phe Leu Lys Gln Phe Tyr Gly
   225                 230                 235                 240
   Ser Val Thr Lys Ser Asp Tyr Val Thr Leu Arg His Gly Phe Ile Met
                   245                 250                 255
   Thr His Cys Arg Thr Asn Pro Lys Phe Asn Phe His Lys Tyr Met Ile
               260                 265                 270
   Arg Ala Leu Glu Asp Asp Phe Lys Gln Val Val Gly Ile Ser Trp Tyr
           275                 280                 285
   Leu Trp Leu Phe Val Val Ile Phe Leu Leu Leu Asn Ile Asn Gly Trp
       290                 295                 300
   His Thr Tyr Phe Trp Ile Ala Phe Ile Pro Val Ile Leu Leu Leu Ala
   305                 310                 315                 320
   Val Gly Thr Lys Leu Glu His Ile Ile Thr Gln Leu Ala His Glu Val
                   325                 330                 335
   Ala Glu Lys His Ala Ala Ile Glu Gly Asp Leu Val Val Gln Pro Ser
               340                 345                 350
   Asp Asp His Phe Trp Phe His Arg Pro Arg Val Val Leu Phe Leu Ile
           355                 360                 365
   His Phe Ile Leu Phe Gln Asn Ala Phe Glu Ile Ala Phe Phe Phe Trp
       370                 375                 380
   Ile Trp Val Thr Tyr Gly Phe Asp Ser Cys Ile Met Gly Gln Val Arg
   385                 390                 395                 400
   Tyr Ile Val Pro Arg Leu Val Ile Gly Val Phe Ile Gln Val Leu Cys
                   405                 410                 415
   Ser Tyr Ser Thr Leu Pro Leu Tyr Ala Ile Val Thr Gln Met Gly Thr
               420                 425                 430
   His Tyr Lys Arg Ala Ile Phe Asn Asp His Leu Gln Gln Asn Ile Val
           435                 440                 445
   Gly Trp Ala Gln Lys Ala Lys Lys Arg Lys Gly Leu Lys Ala Asp Gly
       450                 455                 460
   Asn Pro Gly Gln Gly Ser Ser Gln Glu Ser Ala Asn Thr Gly Ile Gln
   465                 470                 475                 480
   Leu Gly Ser Ile Phe Lys Lys Ala Thr Ala Pro Gly Asp Ser Ser Ser
                   485                 490                 495
   Ala Pro Lys Ala Asp Gly Ile Ser Ser Val
   500 505
<210> 5
   <211> 1133
   <212> DNA
   <213> Glycine max
<400> 5
   cacgctcact tggctatcat tagcaccact agtggtagtt cataattgaa aagttattaa 60
   ttattaacct gaaaagaaaa ttaatactac taacatccat cgaacttcat tgctttgcag 120
   atacttctat tagtgggcac caagcttgaa cttataatca tggaaatggc ccaacaaatc 180
   caagaccgag ccacaattgt tagaggagtc cctattgtag agccaaacaa caagtatttt 240
   tggtttaacc ggccccagtg gattatcttc ttaattcatt ttaccttgtt cgaggtgata 300
   gcttgtgtct ctgtatataa atataaattg ggggttcgtg tcactggaaa atttatcata 360
   tgattcgaga ttataacatg tctatcgtct caattaattt ttacatgata tatgactata 420
   aatgtaaatt tacgggaaaa agttaataaa atttagtatc acttaaagat tggttaaaat 480
   tataataatt ttgaatcata taatattttc tttctctttt gtgattgtga catgcttaac 540
   ttgtaataca atttcattca aagttattta tttgttgttt tgcagaatgc attccaaata 600
   gcctttttct tgtggacatg ggtaagtaac ccatataacc gagatattta tttattatga 660
   gaaattttaa gttttggggt ttcaaattta gaggattcat taattttaat gatgttttca 720
   aagtgcagta tgagtttaag atcacatctt gcttccatga aaacttgccc ctaatactga 780
   caagggttgt ccttgggata gccttacaag tcgtatgcag ttacatcaca ttccctctct 840
   attccctagt aacacaggta tataatgtca aactaaccat caaattactt aattaagaca 900
   ttattatcct ttttttgttg cacaatgatc acattcagat tcaatcgtat gaccttatgt 960
   atacaatcct aattcttcac cactagaccg actctagtgg ttataaatta tttcccttct 1020
   cttactatat atatatatta ccttgaagat gggatctcac atgaagaaaa caatctttga 1080
   ggagcaaact gcaaaggcac ttaagaaatg gcaaaaggct gcaaaggaca aga 1133
<210> 6
   <211> 458
   <212> DNA
   <213> Glycine max
<400> 6
   cacgctcact tggctatcat tagcaccact agtgatactt ctattagtgg gcaccaagct     60
   tgaacttata atcatggaaa tggcccaaca aatccaagac cgagccacaa ttgttagagg    120
   agtccctatt gtagagccaa acaacaagta tttttggttt aaccggcccc agtggattat    180
   cttcttaatt cattttacct tgttcgagaa tgcattccaa atagcctttt tcttgtggac    240
   atggtatgag tttaagatca catcttgctt ccatgaaaac ttgcccctaa tactgacaag    300
   ggttgtcctt gggatagcct tacaagtcgt atgcagttac atcacattcc ctctctattc    360
   cctagtaaca cagatgggat ctcacatgaa gaaaacaatc tttgaggagc aaactgcaaa    420
   ggcacttaag aaatggcaaa aggctgcaaa ggacaaga                            458
<210> 7
   <211> 152
   <212> PRT
   <213> Glycine max
<400> 7
   Thr Leu Thr Trp Leu Ser Leu Ala Pro Leu Val Ile Leu Leu Leu Val
   1               5                   10                  15
   Gly Thr Lys Leu Glu Leu Ile Ile Met Glu Met Ala Gln Gln Ile Gln
               20                  25                  30
   Asp Arg Ala Thr Ile Val Arg Gly Val Pro Ile Val Glu Pro Asn Asn
           35                  40                  45
   Lys Tyr Phe Trp Phe Asn Arg Pro Gln Trp Ile Ile Phe Leu Ile His
       50                  55                  60
   Phe Thr Leu Phe Glu Asn Ala Phe Gln Ile Ala Phe Phe Leu Trp Thr
   65                  70                  75                  80
   Trp Tyr Glu Phe Lys Ile Thr Ser Cys Phe His Glu Asn Leu Pro Leu
                   85                  90                  95
   Ile Leu Thr Arg Val Val Leu Gly Ile Ala Leu Gln Val Val Cys Ser
               100                 105                 110
   Tyr Ile Thr Phe Pro Leu Tyr Ser Leu Val Thr Gln Met Gly Ser His
           115                 120                 125
   Met Lys Lys Thr Ile Phe Glu Glu Gln Thr Ala Lys Ala Leu Lys Lys
       130                 135                 140
   Trp Gln Lys Ala Ala Lys Asp Lys
   145                 150
<210> 8
   <211> 1905
   <212> DNA
   <213> Glycine max
<400> 8
   ttttctcctt cattctagtc tagtcttttt cttctttttt tcccccatgt atagctccaa 60
   gttcagaaag ctgttttgtt ctgtgttgtt ttcatggctc tgttttggag gtttggccat 120
   ggcagcaggt gaaagtagca gcagctccag agacctagac cagacaccaa cgtgggccgt 180
   tgctgctgtc tgtactgttt tcatcttggt atccatagca ctcgaaaaga gtctccacaa 240
   agttgggacg tggcttggac aaaagaaaaa gaaggctttg cttgaagctc tggagaaggt 300
   caaggctgag ttgatgattt taggtttcat ttcactgctt ttgactttcg ggcagagtta 360
   cattgtcaga atatgtattc ccgaaaagct ggcagacaat atgttaccat gtccgtataa 420
   atataaggag gacaaaaagg catcagatag tgaagaggaa catcgtagga aacttttatc 480
   ttatgaacgt agatatttag ctgctgatac tacctcgttc aaatgcagca gggagggaca 540
   cgagccactt ttatctgtca atggattgca ccagttacac atcctcgtat tcttcttagc 600
   agtcattcat gtgctttaca gtgctattac aatgatgctt ggaagactaa agatacgtgg 660
   atggaaggca tgggaagcgg agacttcaac tcataattat gagttcgcca atgctgcttc 720
   aagatttaga cttactcatg aaacatcatt cgtgagagcc cattccagtt ttttgactag 780
   gattcccatc ttcttctaca ttcgctgctt ctttaggcag ttctataggt ctgtaaataa 840
   gactgactac ctcactttgc gcaatgggtt tatcactgtc cacctggctc ctggaagtaa 900
   atttaatttc caaaagtata tcaaaagatc attagaagat gacttcaagg tggtcgtggg 960
   agttagtcct atcctctggg catcagttgt agtttacctt ctcatcaatg ttaatggatg 1020
   gcacaccgta ctttgggcag ccttaattcc tgttgttata attttggctg ttggaacaaa 1080
   acttcaagcc atattggcaa atatggctct tgaaatcacg gaaagacatg cagttgtcca 1140
   aggaatgcct cttgtccaag gctcagacaa atacttttgg tttggtcagc cacagttagt 1200
   tctacatctt atccattttg ctttgttcca gaatgcgttc caaataacat atatcttgtg 1260
   gatatggtat tcttttgggt tgagaaactg tttccgtact gactacaagc ttgcagtagt 1320
   aaaagtagct ctagggattt tgatgctatg cctctgcagc tatatcaccc ttccattata 1380
   tgctcttgta actcagatgg gttcaaggat gaaaacagca atatttgacg agcaaacaaa 1440
   caaggctctg aagaaatggc acatggctgc gaagaagaag cagggaggag cagtgacgct 1500
   aggaaagtcg agtgcacgaa tcatggatgg aagccccatt ggtaattctt caacagtgca 1560
   ctccactggc cccacactac accgtttcaa aactactggc cactcaaccc gctcctcatc 1620
   aacagcgtac gaggatcaag atcaagatca tgaatatgaa tccgatggtg ttgagttgtc 1680
   tccgttggcg tcgcaaacaa caagcttcat tgtaagagtt gatcatggcg accaacaaca 1740
   agcagaacat agacaagata gtgagggaga aaccaacagt agtagtgaag gtgaattctc 1800
   atttgtcaaa cctgaccctg tggaaattag aaccaccaca tagcatatga tcatatattc 1860
   atctctattc ttatacataa atctttacat aaaaaaaaaa aaaaa 1905
<210> 9
   <211> 598
   <212> PRT
   <213> Glycine max
<400> 9
   Met Tyr Ser Ser Lys Phe Arg Lys Leu Phe Cys Ser Val Leu Phe Ser
   1 5 10 15
   Trp Leu Cys Phe Gly Gly Leu Ala Met Ala Ala Gly Glu Ser Ser Ser
   20 25 30
   Ser Ser Arg Asp Leu Asp Gln Thr Pro Thr Trp Ala Val Ala Ala Val
   35 40 45
   Cys Thr Val Phe Ile Leu Val Ser Ile Ala Leu Glu Lys Ser Leu His
   50 55 60
   Lys Val Gly Thr Trp Leu Gly Gln Lys Lys Lys Lys Ala Leu Leu Glu
   65 70 75 80
   Ala Leu Glu Lys Val Lys Ala Glu Leu Met Ile Leu Gly Phe Ile Ser
   85 90 95
   Leu Leu Leu Thr Phe Gly Gln Ser Tyr Ile Val Arg Ile Cys Ile Pro
   100 105 110
   Glu Lys Leu Ala Asp Asn Met Leu Pro Cys Pro Tyr Lys Tyr Lys Glu
   115 120 125
   Asp Lys Lys Ala Ser Asp Ser Glu Glu Glu His Arg Arg Lys Leu Leu
   130 135 140
   Ser Tyr Glu Arg Arg Tyr Leu Ala Ala Asp Thr Thr Ser Phe Lys Cys
   145 150 155 160
   Ser Arg Glu Gly His Glu Pro Leu Leu Ser Val Asn Gly Leu His Gln
   165 170 175
   Leu His Ile Leu Val Phe Phe Leu Ala Val Ile His Val Leu Tyr Ser
   180 185 190
   Ala Ile Thr Met Met Leu Gly Arg Leu Lys Ile Arg Gly Trp Lys Ala
   195 200 205
   Trp Glu Ala Glu Thr Ser Thr His Asn Tyr Glu Phe Ala Asn Ala Ala
   210 215 220
   Ser Arg Phe Arg Leu Thr His Glu Thr Ser Phe Val Arg Ala His Ser
   225 230 235 240
   Ser Phe Leu Thr Arg Ile Pro Ile Phe Phe Tyr Ile Arg Cys Phe Phe
   245 250 255
   Arg Gln Phe Tyr Arg Ser Val Asn Lys Thr Asp Tyr Leu Thr Leu Arg
   260 265 270
   Asn Gly Phe Ile Thr Val His Leu Ala Pro Gly Ser Lys Phe Asn Phe
   275 280 285
   Gln Lys Tyr Ile Lys Arg Ser Leu Glu Asp Asp Phe Lys Val Val Val
   290 295 300
   Gly Val Ser Pro Ile Leu Trp Ala Ser Val Val Val Tyr Leu Leu Ile
   305 310 315 320
   Asn Val Asn Gly Trp His Thr Val Leu Trp Ala Ala Leu Ile Pro Val
                   325                 330                 335
   Val Ile Ile Leu Ala Val Gly Thr Lys Leu Gln Ala Ile Leu Ala Asn
               340                 345                 350
   Met Ala Leu Glu Ile Thr Glu Arg His Ala Val Val Gln Gly Met Pro
           355                 360                 365
   Leu Val Gln Gly Ser Asp Lys Tyr Phe Trp Phe Gly Gln Pro Gln Leu
       370                 375                 380
   Val Leu His Leu Ile His Phe Ala Leu Phe Gln Asn Ala Phe Gln Ile
   385                 390                 395                 400
   Thr Tyr Ile Leu Trp Ile Trp Tyr Ser Phe Gly Leu Arg Asn Cys Phe
                   405                 410                 415
   Arg Thr Asp Tyr Lys Leu Ala Val Val Lys Val Ala Leu Gly Ile Leu
               420                 425                 430
   Met Leu Cys Leu Cys Ser Tyr Ile Thr Leu Pro Leu Tyr Ala Leu Val
           435                 440                 445
   Thr Gln Met Gly Ser Arg Met Lys Thr Ala Ile Phe Asp Glu Gln Thr
       450                 455                 460
   Asn Lys Ala Leu Lys Lys Trp His Met Ala Ala Lys Lys Lys Gln Gly
   465                 470                 475                 480
   Gly Ala Val Thr Leu Gly Lys Ser Ser Ala Arg Ile Met Asp Gly Ser
                   485                 490                 495
   Pro Ile Gly Asn Ser Ser Thr Val His Ser Thr Gly Pro Thr Leu His
               500                 505                 510
   Arg Phe Lys Thr Thr Gly His Ser Thr Arg Ser Ser Ser Thr Ala Tyr
           515                 520                 525
   Glu Asp Gln Asp Gln Asp His Glu Tyr Glu Ser Asp Gly Val Glu Leu
       530                 535                 540
   Ser Pro Leu Ala Ser Gln Thr Thr Ser Phe Ile Val Arg Val Asp His
   545                 550                 555                 560
   Gly Asp Gln Gln Gln Ala Glu His Arg Gln Asp Ser Glu Gly Glu Thr
                   565                 570                 575
   Asn Ser Ser Ser Glu Gly Glu Phe Ser Phe Val Lys Pro Asp Pro Val
               580                 585                 590
   Glu Ile Arg Thr Thr Thr
   595
<210> 10
   <211> 1179
   <212> DNA
   <213> Glycine max
<400> 10
   gctgttggaa caaaacttca agccatactg gcaaatatgg ctcttgaaat cacagaaaga 60
   catgcagttg tccaaggaat gcctcttgtc caaggctcag acaaatactt ttggtttggt 120
   cagccacagt tagttcttca tcttatccat tttgctttgt tccagaatgc gttccaaata 180
   acatatatct tgtggatatg ggtaatgaag ttcattacac tatcttgact attggctaat 240
   gagttttgct tgtctatatt cctgaataat aaaagaacaa ttttttattt gcagtattct 300
   tttgggctga gaaattgttt ccatgctgac tacaagcttg caatagtgaa agtagcttta 360
   gggcttgggg tgctatgcct ctgcagctac atcacccttc cattatatgc tcttgttact 420
   cagatgggct cgagaatgaa aaaatcaata tttgacgaac aaacgtcgaa ggcgttgaag 480
   aaatggcaca tggctgtgaa aaagaagcaa ggagtgaaac ttggaaattc caaggtgcga 540
   gccctggatg gaagcaccac tgattcaaca atacactctt ctggccccac actacaccga 600
   tacaaaacta ccggtcactc aactcacttc acaccaaact atgatgacca agatgattac 660
   cattctgaca ctgagttgtc tcccatttca ccaacagcaa acttgatagt aagagtggac 720
   catgatgagg aagaagcaaa agaaaatgaa cacccaacag ccaacaatca agaggtgcct 780
   ttacgtttga caagcctgga aagaagcatg aaatagcata agaagttact ctctattcaa 840
   ccatttttgg ttcttttact taatatatct tgaccttgtt tagagctttc tagatattgt 900
   tatacttgat aatgatatag tcagttttgt atattcttgt tcttaaaaca cttgccggca 960
   ttggaaagtt tcaccttgct caaaatcatg cttactagct ttgattcatg tgcttgagaa 1020
   aaaggctcat aaggagcaag tagaggacag aattgttggg tatattatgt tatatgttca 1080
   aagtgtgtta cataaatccc aattatataa gctttctggt caagtacaac aaatagaaaa 1140
   atatacattc agttaaaaaa aaaaaaaaaa aaaaaaaaa 1179
<210> 11
   <211> 240
   <212> PRT
   <213> Glycine max
<400> 11
   Ala Val Gly Thr Lys Leu Gln Ala Ile Leu Ala Asn Met Ala Leu Glu
   1               5                   10                  15
   Ile Thr Glu Arg His Ala Val Val Gln Gly Met Pro Leu Val Gln Gly
               20                  25                  30
   Ser Asp Lys Tyr Phe Trp Phe Gly Gln Pro Gln Leu Val Leu His Leu
           35                  40                  45
   Ile His Phe Ala Leu Phe Gln Asn Ala Phe Gln Ile Thr Tyr Ile Leu
       50                  55                  60
   Trp Ile Trp Tyr Ser Phe Gly Leu Arg Asn Cys Phe His Ala Asp Tyr
   65                  70                  75                  80
   Lys Leu Ala Ile Val Lys Val Ala Leu Gly Leu Gly Val Leu Cys Leu
                   85                  90                  95
   Cys Ser Tyr Ile Thr Leu Pro Leu Tyr Ala Leu Val Thr Gln Met Gly
               100                 105                 110
   Ser Arg Met Lys Lys Ser Ile Phe Asp Glu Gln Thr Ser Lys Ala Leu
           115                 120                 125
   Lys Lys Trp His Met Ala Val Lys Lys Lys Gln Gly Val Lys Leu Gly
       130                 135                 140
   Asn Ser Lys Val Arg Ala Leu Asp Gly Ser Thr Thr Asp Ser Thr Ile
   145                 150                 155                 160
   His Ser Ser Gly Pro Thr Leu His Arg Tyr Lys Thr Thr Gly His Ser
                   165                 170                 175
   Thr His Phe Thr Pro Asn Tyr Asp Asp Gln Asp Asp Tyr His Ser Asp
               180                 185                 190
   Thr Glu Leu Ser Pro Ile Ser Pro Thr Ala Asn Leu Ile Val Arg Val
           195                 200                 205
   Asp His Asp Glu Glu Glu Ala Lys Glu Asn Glu His Pro Thr Ala Asn
       210                 215                 220
   Asn Gln Glu Val Pro Leu Arg Leu Thr Ser Leu Glu Arg Ser Met Lys
   225                 230                 235                 240
<210> 12
   <211> 813
   <212> DNA
   <213> Oryza sativa
<400> 12
   cctcgatgca ataactaatt taactataat tgatttttct tgggttttct gcagggcaag 60
   gtggcgctga tgtcggcaaa gagcatgcac cagctgcaca ttttcatctt cgtgctcgcc 120
   gtgttccatg ttacctactg cgtcatcacc atgggtttag ggcgcctcaa agtgagtttg 180
   tcgttctgtc cctcatgcac atgttttctc tagttctagc aagattgtca gtccttcaaa 240
   tggattgttt cgacaagaaa cccaatttat taatttgcca gtaaatatat aataattggt 300
   ctttcttggt tttagatgaa gaaatgggaa gaagtgggag tcacaagacc aactcattgg 360
   agtaccaagt tcgcaatcgg tagtgaatta agaatctccc taactatttc atttcagaac 420
   ctttatgata atgtcttgga agaggaggag caaatcagct gaaaatatga tcgatccatg 480
   cagatccttc acgattcagg ttcacgcatc agacgtcgtt cgtgaagcgg catctgggat 540
   cattctcaag cacccctggg ctcagatgga tcgtgagtta tcaatctccg aatacatgct 600
   tgttttttat tcttgcaact ggcctagctg ttccaattca atccatattt tttgaaaaaa 660
   aaaaatattc atgccgtgtt tgttgttagg tagcattctt caggcagttc tttgggtccg 720
   tcaccaaggt ggactacctg accatgcggc aaggcttcat caatgtatat actaatcaaa 780
   cctgaccaat tcaacattga tgatgcaaac aga 813
<210> 13
   <211> 5277
   <212> DNA
   <213> Oryza sativa
<400> 13
   atggcaggtg ggagatcggg atcgcgggag ttgccggaga cgccgacgtg ggcggtggcc    60
   gtcgtctgcg ccgtcctcgt gctcgtctcc gtcgccatgg agcacggcct ccacaacctc   120
   agccatgtac ccgcgcgcgc acgcggtgtg ctcatctctc gagttaattt ggttgttgtt   180
   gttgttgtgt tcttgtgaca tctcaattaa catccgatcc tggtcgatcg atcgccctgt   240
   ggtggcgcta ctgcttgcat tgcagtggtt ccgtaggcgg cagaagaagg ccatgggcga   300
   cgccctcgac aagatcaaag caggtcaccc tcagcctcag ctcaccctca gctagctctt   360
   actccctcca tccctaaatg tttgacgccg ttgacttttt taaatatgtt tgaccgttcg   420
   tcttattcaa aaaatttaag taattattaa ttctttttct accatttgat tcattgctaa    480
   atatactatt atgtatacat atagttttac atatttcacc aaagttttta aataagacga   540
   atggtcaaac atgtttaaaa cagtcaatgg catcaaacat ttagggaaga agggaatatt   600
   atattgctgc tcccctctag ccactttgct gcctccctcg tcattttttc aagtatttta    660
   cgcaagactg ggtcctccaa atcaaacgtc acaaataagc cgtttatagt ttcctttcgc   720
   tttttaaggg gggactactt gtatttaatc atggaggaaa ctaccagtcg gatgtccgat   780
   tacttaaaaa aaattcgggt gactaatttt tttggttgat catcggtgaa atattaggtt   840
   atatatgtta aaaaaaatca gccacaaaca atgaaatatt ttgtgaaaca catattagac   900
   acgttgaaac gtatcattgt tacgtataaa acatctaatg ttaacagatt aaaacatatg   960
   tttttctttc atcagaatat aatcatgcga tatattattg taaagatata attacaacga  1020
   atacaacggt gtgatcggat tatatatata tattagtagt ttaagagaaa aatcattttg  1080
   aagattacta gatacataca cgtatagatg gatgaagtgg agagagatta gagataagta  1140
   gttatatgaa ttttgtgaaa cacacttaag acatatgttc aaacatactg ctattatgta  1200
   tgaaatattg agttttaacg gtttaaaaca catattcttt taattagaat gtaataatgt  1260
   gatatcttgt tgtaaaattt aattacatct aatataacgg tgtgattaga ttgtatgttg  1320
   gataacatgc ccatcggttg gcttattcag ggaataagcc aaacggtata tttgcaaacg  1380
   aaaaataatt tgtaaataaa acttttatgt atgtgttctt aacgatctag cagcaaaggc  1440
   tgaaaaataa acttcgatga aaaatctcaa aatcaactct taaaattcaa attttggctt 1500
   ataagtatag ttcctaacta gtttagaaga aaaaatattt aaagcgggga agaggaaaag 1560
   gaataaacta atagctaaat tattgcatgc atgtagcgat ttgaggacga ccgagttgtt 1620
   ttgtctggat cagccgaccg agacagagca atcttcttta atcataaatg accagaaaaa 1680
   ccataccagt tcatcacaat ggaccgagtc agagtcatta catatttttc attgttgcgc 1740
   acaggattca ccatgttctt atgggaaata tttttaactc tcaaatggtt atgattttga 1800
   actctcattt ttgagagaga attaacaagc gagcgagcaa tcaggccaaa aaggagaaag 1860
   aaaaattatt tttgttaatt tgttttttta aggtagggtg gaggagtcat tacatgattt 1920
   ttttttatat tccctcgttg attatatgct gttcaaatgg ttatgatttt tttaaaaaat 1980
   aacaacaata caaattagta tgtgatagat catttcacga gcatgtagga ttaaatttaa 2040
   cttctgtaaa ttacaaaaca aacaagttta actgttaata tacattaaat ttgttttttt 2100
   tcaacttagg aattgaattt tatgtatata tttgtaaaat gatatattaa tttatttttt 2160
   taaaaaaata attatttaga tggcacacaa actagaaaac caccgcagtt ctcatatttc 2220
   ttgtcctatc tgcacttgca gagctgatgc tgctgggctt catatccctg cttctcaccg 2280
   tggcacaggc gcccatctcc aagatctgca tccccaagtc ggctgccaac atcttgttgc 2340
   cgtgcaaggc aggccaagat gccatcgaag aagaagcagc aagtgatcgc cggtccttgg 2400
   ccggcgccgg cggcggggac tactgctcga aattcgatgt gagaataaca ccagctgccg 2460
   gcaagcacaa cctcgatgca ataactaatt taactataat tgatttttct tgggttttct 2520
   gcagggcaag gtggcgctga tgtcggcaaa gagcatgcac cagctgcaca ttttcatctt 2580
   cgtgctcgcc gtgttccatg ttacctactg cgtcatcacc atgggtttag ggcgcctcaa 2640
   agtgagtttg tcgttctgtc cctcatgcac atgttttctc tagttctagc aagattgtca 2700
   gtccttcaaa tggattgttt cgacaagaaa cccaatttat taatttgcca gtaaatatat 2760
   aataattggt ctttcttggt tttagatgaa gaaatggaag aagtgggagt cacagaccaa 2820
   ctcattggag tatcagttcg caatcggtag tgaattaaga atctccctaa ctatttcatt 2880
   tcagaacctt tatgataatg tcttgaaaga ggaggagcaa atcagctgaa aaatatgatc 2940
   gatccatgca gatccttcac gattcaggtt cacgcatcag acgtcgttcg tgaagcggca 3000
   tctgggatca ttctcaagca cccctgggct cagatggatc gtgagttatc aatctccgaa 3060
   tacatgcttg ttttttattc ttgcaactgg cctagctgtt ccaattcaat ccatattttt 3120
   tgaaaaaaaa aatattcatg ccgtgtttgt tgttaggtag cattcttcag gcagttcttt 3180
   gggtccgtca ccaaggtgga ctacctgacc atgcggcaag gcttcatcaa tgtatatact 3240
   aatcaaacct gaccaattca acattgatga tgcaaacaga gaccaggttt ttttttcgag 3300
   tgtgcattga gtaatggttt tagcttcttc tcttttgcag gcgcatttgt cgcagaatag 3360
   caagttcgac ttccacaaat acatcaagag gtctttggag gacgacttca aagttgtcgt 3420
   tggcatcagg tccgtcctcg ctttattaat tataggactc ttatattcaa catttttttt 3480
   ataaagaaac atatttagtc tccagttgtg tatgtgtatg tggatcttga cacatttggc 3540
   tggttttgca gcctccctct gtggttcgtc ggaatccttg tactcttcct cgatatccac 3600
   ggtaatcctt gtcctatttc attctgtttt ttactctcaa aaccttgttc tgaattggtc 3660
   ttataatcac catcgatttt ttttcaactt tttccccgcg tgtaggtctt ggcacactta 3720
   tttggatctc ttttgttcct ctcatcgtaa gagcgaaatt tccctgtcca aagaaacagt 3780
   taacataatt aattatgctt taatttatca tgaaaattaa tatgatcata taactaatga 3840
   acaaacattc atgtgaatgc caccgttgtc tcagatcgtc ttgttagttg ggaccaagct 3900
   agagatggtg atcatgcaga tggcccaaga gatacaggac agggccactg tgatccaggg 3960
   agcacctgtg gttgaaccaa gcaacaagta cttctggttc aaccgccctg actgggtctt 4020
   gttcttcata cacctgacac tcttccatgt acatgtttaa aacctaaacc ttgctgctca 4080
   actacaaata gtactttatc tttcacaatt aacacctaat taactaacat agcatccatc 4140
   catttgtggc tactgatcga tgggacgacg gatcgatgat caccagaacg catttcagat 4200
   ggcgcatttc gtatggacta tggtgtgtat gctacttgct tagttgttgc cattatcagt 4260
   tcttaagcaa attaagtgtg atgcatgcac tgactaatga gacaaaaaat gacacagctt 4320
   gttcatcgat ctggttgttt tgtgtgtgac aggcaacacc tggtctgaag aaatgcttcc 4380
   atgaaaatat ttggctgagc atcgtggaag tcattgtggg gatctctctt caggtgctat 4440
   gcagctacat caccttcccg ctctacgcgc tcgtcacaca ggtgaacaag ccattcacaa 4500
   attctattag cagtttctta attgacgacg ctgttaattt ttagacacac gttttgacca 4560
   tttgtcttat taaaaatatt tatgtaatta tcatttgagt tgttttatca ctaaaagtac 4620
   tttttaaata atttatattt tgcatttgta caattctttt aataagataa tggtcaaaca 4680
   agtgtccaaa agttaacagc atcatctatt aagaaaagga gggggttttt ttttggaatt 4740
   ttgcaaaatt tgttcaaaat cagtccaaaa cctttttttt gtttcgaaat ttcagtttca 4800
   ctaccagtcc ccataaaatg tcttttcttt atttccacaa tattgaaccc atgagatgcc 4860
   ctttgtgttg gtatgtgttt tggccatcac ttgcagatgg gatcgaacat gaagaagaca 4920
   atcttcgagg agcaaacgat gaaggcgctg atgaactgga ggaagaaggc gatggagaag 4980
   aagaaggtcc gggacgcgga cgcgttcctg gcgcagatga gcgtcgactt cgcgacgccg 5040
   gcgtcgagcc ggtccgcgtc gccggtgcac ctgctgcagg atcacagggc gaggtcggac 5100
   gacccgccga gcccaatcac ggtggcctca ccaccggcac cggaggagga catatacccg 5160
   gtgccggcgg cggctgcgtc tcgccagctg ctagacgacc cgccggacag gaggtggatg 5220
   gcatcctcgt cggccgacat cgccgattct gatttttcct tcagcgcaca acggtga 5277
<210> 14
   <211> 540
   <212> PRT
   <213> Oryza sativa
<400> 14
   Met Ala Gly Gly Arg Ser Gly Ser Arg Glu Leu Pro Glu Thr Pro Thr
   1               5                   10                  15
   Trp Ala Val Ala Val Val Cys Ala Val Leu Val Leu Val Ser Val Ala
               20                  25                  30
   Met Glu His Gly Leu His Asn Leu Ser His Trp Phe Arg Arg Arg Gln
           35                  40                  45
   Lys Lys Ala Met Gly Asp Ala Leu Asp Lys Ile Lys Ala Glu Leu Met
       50                  55                  60
   Leu Leu Gly Phe Ile Ser Leu Leu Leu Thr Val Ala Gln Ala Pro Ile
   65                  70                  75                  80
   Ser Lys Ile Cys Ile Pro Lys Ser Ala Ala Asn Ile Leu Leu Pro Cys
                   85                  90                  95
   Lys Ala Gly Gln Asp Ala Ile Glu Glu Glu Ala Ala Ser Asp Arg Arg
               100                 105                 110
   Ser Leu Ala Gly Ala Gly Gly Gly Asp Tyr Cys Ser Lys Phe Asp Gly
           115                 120                 125
   Lys Val Ala Leu Met Ser Ala Lys Ser Met His Gln Leu His Ile Phe
       130                 135                 140
   Ile Phe Val Leu Ala Val Phe His Val Thr Tyr Cys Val Ile Thr Met
   145                 150                 155                 160
   Gly Leu Gly Arg Leu Lys Met Lys Lys Trp Lys Lys Trp Glu Ser Gln
                   165                 170                 175
   Thr Asn Ser Leu Glu Tyr Gln Phe Ala Ile Asp Pro Ser Arg Phe Arg
               180                 185                 190
   Phe Thr His Gln Thr Ser Phe Val Lys Arg His Leu Gly Ser Phe Ser
           195                 200                 205
   Ser Thr Pro Gly Leu Arg Trp Ile Val Ala Phe Phe Arg Gln Phe Phe
       210                 215                 220
   Gly Ser Val Thr Lys Val Asp Tyr Leu Thr Met Arg Gln Gly Phe Ile
   225                 230                 235                 240
   Asn Val Tyr Thr Asn Gln Asn Ser Lys Phe Asp Phe His Lys Tyr Ile
                   245                 250                 255
   Lys Arg Ser Leu Glu Asp Asp Phe Lys Val Val Val Gly Ile Ser Leu
               260                 265                 270
   Pro Leu Trp Phe Val Gly Ile Leu Val Leu Phe Leu Asp Ile His Gly
           275                 280                 285
   Leu Gly Thr Leu Ile Trp Ile Ser Phe Val Pro Leu Ile Ile Val Leu
       290                 295                 300
   Leu Val Gly Thr Lys Leu Glu Met Val Ile Met Gln Met Ala Gln Glu
   305                 310                 315                 320
   Ile Gln Asp Arg Ala Thr Val Ile Gln Gly Ala Pro Val Val Glu Pro
                   325                 330                 335
   Ser Asn Lys Tyr Phe Trp Phe Asn Arg Pro Asp Trp Val Leu Phe Phe
               340                 345                 350
   Ile His Leu Thr Leu Phe His Asn Ala Phe Gln Met Ala His Phe Val
           355                 360                 365
   Trp Thr Met Ala Thr Pro Gly Leu Lys Lys Cys Phe His Glu Asn Ile
       370                 375                 380
   Trp Leu Ser Ile Val Glu Val Ile Val Gly Ile Ser Leu Gln Val Leu
   385                 390                 395                 400
   Cys Ser Tyr Ile Thr Phe Pro Leu Tyr Ala Leu Val Thr Gln Met Gly
                   405                 410                 415
   Ser Asn Met Lys Lys Thr Ile Phe Glu Glu Gln Thr Met Lys Ala Leu
               420                 425                 430
   Met Asn Trp Arg Lys Lys Ala Met Glu Lys Lys Lys Val Arg Asp Ala
           435                 440                 445
   Asp Ala Phe Leu Ala Gln Met Ser Val Asp Phe Ala Thr Pro Ala Ser
       450                 455                 460
   Ser Arg Ser Ala Ser Pro Val His Leu Leu Gln Asp His Arg Ala Arg
   465                 470                 475                 480
   Ser Asp Asp Pro Pro Ser Pro Ile Thr Val Ala Ser Pro Pro Ala Pro
                   485                 490                 495
   Glu Glu Asp Ile Tyr Pro Val Pro Ala Ala Ala Ala Ser Arg Gln Leu
               500                 505                 510
   Leu Asp Asp Pro Pro Asp Arg Arg Trp Met Ala Ser Ser Ser Ala Asp
           515                 520                 525
   Ile Ala Asp Ser Asp Phe Ser Phe Ser Ala Gln Arg
       530                 535                 540
<210> 15
   <211> 653
   <212> DNA
   <213> Linum usitatissimum
<400> 15
   acgaattcga gctcggtacc cgatcatagc ggcacattta gctcctggaa gtgagagcag      60
   attcgacttc cagaaatacg tcaacagatc acttgaggat gacttcaaag ttgttgtagg     120
   gatcagtccg attctatggt tttttgcagt cttgttcctg ctatccaata cacatggatg     180
   ggtagcatat ctgtggctac cctttattcc actaatcata atcctggttg ttggtacaaa     240
   gctacaagtg atcataaccc agctaggact aagcatacaa gacagaggag atgtggtgaa     300
   gggtgcccct gtggttcagc caggagatga cctcttctgg tttggacgcc ctcgtctcgt     360
   tctcttcctc atccatttct gcctcttcca gaatgcattt caacttgctt tcttcatatg     420
   gagtgtgtat gaattcggaa tcaaaacttg cttccacgag aaaaccgagg acattgtcag     480
   agcttctgga ttggttccga accgtgatac atcagcaaca caaaccacgg agctgtccaa     540
   aggcaagctg atgatggctg atacttgcct tcctaccgaa gatttggtag ggatggtagt     600
   aacagctgct cattctggga aaaggttttt cgtagattct atacgctatg atc           653
<210> 16
   <211> 217
   <212> PRT
   <213> Linum usitatissimum
<400> 16
   Arg Ile Arg Ala Arg Tyr Pro Ile Ile Ala Ala His Leu Ala Pro Gly
   1               5                   10                  15
   Ser Glu Ser Arg Phe Asp Phe Gln Lys Tyr Val Asn Arg Ser Leu Glu
               20                  25                  30
   Asp Asp Phe Lys Val Val Val Gly Ile Ser Pro Ile Leu Trp Phe Phe
           35                  40                  45
   Ala Val Leu Phe Leu Leu Ser Asn Thr His Gly Trp Val Ala Tyr Leu
       50                  55                  60
   Trp Leu Pro Phe Ile Pro Leu Ile Ile Ile Leu Val Val Gly Thr Lys
   65                  70                  75                  80
   Leu Gln Val Ile Ile Thr Gln Leu Gly Leu Ser Ile Gln Asp Arg Gly
                   85                  90                  95
   Asp Val Val Lys Gly Ala Pro Val Val Gln Pro Gly Asp Asp Leu Phe
               100                 105                 110
   Trp Phe Gly Arg Pro Arg Leu Val Leu Phe Leu Ile His Phe Cys Leu
           115                 120                 125
   Phe Gln Asn Ala Phe Gln Leu Ala Phe Phe Ile Trp Ser Val Tyr Glu
       130                 135                 140
   Phe Gly Ile Lys Thr Cys Phe His Glu Lys Thr Glu Asp Ile Val Arg
   145                 150                 155                 160
   Ala Ser Gly Leu Val Pro Asn Arg Asp Thr Ser Ala Thr Gln Thr Thr
                   165                 170                 175
   Glu Leu Ser Lys Gly Lys Leu Met Met Ala Asp Thr Cys Leu Pro Thr
               180                 185                 190
   Glu Asp Leu Val Gly Met Val Val Thr Ala Ala His Ser Gly Lys Arg
           195                 200                 205
   Phe Phe Val Asp Ser Ile Arg Tyr Asp
       210                 215
<210> 17
   <211> 1239
   <212> DNA
   <213> Triticum aestivum
<400> 17
   atggcggacg acgacgagta ccccccagcg aggacgctgc cggagacgcc gtcctgggcg     60
   gtggccctcg tcttcgccgt catgatcatc gtgtccgtcc tcctggagca cgcgctccat    120
   aagctcggcc attggttcca caagcggcac aagaacgcgc tggcggaggc gctggagaag    180
   atcaaggcgg agctcatgct ggtgggcttc atctcgctgc tgctcgccgt gacgcaggac    240
   cccatctccg ggatatgcat ctccgagaag gccgccagca tcatgcggcc ctgcaagctg    300
   ccccctggct ccgtcaagag caagtacaaa gactactact gcgccaaaca gggcaaggtg    360
   tcgctcatgt ccacgggcag cttgcaccag ctgcacatat tcatcttcgt gctcgccgtc    420
   ttccatgtca cctacagcgt catcatcatg gctctaagcc gtctcaaaat gagaacctgg    480
   aagaaatggg agacagagac cgcctccctg gaataccagt tcgcaaatga tcctgcgcgg    540
   ttccgcttca cgcaccagac gtcgttcgtg aagcggcacc tgggcctctc cagcaccccc    600
   ggcgtcagat gggtggtggc cttcttcagg cagttcttca ggtcggtcac caaggtggac    660
   tacctcacct tgagggcagg cttcatcaac gcgcatttgt cgcataacag caagttcgac    720
   ttccacaagt acatcaagag gtccatggag gacgacttca aagtcgtcgt tggcatcagc    780
   ctcccgctgt ggtgtgtggc gatcctcacc ctcttccttg acattgacgg gatcggcacg    840
   ctcacctgga tttctttcat ccctctcgtc atcctcttgt gtgttggaac caagctggag    900
   atgatcatca tggagatggc cctggagatc caggaccggg cgagcgtcat caagggggcg    960
   cccgtggttg agcccagcaa caagttcttc tggttccacc gccccgactg ggtcctcttc   1020
   ttcatacacc tgacgctatt ccagaacgcg tttcagatgg cacatttcgt gtggacagtg   1080
   gccacgcccg gcttgaagaa atgcttccat atgcacatcg ggctgagcat catgaaggtc   1140
   gtgctggggc tggctcttca gttcctctgc agctatatca ccttcccgct ctacgcgctc   1200
   gtcacacaga tgggatcaaa catgaagagg tccatcttc                          1239
<210> 18
   <211> 413
   <212> PRT
   <213> Triticum aestivum
<400> 18
   Met Ala Asp Asp Asp Glu Tyr Pro Pro Ala Arg Thr Leu Pro Glu Thr
   1               5                   10                  15
   Pro Ser Trp Ala Val Ala Leu Val Phe Ala Val Met Ile Ile Val Ser
               20                  25                  30
   Val Leu Leu Glu His Ala Leu His Lys Leu Gly His Trp Phe His Lys
           35                  40                  45
   Arg His Lys Asn Ala Leu Ala Glu Ala Leu Glu Lys Ile Lys Ala Glu
       50                  55                  60
   Leu Met Leu Val Gly Phe Ile Ser Leu Leu Leu Ala Val Thr Gln Asp
   65                  70                  75                  80
   Pro Ile Ser Gly Ile Cys Ile Ser Glu Lys Ala Ala Ser Ile Met Arg
                   85                  90                  95
   Pro Cys Lys Leu Pro Pro Gly Ser Val Lys Ser Lys Tyr Lys Asp Tyr
               100                 105                 110
   Tyr Cys Ala Lys Gln Gly Lys Val Ser Leu Met Ser Thr Gly Ser Leu
           115                 120                 125
   His Gln Leu His Ile Phe Ile Phe Val Leu Ala Val Phe His Val Thr
       130                 135                 140
   Tyr Ser Val Ile Ile Met Ala Leu Ser Arg Leu Lys Met Arg Thr Trp
   145                 150                 155                 160
   Lys Lys Trp Glu Thr Glu Thr Ala Ser Leu Glu Tyr Gln Phe Ala Asn
                   165                 170                 175
   Asp Pro Ala Arg Phe Arg Phe Thr His Gln Thr Ser Phe Val Lys Arg
               180                 185                 190
   His Leu Gly Leu Ser Ser Thr Pro Gly Val Arg Trp Val Val Ala Phe
           195                 200                 205
   Phe Arg Gln Phe Phe Arg Ser Val Thr Lys Val Asp Tyr Leu Thr Leu
       210                 215                 220
   Arg Ala Gly Phe Ile Asn Ala His Leu Ser His Asn Ser Lys Phe Asp
   225                 230                 235                 240
   Phe His Lys Tyr Ile Lys Arg Ser Met Glu Asp Asp Phe Lys Val Val
                   245                 250                 255
   Val Gly Ile Ser Leu Pro Leu Trp Cys Val Ala Ile Leu Thr Leu Phe
   260 265 270
   Leu Asp Ile Asp Gly Ile Gly Thr Leu Thr Trp Ile Ser Phe Ile Pro
   275 280 285
   Leu Val Ile Leu Leu Cys Val Gly Thr Lys Leu Glu Met Ile Ile Met
   290 295 300
   Glu Met Ala Leu Glu Ile Gln Asp Arg Ala Ser Val Ile Lys Gly Ala
   305 310 315 320
   Pro Val Val Glu Pro Ser Asn Lys Phe Phe Trp Phe His Arg Pro Asp
   325 330 335
   Trp Val Leu Phe Phe Ile His Leu Thr Leu Phe Gln Asn Ala Phe Gln
   340 345 350
   Met Ala His Phe Val Trp Thr Val Ala Thr Pro Gly Leu Lys Lys Cys
   355 360 365
   Phe His Met His Ile Gly Leu Ser Ile Met Lys Val Val Leu Gly Leu
   370 375 380
   Ala Leu Gln Phe Leu Cys Ser Tyr Ile Thr Phe Pro Leu Tyr Ala Leu
   385 390 395 400
   Val Thr Gln Met Gly Ser Asn Met Lys Arg Ser Ile Phe
   405 410
<210> 19
   <211> 1934
   <212> DNA
   <213> Arabidopsis thaliana
<400> 19
   attccagtgt tggtacataa aagactcttc ctttgtctgt tttttgttcc cagattcatc 60
   tttacttatt gactaaattc tctctggtgt gagaagtaaa atgggtcacg gaggagaagg 120
   gatgtcgctt gaattcactc cgacgtgggt cgtcgccgga gtttgtacgg tcatcgtcgc 180
   gatttcactg gcggtggagc gtttgcttca ctatttcggt actgttctta agaagaagaa 240
   gcaaaaaccc ctttacgaag cccttcaaaa ggttaaagaa gagctgatgt tgttagggtt 300
   tatatcgctg ttactgacgg tattccaagg gctcatttcc aaattctgtg tgaaagaaaa 360
   tgtgcttatg catatgcttc catgttctct cgattcaaga cgagaagctg gggcaagtga 420
   acataaaaac gttacagcaa aagaacattt tcagactttt ttacctattg ttggaaccac 480
   taggcgtcta cttgctgaac atgctgctgt gcaagttggt tactgtagcg aaaagggtaa 540
   agtaccattg ctttcgcttg aggcattgca ccatctacat attttcatct tcgtcctcgc 600
   catatcccat gtgacattct gtgtccttac cgtgattttt ggaagcacaa ggattcacca 660
   atggaagaaa tgggaggatt cgatcgcaga tgagaagttt gaccccgaaa cagctctcag 720
   gaaaagaagg gtcactcatg tacacaacca tgcttttatt aaagagcatt ttcttggtat 780
   tggcaaagat tcagtcatcc tcggatggac gcaatccttt ctcaagcaat tctatgattc 840
   tgtgacgaaa tcagattacg tgactttacg tcttggtttc attatgacac attgtaaggg 900
   aaaccccaag cttaatttcc acaagtatat gatgcgcgct ctagaggatg atttcaaaca 960
   agttgttggt attagttggt atctttggat ctttgtcgtc atctttttgc tgctaaatgt 1020
   taacggatgg cacacatatt tctggatagc atttattccc tttgctttgc ttcttgctgt 1080
   gggaacaaag ttggagcatg tgattgcaca gttagctcat gaagttgcag agaaacatgt 1140
   agccattgaa ggagacttag tggtgaaacc ctcagatgag catttctggt tcagcaaacc 1200
   tcaaattgtt ctctacttga tccattttat cctcttccag aatgcttttg agattgcgtt 1260
   tttcttttgg atttgggtta catacggctt cgactcgtgc attatgggac aggtgagata 1320
   cattgttcca agattggtta tcggggtctt cattcaagtg ctttgcagtt acagtacact 1380
   gcctctttac gccatcgtct cacagatggg aagtagcttc aagaaagcta tattcgagga 1440
   gaatgtgcag gttggtcttg ttggttgggc acagaaagtg aaacaaaaga gagacctaaa 1500
   agctgcagct agtaatggag acgaaggaag ctctcaggct ggtcctggtc ctgattctgg 1560
   ttctggttct gctcctgctg ctggtcctgg tgcaggtttt gcaggaattc agctcagcag 1620
   agtaacaaga aacaacgcag gggacacaaa caatgagatt acacctgatc ataacaactg 1680
   agcagagata ttatcttttc catttagagg atcatcatca gattttagct tcaaggtccg 1740
   gttttgtggt ttatacataa gttatagtga cttgattttt ttgttttgtt acaaagttac 1800
   catctttgga ttagaattgg gaaattgaat ctgtttgtat attgtattat ttggaacatt 1860
   gtggatgccc atggatatgt ttctgttcaa ttattttggt tttgggtaat gaaatttgaa 1920
   accaacgaaa aacc 1934
<210> 20
   <211> 526
   <212> PRT
   <213> Arabidopsis thaliana
<400> 20
   Met Gly His Gly Gly Glu Gly Met Ser Leu Glu Phe Thr Pro Thr Trp
   1               5                   10                  15
   Val Val Ala Gly Val Cys Thr Val Ile Val Ala Ile Ser Leu Ala Val
               20                  25                  30
   Glu Arg Leu Leu His Tyr Phe Gly Thr Val Leu Lys Lys Lys Lys Gln
           35                  40                  45
   Lys Pro Leu Tyr Glu Ala Leu Gln Lys Val Lys Glu Glu Leu Met Leu
       50                  55                  60
   Leu Gly Phe Ile Ser Leu Leu Leu Thr Val Phe Gln Gly Leu Ile Ser
   65 70 75 80
   Lys Phe Cys Val Lys Glu Asn Val Leu Met His Met Leu Pro Cys Ser
   85 90 95
   Leu Asp Ser Arg Arg Glu Ala Gly Ala Ser Glu His Lys Asn Val Thr
   100 105 110
   Ala Lys Glu His Phe Gln Thr Phe Leu Pro Ile Val Gly Thr Thr Arg
   115 120 125
   Arg Leu Leu Ala Glu His Ala Ala Val Gln Val Gly Tyr Cys Ser Glu
   130 135 140
   Lys Gly Lys Val Pro Leu Leu Ser Leu Glu Ala Leu His His Leu His
   145 150 155 160
   Ile Phe Ile Phe Val Leu Ala Ile Ser His Val Thr Phe Cys Val Leu
   165 170 175
   Thr Val Ile Phe Gly Ser Thr Arg Ile His Gln Trp Lys Lys Trp Glu
   180 185 190
   Asp Ser Ile Ala Asp Glu Lys Phe Asp Pro Glu Thr Ala Leu Arg Lys
   195 200 205
   Arg Arg Val Thr His Val His Asn His Ala Phe Ile Lys Glu His Phe
   210 215 220
   Leu Gly Ile Gly Lys Asp Ser Val Ile Leu Gly Trp Thr Gln Ser Phe
   225 230 235 240
   Leu Lys Gln Phe Tyr Asp Ser Val Thr Lys Ser Asp Tyr Val Thr Leu
   245 250 255
   Arg Leu Gly Phe Ile Met Thr His Cys Lys Gly Asn Pro Lys Leu Asn
   260 265 270
   Phe His Lys Tyr Met Met Arg Ala Leu Glu Asp Asp Phe Lys Gln Val
   275 280 285
   Val Gly Ile Ser Trp Tyr Leu Trp Ile Phe Val Val Ile Phe Leu Leu
   290 295 300
   Leu Asn Val Asn Gly Trp His Thr Tyr Phe Trp Ile Ala Phe Ile Pro
   305 310 315 320
   Phe Ala Leu Leu Leu Ala Val Gly Thr Lys Leu Glu His Val Ile Ala
   325 330 335
   Gln Leu Ala His Glu Val Ala Glu Lys His Val Ala Ile Glu Gly Asp
   340 345 350
   Leu Val Val Lys Pro Ser Asp Glu His Phe Trp Phe Ser Lys Pro Gln
   355 360 365
   Ile Val Leu Tyr Leu Ile His Phe Ile Leu Phe Gln Asn Ala Phe Glu
   370 375 380
   Ile Ala Phe Phe Phe Trp Ile Trp Val Thr Tyr Gly Phe Asp Ser Cys
   385 390 395 400
   Ile Met Gly Gln Val Arg Tyr Ile Val Pro Arg Leu Val Ile Gly Val
   405 410 415
   Phe Ile Gln Val Leu Cys Ser Tyr Ser Thr Leu Pro Leu Tyr Ala Ile
   420 425 430
   Val Ser Gln Met Gly Ser Ser Phe Lys Lys Ala Ile Phe Glu Glu Asn
   435 440 445
   Val Gln Val Gly Leu Val Gly Trp Ala Gln Lys Val Lys Gln Lys Arg
   450 455 460
   Asp Leu Lys Ala Ala Ala Ser Asn Gly Asp Glu Gly Ser Ser Gln Ala
   465 470 475 480
   Gly Pro Gly Pro Asp Ser Gly Ser Gly Ser Ala Pro Ala Ala Gly Pro
   485 490 495
   Gly Ala Gly Phe Ala Gly Ile Gln Leu Ser Arg Val Thr Arg Asn Asn
   500 505 510
   Ala Gly Asp Thr Asn Asn Glu Ile Thr Pro Asp His Asn Asn
   515 520 525
<210> 21
   <211> 2148
   <212> DNA
   <213> Arabidopsis thaliana
<400> 21
   gaataacgcc ttctttagtt tacagtctct cgttgcgggt cttatcttct tcttcttctt 60
   ccctctttcg tctgctctat attatgacca gattcaaaat tgcgtagcgt actaattcaa 120
   tatagagaga agttagatca aaagaagaac acgaaactct gactgtgtct ctctttctct 180
   tcgtgtgttt ctatatttat atataaaaag acaagatctc tggtctggaa ttagaagaat 240
   cttatttggg gttttttctt aggattaagc tctaatggca gatcaagtaa aagagcggac 300
   tttagaggag acctctacgt gggcagttgc tgttgtttgc tttgtcttac tctttatttc 360
   gattgtcctc gaacattcta ttcacaaaat tggaacctgg tttaaaaaga agcacaagca 420
   ggctcttttt gaagctcttg aaaaggtcaa agcagagctt atgctgttgg gattcatatc 480
   actactactc acaattggac aaacaccaat ctcaaatatc tgcatctccc agaaagttgc    540
   gtcaacaatg cacccttgca gtgctgctga agaagctaaa aaatacggca agaaagacgc 600
   cggaaagaaa gatgatggag atggagataa acccggtcga agacttcttc ttgagttagc 660
   tgaatcttat atccatagaa gaagtttagc caccaaaggc tatgacaaat gtgcagagaa 720
   ggggaaagtg gcttttgtat ctgcttatgg aatccaccag ctgcatatat tcatcttcgt 780
   gctcgcggtt gttcatgttg tttactgcat tgttacttat gctttcggaa agatcaagat 840
   gaggacgtgg aagtcgtggg aggaagagac aaagacaata gagtatcagt attccaacga 900
   tcctgagagg ttcaggtttg cgagggacac atcttttggg agaagacatc tcaatttctg 960
   gagcaagacg agagtcacac tatggattgt ttgttttttt agacagttct ttggatctgt 1020
   caccaaagtt gattacttag cactaagaca tggtttcatc atggcgcatt ttgctcccgg 1080
   taacgaatca agattcgatt tccgcaagta tattcagaga tcattagaga aagacttcaa 1140
   aaccgttgtt gaaatcagtc cggttatctg gtttgtcgct gtgctattcc tcttgaccaa 1200
   ttcatatgga ttacgttctt acctctggtt accattcatt ccactagtcg taattctaat 1260
   agttggaaca aagcttgaag tcataataac aaaattgggt ctaagaatcc aagagaaagg 1320
   tgatgtggtg agaggcgccc cagtggttca gcctggtgat gacctcttct ggtttggcaa 1380
   gccacgcttc attcttttcc ttattcactt ggtccttttt acgaatgcat ttcaacttgc 1440
   attctttgcc tggagtacgt atgaattcaa tctcaataat tgtttccatg aaagcactgc 1500
   agatgtggtc attagacttg tagttggagc tgttgtgcag atactttgca gctatgtgac 1560
   tcttccactc tatgcacttg ttactcagat gggtagtaaa atgaagccaa cagtattcaa 1620
   cgatagagta gccacggcat taaagaagtg gcatcacact gcaaagaacg agacgaaaca 1680
   cggaagacac tcgggatcca acacaccttt ctctagccgt ccaaccacac caacacatgg 1740
   ctcatctcca atccatctcc ttcacaattt caataaccgg agcgttgaaa attacccaag 1800
   ttctccttct cctagatact ctggtcatgg tcatcatgaa caccaatttt gggatcctga 1860
   gtctcaacac caagaagctg aaacttccac acatcattct cttgcgcatg aaagctcaga 1920
   acctgttctt gcatctgtgg aacttcctcc tataaggact agcaaaagct taagagattt 1980
   ttcttttaag aaatgatgat tcttgtttgc tatatttgat ttcgtacagt gggaaatttg 2040
   tcatatgaaa ataatttctt gtacattact agttggataa gaaataacca tatctatatg 2100
   gatacatgat atgttattct ctggcaatgc ttaagagttt cacgaatt 2148
<210> 22
   <211> 573
   <212> PRT
   <213> Arabidopsis thaliana
<400> 22
   Met Ala Asp Gln Val Lys Glu Arg Thr Leu Glu Glu Thr Ser Thr Trp
   1 5 10 15
   Ala Val Ala Val Val Cys Phe Val Leu Leu Phe Ile Ser Ile Val Leu
   20 25 30
   Glu His Ser Ile His Lys Ile Gly Thr Trp Phe Lys Lys Lys His Lys
           35                  40                  45
   Gln Ala Leu Phe Glu Ala Leu Glu Lys Val Lys Ala Glu Leu Met Leu
       50                  55                  60
   Leu Gly Phe Ile Ser Leu Leu Leu Thr Ile Gly Gln Thr Pro Ile Ser
   65                  70                  75                  80
   Asn Ile Cys Ile Ser Gln Lys Val Ala Ser Thr Met His Pro Cys Ser
                   85                  90                  95
   Ala Ala Glu Glu Ala Lys Lys Tyr Gly Lys Lys Asp Ala Gly Lys Lys
               100                 105                 110
   Asp Asp Gly Asp Gly Asp Lys Pro Gly Arg Arg Leu Leu Leu Glu Leu
           115                 120                 125
   Ala Glu Ser Tyr Ile His Arg Arg Ser Leu Ala Thr Lys Gly Tyr Asp
       130                 135                 140
   Lys Cys Ala Glu Lys Gly Lys Val Ala Phe Val Ser Ala Tyr Gly Ile
   145                 150                 155                 160
   His Gln Leu His Ile Phe Ile Phe Val Leu Ala Val Val His Val Val
                   165                 170                 175
   Tyr Cys Ile Val Thr Tyr Ala Phe Gly Lys Ile Lys Met Arg Thr Trp
               180                 185                 190
   Lys Ser Trp Glu Glu Glu Thr Lys Thr Ile Glu Tyr Gln Tyr Ser Asn
           195                 200                 205
   Asp Pro Glu Arg Phe Arg Phe Ala Arg Asp Thr Ser Phe Gly Arg Arg
       210                 215                 220
   His Leu Asn Phe Trp Ser Lys Thr Arg Val Thr Leu Trp Ile Val Cys
   225                 230                 235                 240
   Phe Phe Arg Gln Phe Phe Gly Ser Val Thr Lys Val Asp Tyr Leu Ala
                   245                 250                 255
   Leu Arg His Gly Phe Ile Met Ala His Phe Ala Pro Gly Asn Glu Ser
               260                 265                 270
   Arg Phe Asp Phe Arg Lys Tyr Ile Gln Arg Ser Leu Glu Lys Asp Phe
           275                 280                 285
   Lys Thr Val Val Glu Ile Ser Pro Val Ile Trp Phe Val Ala Val Leu
       290                 295                 300
   Phe Leu Leu Thr Asn Ser Tyr Gly Leu Arg Ser Tyr Leu Trp Leu Pro
   305                 310                 315                 320
   Phe Ile Pro Leu Val Val Ile Leu Ile Val Gly Thr Lys Leu Glu Val
                   325                 330                 335
   Ile Ile Thr Lys Leu Gly Leu Arg Ile Gln Glu Lys Gly Asp Val Val
               340                 345                 350
   Arg Gly Ala Pro Val Val Gln Pro Gly Asp Asp Leu Phe Trp Phe Gly
           355                 360                 365
   Lys Pro Arg Phe Ile Leu Phe Leu Ile His Leu Val Leu Phe Thr Asn
       370                 375                 380
   Ala Phe Gln Leu Ala Phe Phe Ala Trp Ser Thr Tyr Glu Phe Asn Leu
   385                 390                 395                 400
   Asn Asn Cys Phe His Glu Ser Thr Ala Asp Val Val Ile Arg Leu Val
                   405                 410                 415
   Val Gly Ala Val Val Gln Ile Leu Cys Ser Tyr Val Thr Leu Pro Leu
               420                 425                 430
   Tyr Ala Leu Val Thr Gln Met Gly Ser Lys Met Lys Pro Thr Val Phe
           435                 440                 445
   Asn Asp Arg Val Ala Thr Ala Leu Lys Lys Trp His His Thr Ala Lys
       450                 455                 460
   Asn Glu Thr Lys His Gly Arg His Ser Gly Ser Asn Thr Pro Phe Ser
   465                 470                 475                 480
   Ser Arg Pro Thr Thr Pro Thr His Gly Ser Ser Pro Ile His Leu Leu
                   485                 490                 495
   His Asn Phe Asn Asn Arg Ser Val Glu Asn Tyr Pro Ser Ser Pro Ser
               500                 505                 510
   Pro Arg Tyr Ser Gly His Gly His His Glu His Gln Phe Trp Asp Pro
           515                 520                 525
   Glu Ser Gln His Gln Glu Ala Glu Thr Ser Thr His His Ser Leu Ala
       530                 535                 540
   His Glu Ser Ser Glu Pro Val Leu Ala Ser Val Glu Leu Pro Pro Ile
   545                 550                 555                 560
   Arg Thr Ser Lys Ser Leu Arg Asp Phe Ser Phe Lys Lys
                   565                 570
<210> 23
   <211> 1587
   <212> DNA
   <213> Arabidopsis thaliana
<400> 23
   ttctcaacta aacaaatcag tcaccgggaa aaatataaag agatgacgga taaagaagaa 60
   agcaaccact cctcggaggt tggcgccgtc cgatccctcc aagaaactcc cacttgggct 120
   ctggccaccg tttgtttctt cttcatcgct gtttccattt gcctcgagcg tctcatcaat 180
   cttctatcca ctagacttaa gaaaaataga aaaacgtcgc tacttgaagc tgtagagaag 240
   ctcaaatcag ttctaatggt gctaggattc atgtcactaa tgctaaatgt aactgaagga 300
   gaagtttcga agatatgcat tcctataaag tatgccaatc gaatgttgcc atgccgtaaa 360
   accataaaat cacataatga tgttagtgaa gatgatgatg atgatgatgg tgataatcat 420
   gacaacagct tctttcatca atgctcttcc aagggtaaga cttcactaat atcagaagaa 480
   gggttgactc agttgagtta cttcttcttt gtgttggcat gcatgcatat tctctgcaat 540
   cttgctattc ttctccttgg aatggccaag atgagaaaat ggaattcgtg ggagaaagag 600
   acccaaacag tggagtatct agcagcaaac gatccgaata ggtttagaat aacaagagat 660
   acaacatttg ctcgacgaca cctgagttca tggactgaaa catcatttca actttggatt 720
   aaatgtttct tcaggcaatt ttacaactca gtggcgaaag tagattacct cacacttcgg 780
   catgggttca tctttgctca tgtatcgtca aataatgctt tcaacttcca aaactatata 840
   cagagatctt tacatgaaga ttttaaaact gtggttggta taagtccttt aatgtggcta 900
   actgtggtca tctttatgct cctcgacgtt tctggttgga gagtatattt ttatatgtca 960
   tttgtgccac tcattatagt tttagtgatc gggacgaaac tagagatgat agtagcgaaa 1020
   atggcggtca caattaagga gaataacagt gtaattagag gaacccctct cgttgagtca 1080
   aacgacacgc atttctggtt ttccaatcct cgttttctct taagcattct acactacacg 1140
   ctgtttctga acaccttcga gatggcattt attgtgtgga tcacttggca atttgggatt 1200
   aactcttgct accatgataa ccaagggatc ataatcacac gacttgtatt agcggtgaca 1260
   gtccagttct tgagcagcta cattacattg cctctttatg ccattgtaac tcagatgggg 1320
   tcaagctaca agagagcaat cttagaagaa caactagcaa atgtgttaag gcactggcaa 1380
   gggatggtta gagacaagaa aaagactatc caaacgccag acaccgacaa caacagtaac 1440
   aataacaatg gtgatattga ttctggagaa agtccggttc agacagaagt tgcttctgag 1500
   tttagatttt cgggtagaca atcgccgatt ttacaagaga tacagataca agagaaaact 1560
   gaaaggtgat cagattgatg gaaaatg 1587
<210> 24
   <211> 508
   <212> PRT
   <213> Arabidopsis thaliana
<400> 24
   Met Thr Asp Lys Glu Glu Ser Asn His Ser Ser Glu Val Gly Ala Val
   1               5                   10                  15
   Arg Ser Leu Gln Glu Thr Pro Thr Trp Ala Leu Ala Thr Val Cys Phe
               20                  25                  30
   Phe Phe Ile Ala Val Ser Ile Cys Leu Glu Arg Leu Ile Asn Leu Leu
           35                  40                  45
   Ser Thr Arg Leu Lys Lys Asn Arg Lys Thr Ser Leu Leu Glu Ala Val
       50                  55                  60
   Glu Lys Leu Lys Ser Val Leu Met Val Leu Gly Phe Met Ser Leu Met
   65                  70                  75                  80
   Leu Asn Val Thr Glu Gly Glu Val Ser Lys Ile Cys Ile Pro Ile Lys
                   85                  90                  95
   Tyr Ala Asn Arg Met Leu Pro Cys Arg Lys Thr Ile Lys Ser His Asn
               100                 105                 110
   Asp Val Ser Glu Asp Asp Asp Asp Asp Asp Gly Asp Asn His Asp Asn
           115                 120                 125
   Ser Phe Phe His Gln Cys Ser Ser Lys Gly Lys Thr Ser Leu Ile Ser
       130                 135                 140
   Glu Glu Gly Leu Thr Gln Leu Ser Tyr Phe Phe Phe Val Leu Ala Cys
   145                 150                 155                 160
   Met His Ile Leu Cys Asn Leu Ala Ile Leu Leu Leu Gly Met Ala Lys
                   165                 170                 175
   Met Arg Lys Trp Asn Ser Trp Glu Lys Glu Thr Gln Thr Val Glu Tyr
               180                 185                 190
   Leu Ala Ala Asn Asp Pro Asn Arg Phe Arg Ile Thr Arg Asp Thr Thr
           195                 200                 205
   Phe Ala Arg Arg His Leu Ser Ser Trp Thr Glu Thr Ser Phe Gln Leu
       210                 215                 220
   Trp Ile Lys Cys Phe Phe Arg Gln Phe Tyr Asn Ser Val Ala Lys Val
   225                 230                 235                 240
   Asp Tyr Leu Thr Leu Arg His Gly Phe Ile Phe Ala His Val Ser Ser
                   245                 250                 255
   Asn Asn Ala Phe Asn Phe Gln Asn Tyr Ile Gln Arg Ser Leu His Glu
               260                 265                 270
   Asp Phe Lys Thr Val Val Gly Ile Ser Pro Leu Met Trp Leu Thr Val
           275                 280                 285
   Val Ile Phe Met Leu Leu Asp Val Ser Gly Trp Arg Val Tyr Phe Tyr
   290 295 300
   Met Ser Phe Val Pro Leu Ile Ile Val Leu Val Ile Gly Thr Lys Leu
   305 310 315 320
   Glu Met Ile Val Ala Lys Met Ala Val Thr Ile Lys Glu Asn Asn Ser
   325 330 335
   Val Ile Arg Gly Thr Pro Leu Val Glu Ser Asn Asp Thr His Phe Trp
   340 345 350
   Phe Ser Asn Pro Arg Phe Leu Leu Ser Ile Leu His Tyr Thr Leu Phe
   355 360 365
   Leu Asn Thr Phe Glu Met Ala Phe Ile Val Trp Ile Thr Trp Gln Phe
   370 375 380
   Gly Ile Asn Ser Cys Tyr His Asp Asn Gln Gly Ile Ile Ile Thr Arg
   385 390 395 400
   Leu Val Leu Ala Val Thr Val Gln Phe Leu Ser Ser Tyr Ile Thr Leu
   405 410 415
   Pro Leu Tyr Ala Ile Val Thr Gln Met Gly Ser Ser Tyr Lys Arg Ala
   420 425 430
   Ile Leu Glu Glu Gln Leu Ala Asn Val Leu Arg His Trp Gln Gly Met
   435 440 445
   Val Arg Asp Lys Lys Lys Thr Ile Gln Thr Pro Asp Thr Asp Asn Asn
   450 455 460
   Ser Asn Asn Asn Asn Gly Asp Ile Asp Ser Gly Glu Ser Pro Val Gln
   465 470 475 480
   Thr Glu Val Ala Ser Glu Phe Arg Phe Ser Gly Arg Gln Ser Pro Ile
   485 490 495
   Leu Gln Glu Ile Gln Ile Gln Glu Lys Thr Glu Arg
   500 505
<210> 25
   <211> 1971
   <212> DNA
   <213> Arabidopsis thaliana
<400> 25
   gctaaacttt cttaatcaca gatgcagatt gattctccat ttttctcggg agaaaagttc 60
   ctcgccggag atggagcata tgatgaaaga aggaaggtct cttgcagaga cgccgactta 120
   ctctgttgct tcggttgtta ctgttttggt ctttgtttgc tttctcgttg aacgcgccat 180
   ttacagattt ggaaagtggt taaagaagac tagaagaaag gcacttttta cttcacttga   240
   gaaaatgaaa gaggagttga tgttgctggg acttatatca cttctgttgt cacaaagcgc   300
   gagatggatt tcagaaatct gtgttaactc ttcccttttc aatagtaaat tctacatttg    360
   ctctgaagag gactatggaa tccataagaa agttcttctg gaacacacct cttctacaaa   420
   ccagagctcc ttacctcatc atggaataca tgaagcctct catcaatgtg gtcatggccg   480
   tgaaccattt gtgtcgtatg agggactcga gcaactccta agattcttat tcgtcctggg   540
   tatcactcat gttctataca gtggcattgc cattggttta gccatgagca agatttacag   600
   ttggagaaaa tgggaagccc aagcgatcat aatggctgaa tcagatatcc acgcaaagaa   660
   gaccaaggtg atgaagcgtc agtctacatt tgttttccat catgcctctc atccttggag   720
   taacaatcgt tttctcattt ggatgctttg tttcctgcgg caatttagag gctccatacg   780
   aaagtctgac tacttcgcac ttcggttagg tttcctcact aaacataatt tgccatttac   840
   atacaacttc catatgtata tggtacggac gatggaagat gagtttcatg gcattgttgg   900
   aattagctgg ccactttggg tttacgctat agtatgcatc tgcataaatg ttcatggcct   960
   gaatatgtac ttttggatat cattcgttcc tgccattctt gtcatgttgg ttggaaccaa  1020
   acttgagcat gttgtctcca agcttgctct cgaggttaag gagcagcaga caggcacatc  1080
   taatggggct caagtcaaac cacgtgatgg gctcttctgg tttgggaaac cagaaattct  1140
   gctacggttg atacaattta tcatttttca gaatgcattt gaaatggcaa cattcatctg  1200
   gttcttgtgg ggaatcaagg aaagatcttg cttcatgaag aaccatgtga tgatatcaag  1260
   ccggctaatt tctggggttc tcgttcagtt ctggtgtagt tatggcactg tgcctctcaa  1320
   tgtaatcgtt actcagatgg gatctcggca taagaaagct gtgatagcag agagcgtaag  1380
   agactcactt cacagttggt gcaagagagt gaaagagagg tctaagcaca cgagatcagt  1440
   gtgttccctt gacacagcaa caatagacga gagagacgag atgacagtgg ggacattgtc  1500
   taggagctca tcgatgactt cactgaatca gattaccata aactccatag accaagcaga  1560
   gtctatattc ggagcagcag cttcatccag cagtcctcaa gatggataca cgtcgagggt  1620
   ggaagaatat ctgtctgaaa catacaataa catcggttcg ataccgcctt taaacgatga  1680
   gattgagatt gagattgaag gtgaagaaga taatggaggg agaggaagtg ggagtgatga  1740
   gaataacggt gatgctggag aaacacttct tgagttgttt aggaggactt gatcttgttt  1800
   ctgcttttag aagcttggtt cagttgtaat tacttgacat ccatacaaga ataacaatta  1860
   acaaagtaaa tcttttgttg gtccagtaaa aaaaagtatt caatgatcaa agtacatctt  1920
   ttgttggttc agaggtaaat ttaaaatata atagcatgga ttggaacgag a            1971
<210> 26
   <211> 573
   <212> PRT
   <213> Arabidopsis thaliana
<400> 26
   Met Glu His Met Met Lys Glu Gly Arg Ser Leu Ala Glu Thr Pro Thr
   1 5 10 15
   Tyr Ser Val Ala Ser Val Val Thr Val Leu Val Phe Val Cys Phe Leu
   20 25 30
   Val Glu Arg Ala Ile Tyr Arg Phe Gly Lys Trp Leu Lys Lys Thr Arg
   35 40 45
   Arg Lys Ala Leu Phe Thr Ser Leu Glu Lys Met Lys Glu Glu Leu Met
   50 55 60
   Leu Leu Gly Leu Ile Ser Leu Leu Leu Ser Gln Ser Ala Arg Trp Ile
   65 70 75 80
   Ser Glu Ile Cys Val Asn Ser Ser Leu Phe Asn Ser Lys Phe Tyr Ile
   85 90 95
   Cys Ser Glu Glu Asp Tyr Gly Ile His Lys Lys Val Leu Leu Glu His
   100 105 110
   Thr Ser Ser Thr Asn Gln Ser Ser Leu Pro His His Gly Ile His Glu
   115 120 125
   Ala Ser His Gln Cys Gly His Gly Arg Glu Pro Phe Val Ser Tyr Glu
   130 135 140
   Gly Leu Glu Gln Leu Leu Arg Phe Leu Phe Val Leu Gly Ile Thr His
   145 150 155 160
   Val Leu Tyr Ser Gly Ile Ala Ile Gly Leu Ala Met Ser Lys Ile Tyr
   165 170 175
   Ser Trp Arg Lys Trp Glu Ala Gln Ala Ile Ile Met Ala Glu Ser Asp
   180 185 190
   Ile His Ala Lys Lys Thr Lys Val Met Lys Arg Gln Ser Thr Phe Val
   195 200 205
   Phe His His Ala Ser His Pro Trp Ser Asn Asn Arg Phe Leu Ile Trp
   210 215 220
   Met Leu Cys Phe Leu Arg Gln Phe Arg Gly Ser Ile Arg Lys Ser Asp
   225 230 235 240
   Tyr Phe Ala Leu Arg Leu Gly Phe Leu Thr Lys His Asn Leu Pro Phe
   245 250 255
   Thr Tyr Asn Phe His Met Tyr Met Val Arg Thr Met Glu Asp Glu Phe
   260 265 270
   His Gly Ile Val Gly Ile Ser Trp Pro Leu Trp Val Tyr Ala Ile Val
   275 280 285
   Cys Ile Cys Ile Asn Val His Gly Leu Asn Met Tyr Phe Trp Ile Ser
   290 295 300
   Phe Val Pro Ala Ile Leu Val Met Leu Val Gly Thr Lys Leu Glu His
   305 310 315 320
   Val Val Ser Lys Leu Ala Leu Glu Val Lys Glu Gln Gln Thr Gly Thr
   325 330 335
   Ser Asn Gly Ala Gln Val Lys Pro Arg Asp Gly Leu Phe Trp Phe Gly
   340 345 350
   Lys Pro Glu Ile Leu Leu Arg Leu Ile Gln Phe Ile Ile Phe Gln Asn
   355 360 365
   Ala Phe Glu Met Ala Thr Phe Ile Trp Phe Leu Trp Gly Ile Lys Glu
   370 375 380
   Arg Ser Cys Phe Met Lys Asn His Val Met Ile Ser Ser Arg Leu Ile
   385 390 395 400
   Ser Gly Val Leu Val Gln Phe Trp Cys Ser Tyr Gly Thr Val Pro Leu
   405 410 415
   Asn Val Ile Val Thr Gln Met Gly Ser Arg His Lys Lys Ala Val Ile
   420 425 430
   Ala Glu Ser Val Arg Asp Ser Leu His Ser Trp Cys Lys Arg Val Lys
   435 440 445
   Glu Arg Ser Lys His Thr Arg Ser Val Cys Ser Leu Asp Thr Ala Thr
   450 455 460
   Ile Asp Glu Arg Asp Glu Met Thr Val Gly Thr Leu Ser Arg Ser Ser
   465 470 475 480
   Ser Met Thr Ser Leu Asn Gln Ile Thr Ile Asn Ser Ile Asp Gln Ala
   485 490 495
   Glu Ser Ile Phe Gly Ala Ala Ala Ser Ser Ser Ser Pro Gln Asp Gly
   500 505 510
   Tyr Thr Ser Arg Val Glu Glu Tyr Leu Ser Glu Thr Tyr Asn Asn Ile
   515 520 525
   Gly Ser Ile Pro Pro Leu Asn Asp Glu Ile Glu Ile Glu Ile Glu Gly
   530 535 540
   Glu Glu Asp Asn Gly Gly Arg Gly Ser Gly Ser Asp Glu Asn Asn Gly
   545 550 555 560
   Asp Ala Gly Glu Thr Leu Leu Glu Leu Phe Arg Arg Thr
   565 570
<210> 27
   <211> 1666
   <212> DNA
   <213> Arabidopsis thaliana
<400> 27
   ttaccgccgt ttcttggccg tagaagatat aaatattgat agtcgtatat aaaaaaaaaa 60
   catataagac aaggtacata agaaaaaaac aatttcgtta aagaaaaaat ggctggagga 120
   ggaggtggta gcacttctgg agaaggtcct agagagctcg atcagacacc gacatgggcc 180
   gtctccactg tttgtggcgt tatcatcttg atctctatcg ttctagagct catgattcac 240
   aaaatcggag aggttttcac tgaaagaagg aagaaagctt tgtacgaagc gcttcaaaag 300
   atcaagaacg agcttatggt tttgggattc atttctttgt tattaacatt tggacaaaac 360
   tacatagcaa gtttgtgtgt ggcgtcaaga tacggccatg cgatgtcctt ttgtggtcca 420
   tacgatggtc caagtggtga gtctaagaag ccaaagacta ccgaacactt agaacgtcgt 480
   gttttggctg atgctgctcc agctcagtgc aagaagggct atgtaccgct tatatcactc 540
   aacgcgttgc atcaagtgca tatcttcatc tttttcttgg ctgtgtttca tgtcatttat 600
   agtgctatta ccatgatgct tggacgagca aagattcgtg gatggaaagt ttgggaggaa 660
   gaagtcataa atgatcatga aatgatgaat gacccttcaa ggtttaggct cacacatgag 720
   acatcatttg ttagagagca tgtcaatcct tgggccaaaa atagattctc attctacgtt 780
   atgtgtttct ttcgtcaaat gctgagatct gtcagaaaat ctgattattt gacaatgcgt 840
   catggtttca taagtgtcca tttggcaccg ggtatgaagt ttaatttcca aaaatacatc 900
   aaaagatcat tggaagacga cttcaaggta gtcgtgggaa taagtcccga gctatgggcc 960
   tttgtaatgc tctttttgct ctttgatgtt cacgggtggt atgttactgc tgtgatcacc 1020
   atgattcctc cacttttgac attagcgata ggaaccaagc ttcaagccat catctcagac 1080
   atggcgttgg aaattcagga gagacacgcc gtgatacaag ggatgccact tgtcaatgtc 1140
   tctgatcgac atttctggtt tagtcgtccc gccttagtcc tccatatcat ccacttcatt 1200
   ctcttccaga atgcttttga gatcacctac ttcttctgga tatggtatga gttcgggtta 1260
   cggtcctgtt ttcatcacca tttcgcgcta ataatcataa gggttgctct tggggtggga 1320
   gtacaatttc tctgcagtta catcacactt cctctttacg ctctcgtgac tcagatggga 1380
   tcaacgatga agcgatcggt gtttgatgat caaacgtcaa aggcattaaa gaattggcat 1440
   aaaaatgcaa agaaaaagag cgaaactcct ggtcaaacgc agcctccatt gcctaatctc 1500
   cgacctaaaa ccggcggcga tattgagtct gcttctccgg ccaatatcac ggctagtgtt 1560
   gatgttaaag aaagtgatca atctcaatct agagacctct taagcggtcc ctaaaaaacc 1620
   aaacgggtca agacattact ttcacttaac cggcttactc taagtc 1666
<210> 28
   <211> 501
   <212> PRT
   <213> Arabidopsis thaliana
<400> 28
   Met Ala Gly Gly Gly Gly Gly Ser Thr Ser Gly Glu Gly Pro Arg Glu
   1               5                   10                  15
   Leu Asp Gln Thr Pro Thr Trp Ala Val Ser Thr Val Cys Gly Val Ile
               20                  25                  30
   Ile Leu Ile Ser Ile Val Leu Glu Leu Met Ile His Lys Ile Gly Glu
           35                  40                  45
   Val Phe Thr Glu Arg Arg Lys Lys Ala Leu Tyr Glu Ala Leu Gln Lys
       50                  55                  60
   Ile Lys Asn Glu Leu Met Val Leu Gly Phe Ile Ser Leu Leu Leu Thr
   65                  70                  75                  80
   Phe Gly Gln Asn Tyr Ile Ala Ser Leu Cys Val Ala Ser Arg Tyr Gly
                   85                  90                  95
   His Ala Met Ser Phe Cys Gly Pro Tyr Asp Gly Pro Ser Gly Glu Ser
               100                 105                 110
   Lys Lys Pro Lys Thr Thr Glu His Leu Glu Arg Arg Val Leu Ala Asp
           115                 120                 125
   Ala Ala Pro Ala Gln Cys Lys Lys Gly Tyr Val Pro Leu Ile Ser Leu
       130                 135                 140
   Asn Ala Leu His Gln Val His Ile Phe Ile Phe Phe Leu Ala Val Phe
   145                 150                 155                 160
   His Val Ile Tyr Ser Ala Ile Thr Met Met Leu Gly Arg Ala Lys Ile
                   165                 170                 175
   Arg Gly Trp Lys Val Trp Glu Glu Glu Val Ile Asn Asp His Glu Met
               180                 185                 190
   Met Asn Asp Pro Ser Arg Phe Arg Leu Thr His Glu Thr Ser Phe Val
           195                 200                 205
   Arg Glu His Val Asn Pro Trp Ala Lys Asn Arg Phe Ser Phe Tyr Val
       210                 215                 220
   Met Cys Phe Phe Arg Gln Met Leu Arg Ser Val Arg Lys Ser Asp Tyr
   225                 230                 235                 240
   Leu Thr Met Arg His Gly Phe Ile Ser Val His Leu Ala Pro Gly Met
                   245                 250                 255
   Lys Phe Asn Phe Gln Lys Tyr Ile Lys Arg Ser Leu Glu Asp Asp Phe
               260                 265                 270
   Lys Val Val Val Gly Ile Ser Pro Glu Leu Trp Ala Phe Val Met Leu
           275                 280                 285
   Phe Leu Leu Phe Asp Val His Gly Trp Tyr Val Thr Ala Val Ile Thr
       290                 295                 300
   Met Ile Pro Pro Leu Leu Thr Leu Ala Ile Gly Thr Lys Leu Gln Ala
   305                 310                 315                 320
   Ile Ile Ser Asp Met Ala Leu Glu Ile Gln Glu Arg His Ala Val Ile
                   325                 330                 335
   Gln Gly Met Pro Leu Val Asn Val Ser Asp Arg His Phe Trp Phe Ser
               340                 345                 350
   Arg Pro Ala Leu Val Leu His Ile Ile His Phe Ile Leu Phe Gln Asn
           355                 360                 365
   Ala Phe Glu Ile Thr Tyr Phe Phe Trp Ile Trp Tyr Glu Phe Gly Leu
       370                 375                 380
   Arg Ser Cys Phe His His His Phe Ala Leu Ile Ile Ile Arg Val Ala
   385                 390                 395                 400
   Leu Gly Val Gly Val Gln Phe Leu Cys Ser Tyr Ile Thr Leu Pro Leu
                   405                 410                 415
   Tyr Ala Leu Val Thr Gln Met Gly Ser Thr Met Lys Arg Ser Val Phe
               420                 425                 430
   Asp Asp Gln Thr Ser Lys Ala Leu Lys Asn Trp His Lys Asn Ala Lys
           435                 440                 445
   Lys Lys Ser Glu Thr Pro Gly Gln Thr Gln Pro Pro Leu Pro Asn Leu
       450                 455                 460
   Arg Pro Lys Thr Gly Gly Asp Ile Glu Ser Ala Ser Pro Ala Asn Ile
   465                 470                 475                 480
   Thr Ala Ser Val Asp Val Lys Glu Ser Asp Gln Ser Gln Ser Arg Asp
                   485                 490                 495
   Leu Leu Ser Gly Pro
               500
<210> 29
   <211> 1942
   <212> DNA
   <213> Arabidopsis thaliana
<400> 29
   ctgtgagttc taatggcgga tcaagttaaa gaaaagacat tggaggaaac ttctacatgg     60
   gccgtcgcgg tggtttgctt cgtcttgctt ctgatttcga tcgttattga aaaacttatt    120
   cataaaattg gatcctggtt taaaaagaag aacaaaaaag ctctatatga agcacttgaa    180
   aaagtgaaag cagagcttat gctgatggga ttcatatcac tacttctaac aattggacaa    240
   ggatatatct caaatatttg catccctaag aacatcgcag catcgatgca cccttgtagt    300
   gcatccgaag aagcaagaaa gtatggtaag aaagatgtcc caaaggaaga tgaagaagaa    360
   aacttgcgtc gaaagcttct acagttagtt gattctctta ttcctcgaag gagtttggct    420
   actaaaggtt atgataagtg tgcagagaag ggaaaagtcg cttttgtatc ggcttatggc    480
   atgcatcagc tgcatatatt catctttgtt cttgcggttt gtcatgtgat ctactgcatt    540
   gttacttatg ctttgggaaa gaccaaaatg agaaggtgga agaagtggga agaggagact    600
   aagactatcg aatatcagta ttcacacgat cccgagaggt ttaggtttgc gcgggataca    660
   tctttcgggc gtagacatct gagtttctgg agcaaatcaa ctattacgct gtggattgta    720
   tgtttcttca ggcagttctt tagatctgta accaaagttg attacttaac actgagacat    780
   ggtttcatca tggcccattt ggctcctggg agcgacgcaa ggttcgattt ccgaaagtat    840
   attcagagat cactagagga agacttcaaa accattgtcg agatcaaccc tgtgatctgg    900
   ttcatagctg tgctattcct cctgacaaac acaaacggat tgaattctta cctctggcta    960
   ccgttcattc ccttcatcgt gattcttatt gttggaacaa aacttcaagt gataataaca   1020
   aaactaggac ttagaatcca agagaaaggc gacgtagtga aaggcacacc gctagttcaa   1080
   cccggtgatc atttcttctg gttcggtcgt ccacgtttca ttctcttcct cattcactta   1140
   gtcctcttca cgaacgcgtt tcaactagct ttctttgtct ggagtacgta tgaattcggt   1200
   ctaaagaact gtttccatga aagcagagtt gacgtgatca tcagaatctc aatcggactt   1260
   ttagttcaga ttctttgcag ctacgttact cttcctctat atgctcttgt tactcaaatg   1320
   ggttcaaaga tgaaaccaac agtgttcaac gagagagtag caacagcgtt aaagagttgg   1380
   catcacacag ctaagaaaaa tatcaaacat ggaagaactt cagaatcaac aacacctttc   1440
   tctagtagac caacaacacc aactcatggt tcttctccga ttcatctcct tcgtaatgct   1500
   cctcacaaac gaagcagaag cgttgacgaa agctttgcga attcgttttc tccgagaaac   1560
   tctgatttcg attcgtggga tcctgagtct caacatgaaa ctgctgagac ttcgaattcg   1620
   aatcatcgtt ctaggtttgg agaagaagaa tcggagaaaa agtttgtttc ttcatcagtg   1680
   gaacttcctc ctggacctgg acaaatacga acacagcatg agattagtac tataagctta   1740
   agggattttt cgtttaagcg atgatttaat gttttcttaa ttcattattt agtgatttgt   1800
   taatatgcta aatgaaacca gttcttgtaa attttctata ctatgtttgt taaaaccagc   1860
   ccttgaagct ttaatttctc aatgtaaaag tctgatgatt ttggatttta agactatgga   1920
   gattcatggg tagttggtga ag                                              1942
<210> 30
   <211> 583
   <212> PRT
   <213> Arabidopsis thaliana
<400> 30
   Met Ala Asp Gln Val Lys Glu Lys Thr Leu Glu Glu Thr Ser Thr Trp
   1               5                   10                  15
   Ala Val Ala Val Val Cys Phe Val Leu Leu Leu Ile Ser Ile Val Ile
               20                  25                  30
   Glu Lys Leu Ile His Lys Ile Gly Ser Trp Phe Lys Lys Lys Asn Lys
           35                  40                  45
   Lys Ala Leu Tyr Glu Ala Leu Glu Lys Val Lys Ala Glu Leu Met Leu
       50                  55                  60
   Met Gly Phe Ile Ser Leu Leu Leu Thr Ile Gly Gln Gly Tyr Ile Ser
   65                  70                  75                  80
   Asn Ile Cys Ile Pro Lys Asn Ile Ala Ala Ser Met His Pro Cys Ser
                   85                  90                  95
   Ala Ser Glu Glu Ala Arg Lys Tyr Gly Lys Lys Asp Val Pro Lys Glu
               100                 105                 110
   Asp Glu Glu Glu Asn Leu Arg Arg Lys Leu Leu Gln Leu Val Asp Ser
           115                 120                 125
   Leu Ile Pro Arg Arg Ser Leu Ala Thr Lys Gly Tyr Asp Lys Cys Ala
       130                 135                 140
   Glu Lys Gly Lys Val Ala Phe Val Ser Ala Tyr Gly Met His Gln Leu
   145                 150                 155                 160
   His Ile Phe Ile Phe Val Leu Ala Val Cys His Val Ile Tyr Cys Ile
                   165                 170                 175
   Val Thr Tyr Ala Leu Gly Lys Thr Lys Met Arg Arg Trp Lys Lys Trp
               180                 185                 190
   Glu Glu Glu Thr Lys Thr Ile Glu Tyr Gln Tyr Ser His Asp Pro Glu
           195                 200                 205
   Arg Phe Arg Phe Ala Arg Asp Thr Ser Phe Gly Arg Arg His Leu Ser
       210                 215                 220
   Phe Trp Ser Lys Ser Thr Ile Thr Leu Trp Ile Val Cys Phe Phe Arg
   225                 230                 235                 240
   Gln Phe Phe Arg Ser Val Thr Lys Val Asp Tyr Leu Thr Leu Arg His
   245 250 255
   Gly Phe Ile Met Ala His Leu Ala Pro Gly Ser Asp Ala Arg Phe Asp
   260 265 270
   Phe Arg Lys Tyr Ile Gln Arg Ser Leu Glu Glu Asp Phe Lys Thr Ile
   275 280 285
   Val Glu Ile Asn Pro Val Ile Trp Phe Ile Ala Val Leu Phe Leu Leu
   290 295 300
   Thr Asn Thr Asn Gly Leu Asn Ser Tyr Leu Trp Leu Pro Phe Ile Pro
   305 310 315 320
   Phe Ile Val Ile Leu Ile Val Gly Thr Lys Leu Gln Val Ile Ile Thr
   325 330 335
   Lys Leu Gly Leu Arg Ile Gln Glu Lys Gly Asp Val Val Lys Gly Thr
   340 345 350
   Pro Leu Val Gln Pro Gly Asp His Phe Phe Trp Phe Gly Arg Pro Arg
   355 360 365
   Phe Ile Leu Phe Leu Ile His Leu Val Leu Phe Thr Asn Ala Phe Gln
   370 375 380
   Leu Ala Phe Phe Val Trp Ser Thr Tyr Glu Phe Gly Leu Lys Asn Cys
   385 390 395 400
   Phe His Glu Ser Arg Val Asp Val Ile Ile Arg Ile Ser Ile Gly Leu
   405 410 415
   Leu Val Gln Ile Leu Cys Ser Tyr Val Thr Leu Pro Leu Tyr Ala Leu
   420 425 430
   Val Thr Gln Met Gly Ser Lys Met Lys Pro Thr Val Phe Asn Glu Arg
   435 440 445
   Val Ala Thr Ala Leu Lys Ser Trp His His Thr Ala Lys Lys Asn Ile
   450 455 460
   Lys His Gly Arg Thr Ser Glu Ser Thr Thr Pro Phe Ser Ser Arg Pro
   465 470 475 480
   Thr Thr Pro Thr His Gly Ser Ser Pro Ile His Leu Leu Arg Asn Ala
   485 490 495
   Pro His Lys Arg Ser Arg Ser Val Asp Glu Ser Phe Ala Asn Ser Phe
   500 505 510
   Ser Pro Arg Asn Ser Asp Phe Asp Ser Trp Asp Pro Glu Ser Gln His
   515 520 525
   Glu Thr Ala Glu Thr Ser Asn Ser Asn His Arg Ser Arg Phe Gly Glu
       530                 535                 540
   Glu Glu Ser Glu Lys Lys Phe Val Ser Ser Ser Val Glu Leu Pro Pro
   545                 550                 555                 560
   Gly Pro Gly Gln Ile Arg Thr Gln His Glu Ile Ser Thr Ile Ser Leu
                   565                 570                 575
   Arg Asp Phe Ser Phe Lys Arg
               580
<210> 31
   <211> 1695
   <212> DNA
   <213> Arabidopsis thaliana
<400> 31
   atgatcacaa gaagcaggtg tcgaagatct ttgttatggt ttctagtgtt ccatggcgga 60
   gctacagcca ccggagctcc ctctggtggg aaagagcttt ctcagacgcc tacttgggca 120
   gtcgccgtcg tctgcacctt tctcatcctc atttcccatc tccttgaaaa gggtcttcaa 180
   agactcgcca actggctatg gaagaagcat aaaaactctc tccttgaagc cttagagaaa 240
   atcaaagctg agctgatgat ccttggattc atttctttat tactcacttt tggagaacca 300
   tatattctca agatctgtgt tcctcgaaaa gctgctctct ctatgttacc ttgtttatct 360
   gaagacacag tgcttttcca gaaacttgct ccatcatctc ttagcaggca tcttttggct 420
   gctggtgata catctattaa ttgcaaacaa ggatctgagc cactcataac attgaaaggc 480
   ttgcaccaac ttcacatctt gttgttcttc ttggccatct ttcatatcgt atatagttta 540
   atcaccatga tgcttagcag gctcaagatt cgtggatgga aaaagtggga gcaagagaca 600
   ttatctaatg actatgagtt ttctattgat cattcaagac ttaggctcac tcatgagact 660
   tcttttgtga gagaacatac aagtttctgg acaacaactc ctttcttctt ttacgtcgga 720
   tgcttcttta ggcagttctt tgtatctgtt gaaagaaccg actacttgac tctgcgccat 780
   ggattcatct ctgcccattt agctccagga agaaagttca acttccagag atatatcaaa 840
   agatctctcg aggatgattt caagttggta gttggaataa gtccagttct ttgggcatca 900
   tttgtaatct tcttgctgtt caatgttaat ggctggagaa cattgttttg ggcatcgata 960
   cctcctctac tcataatcct agctgttgga acaaagcttc aagcaattat ggcaacaatg 1020
   gcgctagaaa tcgtagagac acatgcagta gttcagggga tgcctttagt gcaaggttca 1080
   gatcgatact tttggttcga ctgtcctcaa ctacttcttc atcttatcca ctttgccttg 1140
   tttcagaatg ctttccagat aacacacttc ttctggatat ggtattcttt tggattaaaa 1200
   tcatgcttcc ataaagattt caatcttgta gtcagcaaac tctttctatg cctaggagct 1260
   ttgatcttat gcagctacat cactctccca ttgtacgccc tcgttactca gatgggttca 1320
   cacatgaaga aagcagtgtt tgatgagcaa atggcaaagg cattgaagaa gtggcacaaa 1380
   gacatcaaat tgaagaaagg taaagcgagg aagctcccga gcaagacact tggtgtttca 1440
   gagagtttca gcctctcttc ctcttcctct gcaaccactc ttcaccgttc caagaccact 1500
   ggtcactctt ctaacatcat atactacaaa caagaagatg aagaagacga aatgtctgat 1560
   cttgaagctg gagcagagga tgctattgac aggattcaac aacaggagat gcaattccac 1620
   aactcttagc tgctttagtt tcttctgttg ttacttcata gttcaaatga aacgatgctg 1680
   tttactaact tagtc 1695
<210> 32
   <211> 542
   <212> PRT
   <213> Arabidopsis thaliana
<400> 32
   Met Ile Thr Arg Ser Arg Cys Arg Arg Ser Leu Leu Trp Phe Leu Val
   1               5                   10                  15
   Phe His Gly Gly Ala Thr Ala Thr Gly Ala Pro Ser Gly Gly Lys Glu
               20                  25                  30
   Leu Ser Gln Thr Pro Thr Trp Ala Val Ala Val Val Cys Thr Phe Leu
           35                  40                  45
   Ile Leu Ile Ser His Leu Leu Glu Lys Gly Leu Gln Arg Leu Ala Asn
       50                  55                  60
   Trp Leu Trp Lys Lys His Lys Asn Ser Leu Leu Glu Ala Leu Glu Lys
   65                  70                  75                  80
   Ile Lys Ala Glu Leu Met Ile Leu Gly Phe Ile Ser Leu Leu Leu Thr
                   85                  90                  95
   Phe Gly Glu Pro Tyr Ile Leu Lys Ile Cys Val Pro Arg Lys Ala Ala
               100                 105                 110
   Leu Ser Met Leu Pro Cys Leu Ser Glu Asp Thr Val Leu Phe Gln Lys
           115                 120                 125
   Leu Ala Pro Ser Ser Leu Ser Arg His Leu Leu Ala Ala Gly Asp Thr
       130                 135                 140
   Ser Ile Asn Cys Lys Gln Gly Ser Glu Pro Leu Ile Thr Leu Lys Gly
   145                 150                 155                 160
   Leu His Gln Leu His Ile Leu Leu Phe Phe Leu Ala Ile Phe His Ile
                   165                 170                 175
   Val Tyr Ser Leu Ile Thr Met Met Leu Ser Arg Leu Lys Ile Arg Gly
               180                 185                 190
   Trp Lys Lys Trp Glu Gln Glu Thr Leu Ser Asn Asp Tyr Glu Phe Ser
   195 200 205
   Ile Asp His Ser Arg Leu Arg Leu Thr His Glu Thr Ser Phe Val Arg
   210 215 220
   Glu His Thr Ser Phe Trp Thr Thr Thr Pro Phe Phe Phe Tyr Val Gly
   225 230 235 240
   Cys Phe Phe Arg Gln Phe Phe Val Ser Val Glu Arg Thr Asp Tyr Leu
   245 250 255
   Thr Leu Arg His Gly Phe Ile Ser Ala His Leu Ala Pro Gly Arg Lys
   260 265 270
   Phe Asn Phe Gln Arg Tyr Ile Lys Arg Ser Leu Glu Asp Asp Phe Lys
   275 280 285
   Leu Val Val Gly Ile Ser Pro Val Leu Trp Ala Ser Phe Val Ile Phe
   290 295 300
   Leu Leu Phe Asn Val Asn Gly Trp Arg Thr Leu Phe Trp Ala Ser Ile
   305 310 315 320
   Pro Pro Leu Leu Ile Ile Leu Ala Val Gly Thr Lys Leu Gln Ala Ile
   325 330 335
   Met Ala Thr Met Ala Leu Glu Ile Val Glu Thr His Ala Val Val Gln
   340 345 350
   Gly Met Pro Leu Val Gln Gly Ser Asp Arg Tyr Phe Trp Phe Asp Cys
   355 360 365
   Pro Gln Leu Leu Leu His Leu Ile His Phe Ala Leu Phe Gln Asn Ala
   370 375 380
   Phe Gln Ile Thr His Phe Phe Trp Ile Trp Tyr Ser Phe Gly Leu Lys
   385 390 395 400
   Ser Cys Phe His Lys Asp Phe Asn Leu Val Val Ser Lys Leu Phe Leu
   405 410 415
   Cys Leu Gly Ala Leu Ile Leu Cys Ser Tyr Ile Thr Leu Pro Leu Tyr
   420 425 430
   Ala Leu Val Thr Gln Met Gly Ser His Met Lys Lys Ala Val Phe Asp
   435 440 445
   Glu Gln Met Ala Lys Ala Leu Lys Lys Trp His Lys Asp Ile Lys Leu
   450 455 460
   Lys Lys Gly Lys Ala Arg Lys Leu Pro Ser Lys Thr Leu Gly Val Ser
   465 470 475 480
   Glu Ser Phe Ser Leu Ser Ser Ser Ser Ser Ala Thr Thr Leu His Arg
   485 490 495
   Ser Lys Thr Thr Gly His Ser Ser Asn Ile Ile Tyr Tyr Lys Gln Glu
   500 505 510
   Asp Glu Glu Asp Glu Met Ser Asp Leu Glu Ala Gly Ala Glu Asp Ala
   515 520 525
   Ile Asp Arg Ile Gln Gln Gln Glu Met Gln Phe His Asn Ser
   530 535 540
<210> 33
   <211> 2368
   <212> DNA
   <213> Arabidopsis thaliana
<400> 33
   aataaataaa gagtttagtc attttatcgt aaaaccaaaa gaaagagaaa aagattaatc 60
   atcatcaatc taaagattta ggaatctaca atttcccagt agacagattt cacttttgcg 120
   tcttcttctt attccatttg atctaagttt ctcgaaatag gtacaaagtt cgagtctttt 180
   tcttgtaacc gcgtgaaacc accaagaaac ctatagtaag agtttccgca aatgtgaatt 240
   aaagacagct catcactctg tcaaacttct acagtattaa gttaacgaag caggtacagc 300
   caaactaaaa taacgcggag ttatgggaat catcgacggt tctttgcttc ggcggttgat 360
   ttgtctctgt ctctggtgtc ttctcggtgg aggagtgacg gtggttacgg cggaggatga 420
   gaagaaagtg gtacataaac agcttaatca aactccgact tgggctgttg ctgctgtttg 480
   tactttcttc atcgttgttt ctgttcttct tgaaaaactt cttcacaaag ttggaaaggt 540
   tctatgggat cggcacaaga cagctcttct tgacgctttg gagaagatca aagcagagct 600
   gatggttctt ggattcatct ctttgcttct gacatttgga caaacctaca ttttggatat 660
   ttgtatccct tcacatgttg ctcgtacgat gctcccgtgt cctgctccta acttgaaaaa 720
   ggaggatgat gacaatggtg aaagtcacag gagactcttg tcgtttgagc acagattttt 780
   atctggaggt gaagcatctc ccactaaatg cacgaaggag ggttatgtag agcttatctc 840
   tgccgaggca ctccatcagt tgcacatcct tatattcttc ttagccattt tccacgttct 900
   ttacagcttc ttaactatga tgcttggaag gttgaagatt cgcggatgga agcattggga 960
   gaatgagaca tcatcccata attacgagtt ttcaacagac acttccagat tcaggctaac 1020
   tcatgaaaca tcttttgtga gagcgcacac cagtttctgg acccggattc cattcttttt 1080
   ctatgttgga tgctttttca gacagttttt cagatccgtt gggagaactg actatttgac 1140
   attgagaaat ggtttcatcg ctgttcattt agctccagga agtcaattta acttccaaaa 1200
   atacattaaa agatcgttgg aggatgattt caaggtagtc gttggagtca gccctgtctt 1260
   gtggggatct tttgtgctat tcctcctcct aaatattgac ggcttcaaga tgatgttcat 1320
   cggcactgca ataccggtta ttatcatttt agctgtaggg acaaagcttc aagcgattat 1380
   gacaaggatg gctcttggta tcacagatag acatgcggta gttcaaggaa tgccgcttgt 1440
   acaaggcaac gatgagtatt tctggttcgg tcgtccccat ttgattctcc atctcatgca 1500
   tttcgccttg tttcagaacg catttcagat cacttatttc ttctggatat ggtattcctt 1560
   tggatcagat tcttgctacc atcctaattt caagattgca cttgtaaaag tagcgattgc 1620
   tttaggagta ttgtgtcttt gcagctacat cacacttcct ctttacgcac tcgtaactca 1680
   gatgggttct cggatgaaaa aatcggtatt cgatgaacaa acgtcaaaag cactcaagaa 1740
   atggagaatg gcagtgaaga agaagaaagg tgtgaaagcc actactaaga gactaggtgg 1800
   agatggaagt gcgagcccta cggcatcgac agttaggtct acttcgtctg tacgttcatt 1860
   gcagcgttac aaaaccacac cacattcgat gagatacgaa ggacttgacc ctgaaacatc 1920
   ggatctcgac acagataatg aagctttgac tcctcccaaa tctcctccaa gcttcgagct 1980
   tgttgtgaaa gttgaaccaa ataagaccaa taccggtgag actagccgtg acactgaaac 2040
   tgattctaaa gagttctctt tcgtcaagcc tgctccgagt aatgaatcat ctcaagaccg 2100
   gtgagactag tcaggattgt gaaggtgtag cctgcgccaa gtaaagactt cttttcgagt 2160
   taagcctgcg ccaagtaaag aaccatcgta gtaaagatgt gtcactccag agtttttttt 2220
   tttttggttt catttatcta atgctattgt aaatcaatgc cgtggcgata aagtaatgta 2280
   ttgatgacca tattaattaa aataggctga agaaaatgag ggacaaatat taagaatcag 2340
   tttagtaaaa aggttaaaag actcgtgt 2368
<210> 34
   <211> 593
   <212> PRT
   <213> Arabidopsis thaliana
<400> 34
   Met Gly Ile Ile Asp Gly Ser Leu Leu Arg Arg Leu Ile Cys Leu Cys
   1 5 10 15
   Leu Trp Cys Leu Leu Gly Gly Gly Val Thr Val Val Thr Ala Glu Asp
   20 25 30
   Glu Lys Lys Val Val His Lys Gln Leu Asn Gln Thr Pro Thr Trp Ala
   35 40 45
   Val Ala Ala Val Cys Thr Phe Phe Ile Val Val Ser Val Leu Leu Glu
   50 55 60
   Lys Leu Leu His Lys Val Gly Lys Val Leu Trp Asp Arg His Lys Thr
   65 70 75 80
   Ala Leu Leu Asp Ala Leu Glu Lys Ile Lys Ala Glu Leu Met Val Leu
   85 90 95
   Gly Phe Ile Ser Leu Leu Leu Thr Phe Gly Gln Thr Tyr Ile Leu Asp
   100 105 110
   Ile Cys Ile Pro Ser His Val Ala Arg Thr Met Leu Pro Cys Pro Ala
   115 120 125
   Pro Asn Leu Lys Lys Glu Asp Asp Asp Asn Gly Glu Ser His Arg Arg
   130 135 140
   Leu Leu Ser Phe Glu His Arg Phe Leu Ser Gly Gly Glu Ala Ser Pro
   145 150 155 160
   Thr Lys Cys Thr Lys Glu Gly Tyr Val Glu Leu Ile Ser Ala Glu Ala
   165 170 175
   Leu His Gln Leu His Ile Leu Ile Phe Phe Leu Ala Ile Phe His Val
   180 185 190
   Leu Tyr Ser Phe Leu Thr Met Met Leu Gly Arg Leu Lys Ile Arg Gly
   195 200 205
   Trp Lys His Trp Glu Asn Glu Thr Ser Ser His Asn Tyr Glu Phe Ser
   210 215 220
   Thr Asp Thr Ser Arg Phe Arg Leu Thr His Glu Thr Ser Phe Val Arg
   225 230 235 240
   Ala His Thr Ser Phe Trp Thr Arg Ile Pro Phe Phe Phe Tyr Val Gly
   245 250 255
   Cys Phe Phe Arg Gln Phe Phe Arg Ser Val Gly Arg Thr Asp Tyr Leu
   260 265 270
   Thr Leu Arg Asn Gly Phe Ile Ala Val His Leu Ala Pro Gly Ser Gln
   275 280 285
   Phe Asn Phe Gln Lys Tyr Ile Lys Arg Ser Leu Glu Asp Asp Phe Lys
   290 295 300
   Val Val Val Gly Val Ser Pro Val Leu Trp Gly Ser Phe Val Leu Phe
   305 310 315 320
   Leu Leu Leu Asn Ile Asp Gly Phe Lys Met Met Phe Ile Gly Thr Ala
   325 330 335
   Ile Pro Val Ile Ile Ile Leu Ala Val Gly Thr Lys Leu Gln Ala Ile
   340 345 350
   Met Thr Arg Met Ala Leu Gly Ile Thr Asp Arg His Ala Val Val Gln
   355 360 365
   Gly Met Pro Leu Val Gln Gly Asn Asp Glu Tyr Phe Trp Phe Gly Arg
   370 375 380
   Pro His Leu Ile Leu His Leu Met His Phe Ala Leu Phe Gln Asn Ala
   385 390 395 400
   Phe Gln Ile Thr Tyr Phe Phe Trp Ile Trp Tyr Ser Phe Gly Ser Asp
   405 410 415
   Ser Cys Tyr His Pro Asn Phe Lys Ile Ala Leu Val Lys Val Ala Ile
   420 425 430
   Ala Leu Gly Val Leu Cys Leu Cys Ser Tyr Ile Thr Leu Pro Leu Tyr
   435 440 445
   Ala Leu Val Thr Gln Met Gly Ser Arg Met Lys Lys Ser Val Phe Asp
   450 455 460
   Glu Gln Thr Ser Lys Ala Leu Lys Lys Trp Arg Met Ala Val Lys Lys
   465 470 475 480
   Lys Lys Gly Val Lys Ala Thr Thr Lys Arg Leu Gly Gly Asp Gly Ser
   485 490 495
   Ala Ser Pro Thr Ala Ser Thr Val Arg Ser Thr Ser Ser Val Arg Ser
   500 505 510
   Leu Gln Arg Tyr Lys Thr Thr Pro His Ser Met Arg Tyr Glu Gly Leu
   515 520 525
   Asp Pro Glu Thr Ser Asp Leu Asp Thr Asp Asn Glu Ala Leu Thr Pro
   530 535 540
   Pro Lys Ser Pro Pro Ser Phe Glu Leu Val Val Lys Val Glu Pro Asn
   545 550 555 560
   Lys Thr Asn Thr Gly Glu Thr Ser Arg Asp Thr Glu Thr Asp Ser Lys
   565 570 575
   Glu Phe Ser Phe Val Lys Pro Ala Pro Ser Asn Glu Ser Ser Gln Asp
   580 585 590
   Arg
<210> 35
   <211> 1096
   <212> DNA
   <213> Arabidopsis thaliana
<400> 35
   caatgcgtat tctctactct gttcaacgtc gttctttggc cgacgctcct ccagtaaatt 60
   gcaagaaaga ttatgtcgca cttatatcat taaacgcatt acatcaagtg catatattca 120
   tattcttctt ggccgtgttc catgttatat atagtgctat aaccatgatg cttggaagag 180
   ccaagattcg tggctggaaa gtatgggagc aagaggtcat ccatgaacaa gaaatgatga 240
   atgatccatc aagatttaga ctcacacatg agacatcatt tgtccgagaa catgtcaatt 300
   cttgggctag caataaattc ttcttctacg ttatgtgctt cttccgtcaa atacttagat 360
   cagtgaggaa gtctgattac ttgacaatgc gacatggatt cataagtgtt catttggcac 420
   ctggtatgaa gtttgatttt caaaagtaca tcaaaagatc tttggaagac gacttcaagg 480
   tggttgtagg aataagaccc gagctttggg cctttgtaat gttgttttta ctcttcgatg 540
   ttcacggatg gtatgttact gccgtaatca ccatgattcc tcctctatta acactagcca 600
   taggaacaaa gctacaagcc attatatcgt acatggcatt ggagattcaa gagagacatg 660
   cagtaattca agggatgcca gttgtcaacg tctcagacca acatttttgg tttgaaaaac 720
   ccgatctagt acttcatatg atccacttcg ttctgtttca gaatgctttt gagataactt 780
   attttttttg gatatggtat gagtttgggc taaggtcctg ttttcatcac cattttggcc 840
   ttataatcat tcgtgtctgc ctaggggtgg gagtacaatt cctatgcagt tatatcacat 900
   tgccccttta cgctctcgtc actcagatgg gatccacaat gaagcgatca gtgtttgatg 960
   agcaaacttc aaaagcatta gaacaatggc ataagaaggc gaggaaaaag aatgaaaagt 1020
   gagaataatt ggcttctcct tttaatctat ggatagacca aatttaacag tctaatagtt 1080
   tggttactta atagtc 1096
<210> 36
   <211> 460
   <212> PRT
   <213> Arabidopsis thaliana
<400> 36
   Met Ala Gly Gly Gly Gly Gly Gly Gly Gly Glu Gly Pro Arg Gln Leu
   1 5 10 15
   Asp Gln Thr Pro Thr Trp Ala Val Ser Thr Val Cys Gly Val Ile Ile
   20 25 30
   Leu Ile Ser Ile Ile Leu Glu Leu Ile Ile His Lys Val Gly Glu Val
   35 40 45
   Phe Glu Arg Lys Lys Lys Lys Ala Leu Phe Glu Ala Leu Glu Lys Ile
   50 55 60
   Lys Asn Glu Leu Met Val Leu Gly Phe Ile Ser Leu Leu Leu Thr Phe
   65 70 75 80
   Gly Gln Asn Tyr Ile Ala Ser Ile Cys Val Pro Ser Arg Tyr Gly His
   85 90 95
   Ala Met Ser Phe Cys Gly Pro Tyr Asp Gly Pro Ser Glu Asp Asp Arg
   100 105 110
   Lys Lys Leu Lys Lys Thr Asp His Ala Met Arg Ile Leu Tyr Ser Val
           115                 120                 125
   Gln Arg Arg Ser Leu Ala Asp Ala Pro Pro Val Asn Cys Lys Lys Asp
       130                 135                 140
   Tyr Val Ala Leu Ile Ser Leu Asn Ala Leu His Gln Val His Ile Phe
   145                 150                 155                 160
   Ile Phe Phe Leu Ala Val Phe His Val Ile Tyr Ser Ala Ile Thr Met
                   165                 170                 175
   Met Leu Gly Arg Ala Lys Ile Arg Gly Trp Lys Val Trp Glu Gln Glu
               180                 185                 190
   Val Ile His Glu Gln Glu Met Met Asn Asp Pro Ser Arg Phe Arg Leu
           195                 200                 205
   Thr His Glu Thr Ser Phe Val Arg Glu His Val Asn Ser Trp Ala Ser
       210                 215                 220
   Asn Lys Phe Phe Phe Tyr Val Met Cys Phe Phe Arg Gln Ile Leu Arg
   225                 230                 235                 240
   Ser Val Arg Lys Ser Asp Tyr Leu Thr Met Arg His Gly Phe Ile Ser
                   245                 250                 255
   Val His Leu Ala Pro Gly Met Lys Phe Asp Phe Gln Lys Tyr Ile Lys
               260                 265                 270
   Arg Ser Leu Glu Asp Asp Phe Lys Val Val Val Gly Ile Arg Pro Glu
           275                 280                 285
   Leu Trp Ala Phe Val Met Leu Phe Leu Leu Phe Asp Val His Gly Trp
       290                 295                 300
   Tyr Val Thr Ala Val Ile Thr Met Ile Pro Pro Leu Leu Thr Leu Ala
   305                 310                 315                 320
   Ile Gly Thr Lys Leu Gln Ala Ile Ile Ser Tyr Met Ala Leu Glu Ile
                   325                 330                 335
   Gln Glu Arg His Ala Val Ile Gln Gly Met Pro Val Val Asn Val Ser
               340                 345                 350
   Asp Gln His Phe Trp Phe Glu Lys Pro Asp Leu Val Leu His Met Ile
           355                 360                 365
   His Phe Val Leu Phe Gln Asn Ala Phe Glu Ile Thr Tyr Phe Phe Trp
       370                 375                 380
   Ile Trp Tyr Glu Phe Gly Leu Arg Ser Cys Phe His His His Phe Gly
   385 390 395 400
   Leu Ile Ile Ile Arg Val Cys Leu Gly Val Gly Val Gln Phe Leu Cys
   405 410 415
   Ser Tyr Ile Thr Leu Pro Leu Tyr Ala Leu Val Thr Gln Met Gly Ser
   420 425 430
   Thr Met Lys Arg Ser Val Phe Asp Glu Gln Thr Ser Lys Ala Leu Glu
   435 440 445
   Gln Trp His Lys Lys Ala Arg Lys Lys Asn Glu Lys
   450 455 460
<210> 37
   <211> 1836
   <212> DNA
   <213> Arabidopsis thaliana
<400> 37
   cacgtacgta atcaaggacc aagggatttt cttcttttgg ctaccatggc cacaagatgc     60
   ttttggtgtt ggaccacttt gctcttctgc tctcagctgc ttaccggctt tgcccgagct    120
   tcctctgcag gcggcgccaa agagaaagga ctctcccaaa ctcccacctg ggccgttgcc    180
   ctcgtctgta ccttcttcat tcttgtctcc gtcctcctcg agaaggctct tcacagagtt    240
   gccacgtggt tgtgggagaa acataagaac tctctgcttg aagccttgga gaaaataaag    300
   gcggagctga tgattctagg attcatttcc ttgttgctca ccttcggaga gcagtacatt    360
   ctcaagattt gtattcctga aaaggctgca gcctctatgt taccttgtcc agctccttct    420
   actcatgacc aagacaagac ccaccgcaga cgtctagctg ctgctacgac ctcttcccgc    480
   tgcgatgagg gtcatgaacc actcatacct gccacgggtt tgcaccagct acacattcta    540
   ttgttcttca tggctgcctt tcatatcctc tacagtttca tcaccatgat gcttggcaga    600
   ctcaagatcc gtggctggaa aaagtgggag caggagacat gttctcatga ttacgagttt    660
   tcaatcgatc catcaagatt cagactcact catgagacgt cctttgttag acaacattcc    720
   agtttctgga caaaaatccc cttcttcttt tatgctgggt gcttcctaca gcagtttttc    780
   cgatctgtag ggaggactga ctacttaact ctgcgccatg gcttcatcgc tgcccattta    840
   gctccaggaa gaaagttcga cttccagaag tatatcaaaa gatcattgga agacgatttc    900
   aaggtggtag ttggaataag tcctcttttg tgggcatcat ttgtaatttt cctacttctg    960
   aatgttaatg gctgggaagc attgttttgg gcgtcaatcc tacctgtact tatcattcta   1020
   gctgtcagta cgaagcttca agcgatccta acaagaatgg ctctgggaat cacggagaga   1080
   cacgcagttg ttcaagggat acctctcgtg catggttcag ataagtactt ttggtttaat   1140
   cgccctcagt tgctacttca tcttcttcac ttcgccttat ttcagaatgc tttccagcta   1200
   acatacttct tctgggtctg gtattccttt gggctaaaat cttgctttca cacggatttc   1260
   aaactagtca tcgtaaaact ctctctaggc gttggagctt tgattttgtg cagctacatc   1320
   acacttcctt tgtatgcact agttactcag atgggttcaa acatgaagaa agctgtgttt   1380
   gatgagcaaa tggcaaaagc gttgaagaaa tggcacatga ctgtgaagaa gaagaaaggc   1440
   aaagcgagaa agccaccaac agagaccctt ggtgtttctg acactgtcag cacctctacc   1500
   tcatcctttc acgcctctgg agccactcta ctccgctcca agaccactgg tcactcgaca   1560
   gcctcttata tgagtaattt cgaggaccaa agcatgtctg atcttgaagc tgagccatta   1620
   tcccctgaac caatagaggg gcacactctc gtcagggttg gtgatcagaa cacagagata   1680
   gaatatactg gagatattag tcctggaaac caattctcct ttgtgaagaa cgttcctgct   1740
   aatgatattg actaatattc aaaatgaatg cagaacaaat ccatcatccg gtctttattt   1800
   tctattacat gtatgccaac aattgcttcg ccaagt                             1836
<210> 38
   <211> 569
   <212> PRT
   <213> Arabidopsis thaliana
<400> 38
   Met Ala Thr Arg Cys Phe Trp Cys Trp Thr Thr Leu Leu Phe Cys Ser
   1               5                   10                  15
   Gln Leu Leu Thr Gly Phe Ala Arg Ala Ser Ser Ala Gly Gly Ala Lys
               20                  25                  30
   Glu Lys Gly Leu Ser Gln Thr Pro Thr Trp Ala Val Ala Leu Val Cys
           35                  40                  45
   Thr Phe Phe Ile Leu Val Ser Val Leu Leu Glu Lys Ala Leu His Arg
       50                  55                  60
   Val Ala Thr Trp Leu Trp Glu Lys His Lys Asn Ser Leu Leu Glu Ala
   65                  70                  75                  80
   Leu Glu Lys Ile Lys Ala Glu Leu Met Ile Leu Gly Phe Ile Ser Leu
                   85                  90                  95
   Leu Leu Thr Phe Gly Glu Gln Tyr Ile Leu Lys Ile Cys Ile Pro Glu
               100                 105                 110
   Lys Ala Ala Ala Ser Met Leu Pro Cys Pro Ala Pro Ser Thr His Asp
           115                 120                 125
   Gln Asp Lys Thr His Arg Arg Arg Leu Ala Ala Ala Thr Thr Ser Ser
       130                 135                 140
   Arg Cys Asp Glu Gly His Glu Pro Leu Ile Pro Ala Thr Gly Leu His
   145                 150                 155                 160
   Gln Leu His Ile Leu Leu Phe Phe Met Ala Ala Phe His Ile Leu Tyr
                   165                 170                 175
   Ser Phe Ile Thr Met Met Leu Gly Arg Leu Lys Ile Arg Gly Trp Lys
   180 185 190
   Lys Trp Glu Gln Glu Thr Cys Ser His Asp Tyr Glu Phe Ser Ile Asp
   195 200 205
   Pro Ser Arg Phe Arg Leu Thr His Glu Thr Ser Phe Val Arg Gln His
   210 215 220
   Ser Ser Phe Trp Thr Lys Ile Pro Phe Phe Phe Tyr Ala Gly Cys Phe
   225 230 235 240
   Leu Gln Gln Phe Phe Arg Ser Val Gly Arg Thr Asp Tyr Leu Thr Leu
   245 250 255
   Arg His Gly Phe Ile Ala Ala His Leu Ala Pro Gly Arg Lys Phe Asp
   260 265 270
   Phe Gln Lys Tyr Ile Lys Arg Ser Leu Glu Asp Asp Phe Lys Val Val
   275 280 285
   Val Gly Ile Ser Pro Leu Leu Trp Ala Ser Phe Val Ile Phe Leu Leu
   290 295 300
   Leu Asn Val Asn Gly Trp Glu Ala Leu Phe Trp Ala Ser Ile Leu Pro
   305 310 315 320
   Val Leu Ile Ile Leu Ala Val Ser Thr Lys Leu Gln Ala Ile Leu Thr
   325 330 335
   Arg Met Ala Leu Gly Ile Thr Glu Arg His Ala Val Val Gln Gly Ile
   340 345 350
   Pro Leu Val His Gly Ser Asp Lys Tyr Phe Trp Phe Asn Arg Pro Gln
   355 360 365
   Leu Leu Leu His Leu Leu His Phe Ala Leu Phe Gln Asn Ala Phe Gln
   370 375 380
   Leu Thr Tyr Phe Phe Trp Val Trp Tyr Ser Phe Gly Leu Lys Ser Cys
   385 390 395 400
   Phe His Thr Asp Phe Lys Leu Val Ile Val Lys Leu Ser Leu Gly Val
   405 410 415
   Gly Ala Leu Ile Leu Cys Ser Tyr Ile Thr Leu Pro Leu Tyr Ala Leu
   420 425 430
   Val Thr Gln Met Gly Ser Asn Met Lys Lys Ala Val Phe Asp Glu Gln
   435 440 445
   Met Ala Lys Ala Leu Lys Lys Trp His Met Thr Val Lys Lys Lys Lys
   450 455 460
   Gly Lys Ala Arg Lys Pro Pro Thr Glu Thr Leu Gly Val Ser Asp Thr
   465 470 475 480
   Val Ser Thr Ser Thr Ser Ser Phe His Ala Ser Gly Ala Thr Leu Leu
   485 490 495
   Arg Ser Lys Thr Thr Gly His Ser Thr Ala Ser Tyr Met Ser Asn Phe
   500 505 510
   Glu Asp Gln Ser Met Ser Asp Leu Glu Ala Glu Pro Leu Ser Pro Glu
   515 520 525
   Pro Ile Glu Gly His Thr Leu Val Arg Val Gly Asp Gln Asn Thr Glu
   530 535 540
   Ile Glu Tyr Thr Gly Asp Ile Ser Pro Gly Asn Gln Phe Ser Phe Val
   545 550 555 560
   Lys Asn Val Pro Ala Asn Asp Ile Asp
   565
<210> 39
   <211> 2361
   <212> DNA
   <213> Arabidopsis thaliana
<400> 39
   aaaaataaaa atacgaaaaa acaagtattt ttcactttgc tcaagaccat ctctgcacat     60
   gaagttatga actaagtgag accagaagaa cttctttcga tcgatttcta cagtgattct    120
   gtttgctcaa gactacaact ttgtgtactt tctctttcaa cgagctgtgt atcgtgtact    180
   tcatggtgtg ttcctctgct tctgattgag attgaatctc ctgtggtttt tcagttgctc    240
   cggtgatcgg ctccggtgga ttgaaatttc aaagcttttc ctaatttctt ggagtctaca    300
   gatttcaaag tttctgggtt actaccaaag tagaatctca gtttttgtct aagctaatca    360
   gatttctgcg attctgattg ttaactaatc caagaacgat cgatgggggt ttaaagttga    420
   atttgggtct agaggaggaa cttgttttgt ttttgtgaag aatcagactt taaaagaaat    480
   gggagaagga gaagaaaatg gaaatgaagc agattcaaat gagagatctt tggcattgtc    540
   acctacttgg tctgttgcta ttgtcttgac tgttttcgtt gttgtttctt tgatcgttga    600
   gcgctccatt tatcgtctca gcacttggtt gagaaaaaca aagaggaaac ctatgtttgc    660
   tgcattagag aagatgaaag aagagttgat gctgcttggc ttcatttcac ttctattaac    720
   agctacctca agtacaatag ccaatatatg tgtcccttca agtttctaca atgacagatt    780
   tcttccgtgt acacgatctg agatccaaga ggagctcgaa agcggttcaa ctgtcaagcg    840
   gaatctttta acaaaatccc tctttttcaa catctttagg agaaggttgg atgtcataaa    900
   acgaaccacc tgcagcgagg ggcatgagcc gtttgtttca tatgaaggcc ttgaacagtt    960
   gcatcgcttc atctttataa tggcagttac tcatgtaact tatagctgct tgaccatgct    1020
   ccttgcaatc gtcaagattc atagttggag gatatgggaa gatgtagctc gtctagatag    1080
   acacgattgc ttaactgcgg tggcacgtga aaaaatattc cgaaggcaaa caacgtttgt    1140
   ccagtatcat acatcagcac ctctggccaa gaatagaatt cttatatggg tgacatgttt    1200
   cttccggcaa ttcggacgtt ctgttgatcg ttctgactat cttactctcc gcaaaggatt    1260
   cattgtgaat caccacctca cattaaagta cgattttcac agctacatga ttcgttctat    1320
   ggaagaagag ttccaaagga tcgttggtgt gagtgggccg ctttggggtt tcgtagttgc    1380
   tttcatgctc tttaacataa aaggatcaaa tctttacttc tggatagcga ttattcctgt    1440
   aacccttgtt cttctggttg gtgctaagtt gcaacacgta atagcaactt tggcattgga    1500
   gaatgccggg ttaaccgagt atccttccgg agtaaagctg agacctcgag atgaactttt    1560
   ctggttcaat aaaccagaac tactcttgtc ccttatccat ttcattctgt ttcagaattc    1620
   ttttgaactg gcttcgttct tctggttctg gtggcagttt gggtacagct cttgcttcct    1680
   taagaaccat taccttgttt acttcagact tcttttaggg tttgctggac agtttctatg    1740
   cagctacagt acactgccac tatatgcact agtcactcag atgggaacga actataaagc    1800
   ggctcttata cctcagagaa taagagagac gattcgcggt tggggtaaag caacgagaag    1860
   gaaaagaagg cacgggttat atggcgatga ttcaacagtt agaacagaaa caagcacaat    1920
   tgcatcactt gaagaatatg atcatcaagt gcttgacgtt actgaaactt ctttcgaaca    1980
   acaacggaaa caacaagaac aaggtactac tgagcttgag ttgcaaccaa tccaacctcg    2040
   taatgattgt gtacccaatg atacttcaag tagagtcgga acacctcttc ttcgaccttg    2100
   gctctccatt tcttcaccaa caacgaccat agagttgaga tcagaaccta tggaaacact    2160
   ctcgagatcg tcttccttgc caagtgagaa gagagtctga atataaataa gttcgtagga    2220
   gtaacagtag aaaacaagta tgtctcgatg aacatagatt gaaaacttac atcaatcaag    2280
   tgttagcaac actaatattt gtatgtgctc tacaaaaaaa ttatattgca atattgagaa    2340
   acatttaaat ttgttagaac c                                               2361
<210> 40
   <211> 573
   <212> PRT
   <213> Arabidopsis thaliana
<400> 40
   Met Gly Glu Gly Glu Glu Asn Gly Asn Glu Ala Asp Ser Asn Glu Arg
   1               5                   10                  15
   Ser Leu Ala Leu Ser Pro Thr Trp Ser Val Ala Ile Val Leu Thr Val
               20                  25                  30
   Phe Val Val Val Ser Leu Ile Val Glu Arg Ser Ile Tyr Arg Leu Ser
           35                  40                  45
   Thr Trp Leu Arg Lys Thr Lys Arg Lys Pro Met Phe Ala Ala Leu Glu
       50                  55                  60
   Lys Met Lys Glu Glu Leu Met Leu Leu Gly Phe Ile Ser Leu Leu Leu
   65                  70                  75                  80
   Thr Ala Thr Ser Ser Thr Ile Ala Asn Ile Cys Val Pro Ser Ser Phe
                   85                  90                  95
   Tyr Asn Asp Arg Phe Leu Pro Cys Thr Arg Ser Glu Ile Gln Glu Glu
               100                 105                 110
   Leu Glu Ser Gly Ser Thr Val Lys Arg Asn Leu Leu Thr Lys Ser Leu
           115                 120                 125
   Phe Phe Asn Ile Phe Arg Arg Arg Leu Asp Val Ile Lys Arg Thr Thr
       130                 135                 140
   Cys Ser Glu Gly His Glu Pro Phe Val Ser Tyr Glu Gly Leu Glu Gln
   145                 150                 155                 160
   Leu His Arg Phe Ile Phe Ile Met Ala Val Thr His Val Thr Tyr Ser
                   165                 170                 175
   Cys Leu Thr Met Leu Leu Ala Ile Val Lys Ile His Ser Trp Arg Ile
               180                 185                 190
   Trp Glu Asp Val Ala Arg Leu Asp Arg His Asp Cys Leu Thr Ala Val
           195                 200                 205
   Ala Arg Glu Lys Ile Phe Arg Arg Gln Thr Thr Phe Val Gln Tyr His
       210                 215                 220
   Thr Ser Ala Pro Leu Ala Lys Asn Arg Ile Leu Ile Trp Val Thr Cys
   225                 230                 235                 240
   Phe Phe Arg Gln Phe Gly Arg Ser Val Asp Arg Ser Asp Tyr Leu Thr
                   245                 250                 255
   Leu Arg Lys Gly Phe Ile Val Asn His His Leu Thr Leu Lys Tyr Asp
               260                 265                 270
   Phe His Ser Tyr Met Ile Arg Ser Met Glu Glu Glu Phe Gln Arg Ile
           275                 280                 285
   Val Gly Val Ser Gly Pro Leu Trp Gly Phe Val Val Ala Phe Met Leu
       290                 295                 300
   Phe Asn Ile Lys Gly Ser Asn Leu Tyr Phe Trp Ile Ala Ile Ile Pro
   305                 310                 315                 320
   Val Thr Leu Val Leu Leu Val Gly Ala Lys Leu Gln His Val Ile Ala
   325 330 335
   Thr Leu Ala Leu Glu Asn Ala Gly Leu Thr Glu Tyr Pro Ser Gly Val
   340 345 350
   Lys Leu Arg Pro Arg Asp Glu Leu Phe Trp Phe Asn Lys Pro Glu Leu
   355 360 365
   Leu Leu Ser Leu Ile His Phe Ile Leu Phe Gln Asn Ser Phe Glu Leu
   370 375 380
   Ala Ser Phe Phe Trp Phe Trp Trp Gln Phe Gly Tyr Ser Ser Cys Phe
   385 390 395 400
   Leu Lys Asn His Tyr Leu Val Tyr Phe Arg Leu Leu Leu Gly Phe Ala
   405 410 415
   Gly Gln Phe Leu Cys Ser Tyr Ser Thr Leu Pro Leu Tyr Ala Leu Val
   420 425 430
   Thr Gln Met Gly Thr Asn Tyr Lys Ala Ala Leu Ile Pro Gln Arg Ile
   435 440 445
   Arg Glu Thr Ile Arg Gly Trp Gly Lys Ala Thr Arg Arg Lys Arg Arg
   450 455 460
   His Gly Leu Tyr Gly Asp Asp Ser Thr Val Arg Thr Glu Thr Ser Thr
   465 470 475 480
   Ile Ala Ser Leu Glu Glu Tyr Asp His Gln Val Leu Asp Val Thr Glu
   485 490 495
   Thr Ser Phe Glu Gln Gln Arg Lys Gln Gln Glu Gln Gly Thr Thr Glu
   500 505 510
   Leu Glu Leu Gln Pro Ile Gln Pro Arg Asn Asp Cys Val Pro Asn Asp
   515 520 525
   Thr Ser Ser Arg Val Gly Thr Pro Leu Leu Arg Pro Trp Leu Ser Ile
   530 535 540
   Ser Ser Pro Thr Thr Thr Ile Glu Leu Arg Ser Glu Pro Met Glu Thr
   545 550 555 560
   Leu Ser Arg Ser Ser Ser Leu Pro Ser Glu Lys Arg Val
   565 570
<210> 41
   <211> 1843
   <212> DNA
   <213> Arabidopsis thaliana
<400> 41
   ttgagcagtg aaattaatgg caataaaaga gcgatcacta gaggaaacac caacatgggc      60
   tgttgctgta gtttgcttcg ttctcctttt catttccatc atgatcgaat acttcttgca     120
   ctttattggt cactggttta agaagaagca caagaaagct ttatctgaag ctcttgaaaa     180
   ggttaaagca gaactgatgc ttctgggatt catatcgctt ctattggttg tattgcaaac     240
   accagtctcc gagatttgca ttccaagaaa tattgctgcg acttggcatc cttgtagcaa     300
   ccatcaagaa atcgccaaat atggtaaaga ttatatcgac gatggccgca aaattcttga     360
   agattttgac tccaacgact tttacagtcc tcgccgaaat ttagccacca agggttatga     420
   caaatgcgca gaaaagggga aagtagcatt agtatctgcc tatggtatcc accagctgca     480
   tatattcatc tttgtgctcg ctgtttttca tgttctctac tgcattataa cctatgcttt     540
   aggaaagacc aagatgaaga aatggaagtc atgggagaga gagaccaaaa caattgagta     600
   ccaatatgcc aatgatccag agaggttcag atttgcaaga gatacatcgt tcggacgtag     660
   acatctgaat atatggagca agtctacctt taccctctgg attacatgtt tcttcagaca     720
   gttctttgga tcagtgacaa aagtagatta tcttacacta agacatggct ttattatggc     780
   acatttgcca gcaggaagtg ccgctcgttt cgatttccaa aaatacattg aaagatcttt     840
   ggaacaagat ttcacggtgg ttgtcggtat aagcccactg atatggtgca ttgctgtctt     900
   attcatattg accaatacac atggatggga ctcatatctt tggctgccct tccttccctt     960
   gattgtgata ttgatagtag gagcaaaact tcaaatgata atatcgaaat taggattaag    1020
   gattcaagaa aaaggagatg tggttaaagg agctcctgtg gttgaaccgg gcgatgatct    1080
   cttttggttt ggtcgtcctc gtttcattct cttcctcatt cacttggttc ttttcacgaa    1140
   tgcatttcaa ctggctttct tcgtttggag cacttacgaa ttcacactca aaaactgctt    1200
   ccaccacaaa acagaagaca ttgcaattag gatcaccatg ggggtattaa tacaagttct    1260
   atgcagctac atcactctac ctctctatgc tcttgttact cagatgggaa catcaatgag    1320
   gccgacaata ttcaacgaca gggtagccaa tgcattgaag aaatggcacc acacagccaa    1380
   gaaacagacg aaacatggac actcaggatc taacacacct cactcgagcc gtcctactac    1440
   gccaacacat ggcatgtcac cggtgcatct cctccacaac tacaataacc gcagcctcga    1500
   ccaacaaacc agcttcactg cttctccttc tcctcctaga ttctctgatt atagcggcca    1560
   aggccatggc catcagcatt tcttcgaccc tgaatctcag aatcactctt accaacgtga    1620
   gatcacagat tctgaattca gcaatagtca tcatccccaa gttgacatgg caagtcctgt    1680
   tagagaagag aaggagattg ttgagcatgt caaggttgat ttgtctgagt ttacgttcaa    1740
   gaagtgatag gactatactt tttacattct gtttgttcat tctttgtaaa tgatatgaac    1800
   ttaggaatga acacttttct tatttcttta tctgaatttt gct                      1843
<210> 42
   <211> 576
   <212> PRT
   <213> Arabidopsis thaliana
<400> 42
   Met Ala Ile Lys Glu Arg Ser Leu Glu Glu Thr Pro Thr Trp Ala Val
   1               5                   10                  15
   Ala Val Val Cys Phe Val Leu Leu Phe Ile Ser Ile Met Ile Glu Tyr
               20                  25                  30
   Phe Leu His Phe Ile Gly His Trp Phe Lys Lys Lys His Lys Lys Ala
           35                  40                  45
   Leu Ser Glu Ala Leu Glu Lys Val Lys Ala Glu Leu Met Leu Leu Gly
       50                  55                  60
   Phe Ile Ser Leu Leu Leu Val Val Leu Gln Thr Pro Val Ser Glu Ile
   65                  70                  75                  80
   Cys Ile Pro Arg Asn Ile Ala Ala Thr Trp His Pro Cys Ser Asn His
                   85                  90                  95
   Gln Glu Ile Ala Lys Tyr Gly Lys Asp Tyr Ile Asp Asp Gly Arg Lys
               100                 105                 110
   Ile Leu Glu Asp Phe Asp Ser Asn Asp Phe Tyr Ser Pro Arg Arg Asn
           115                 120                 125
   Leu Ala Thr Lys Gly Tyr Asp Lys Cys Ala Glu Lys Gly Lys Val Ala
       130                 135                 140
   Leu Val Ser Ala Tyr Gly Ile His Gln Leu His Ile Phe Ile Phe Val
   145                 150                 155                 160
   Leu Ala Val Phe His Val Leu Tyr Cys Ile Ile Thr Tyr Ala Leu Gly
                   165                 170                 175
   Lys Thr Lys Met Lys Lys Trp Lys Ser Trp Glu Arg Glu Thr Lys Thr
               180                 185                 190
   Ile Glu Tyr Gln Tyr Ala Asn Asp Pro Glu Arg Phe Arg Phe Ala Arg
           195                 200                 205
   Asp Thr Ser Phe Gly Arg Arg His Leu Asn Ile Trp Ser Lys Ser Thr
       210                 215                 220
   Phe Thr Leu Trp Ile Thr Cys Phe Phe Arg Gln Phe Phe Gly Ser Val
   225                 230                 235                 240
   Thr Lys Val Asp Tyr Leu Thr Leu Arg His Gly Phe Ile Met Ala His
                   245                 250                 255
   Leu Pro Ala Gly Ser Ala Ala Arg Phe Asp Phe Gln Lys Tyr Ile Glu
               260                 265                 270
   Arg Ser Leu Glu Gln Asp Phe Thr Val Val Val Gly Ile Ser Pro Leu
           275                 280                 285
   Ile Trp Cys Ile Ala Val Leu Phe Ile Leu Thr Asn Thr His Gly Trp
       290                 295                 300
   Asp Ser Tyr Leu Trp Leu Pro Phe Leu Pro Leu Ile Val Ile Leu Ile
   305                 310                 315                 320
   Val Gly Ala Lys Leu Gln Met Ile Ile Ser Lys Leu Gly Leu Arg Ile
                   325                 330                 335
   Gln Glu Lys Gly Asp Val Val Lys Gly Ala Pro Val Val Glu Pro Gly
               340                 345                 350
   Asp Asp Leu Phe Trp Phe Gly Arg Pro Arg Phe Ile Leu Phe Leu Ile
           355                 360                 365
   His Leu Val Leu Phe Thr Asn Ala Phe Gln Leu Ala Phe Phe Val Trp
       370                 375                 380
   Ser Thr Tyr Glu Phe Thr Leu Lys Asn Cys Phe His His Lys Thr Glu
   385                 390                 395                 400
   Asp Ile Ala Ile Arg Ile Thr Met Gly Val Leu Ile Gln Val Leu Cys
                   405                 410                 415
   Ser Tyr Ile Thr Leu Pro Leu Tyr Ala Leu Val Thr Gln Met Gly Thr
               420                 425                 430
   Ser Met Arg Pro Thr Ile Phe Asn Asp Arg Val Ala Asn Ala Leu Lys
           435                 440                 445
   Lys Trp His His Thr Ala Lys Lys Gln Thr Lys His Gly His Ser Gly
       450                 455                 460
   Ser Asn Thr Pro His Ser Ser Arg Pro Thr Thr Pro Thr His Gly Met
   465                 470                 475                 480
   Ser Pro Val His Leu Leu His Asn Tyr Asn Asn Arg Ser Leu Asp Gln
                   485                 490                 495
   Gln Thr Ser Phe Thr Ala Ser Pro Ser Pro Pro Arg Phe Ser Asp Tyr
               500                 505                 510
   Ser Gly Gln Gly His Gly His Gln His Phe Phe Asp Pro Glu Ser Gln
           515                 520                 525
   Asn His Ser Tyr Gln Arg Glu Ile Thr Asp Ser Glu Phe Ser Asn Ser
       530                 535                 540
   His His Pro Gln Val Asp Met Ala Ser Pro Val Arg Glu Glu Lys Glu
   545 550 555 560
   Ile Val Glu His Val Lys Val Asp Leu Ser Glu Phe Thr Phe Lys Lys
   565 570 575
<210> 43
   <211> 1699
   <212> DNA
   <213> Arabidopsis thaliana
<400> 43
   gtttattctc tgccttaaat aataataata ttaattcaga gttgtctcca tcttggaggt 60
   gcagaaatgg cagaagcaag gtctggttct cttgagtata cacccacatg ggtcgttgcg 120
   tttatctgtt tcatcattgt tctcttatct cttctcgctg aacgtggtct tcatcatctt 180
   ggaaagtgtc tgaagcgtag gcaacaagat gccttgttcg aagccttgca gaaactcaaa 240
   gaagaattga tgcttcttgg atttatctcc ctgatgttaa cggtatctca ggccgcaatt 300
   cggcatatct gtgtcccacc agctcttgta aacaacatgt ttccctgtaa gaagccattg 360
   gaggagcatc atgcgcctaa atcatctcat tcgattatca acaatgcacg acatctcctt 420
   tcaacaggag aaagtccaga ccattgtgct gccaaggggc aggttccatt agtatctgtg 480
   gaagcgttgc atcaactcca tatcttcatc tttgtgctag cggtttttca cgtcatcttc 540
   tgcgcctcaa ccatggttct tggaggagca agaatacaac aatggaaaca ttgggaggat 600
   tggttcaaga aacgtccttc tcaaaagggc actacaaggc gtggtcatca tgctcatgct 660
   cacgagttat tcagtgcaaa tcacgagttc ttcgagatgc atgctggagg attttggaga 720
   agatctgttg tcatcagctg ggtgagatca ttcttcaagc agttttatgg ttctgtcacc 780
   aaatcagaat acatagctct gcgacaagca ttcatcatga gtcattgccg cacaaaccca 840
   tcatttgatt ttcacaagta catgctaaga acactggaaa tagatttcaa gaaagttgtg 900
   agcataagtt ggtatctatg gctctttgtc gtcgtctttt tgctgctcaa tgttggagga 960
   tggaacactt acttctggtt atctttcttg cctttgattt tgttactaat ggtgggtgcc 1020
   aagttggaat atataataag tagcttagct ttggatgttt ccgagaagcg aagccgcgcg 1080
   gaagaagcag tgatcacacc ttctgatgaa ctcttttggt tccataggcc aggcattgtt 1140
   ctccaactca tccatttcat tctctttcag aattcatttg agattgcttt cttcttctgg 1200
   attttgttca catacggaat acattcatgt atcatggaga aactaggcta ccttatccca 1260
   agactcgtca tgggagtgtt agtccaagtg ctttgcagtt acagcacatt accactatat 1320
   gcccttgtta cacaaatggg tagcaaattc aagaaaggga tattcgacaa tgtagtacag 1380
   tccacactgg aaggatggtt agaagatacg aggaacagag gcgaatccac gagcgaggct 1440
   cataggatag agatgcaacc tacaactcct gaatcttata atgtccaaag tgaaaaccct 1500
   taattaagtt gcaatttgtg tcgtaatcca ataaggctat ccctttatta tttcacattt 1560
   agagctctca tcagaggatt gtgtgcatct atgacattga cgccaacgtt cctttctact 1620
   tttgaggcta ctagattaag ttgacttgtt actttttata agaaagcttc gatgatacat 1680
   ggattgatat ataattaaa 1699
<210> 44
   <211> 478
   <212> PRT
   <213> Arabidopsis thaliana
<400> 44
   Met Ala Glu Ala Arg Ser Gly Ser Leu Glu Tyr Thr Pro Thr Trp Val
   1               5                   10                  15
   Val Ala Phe Ile Cys Phe Ile Ile Val Leu Leu Ser Leu Leu Ala Glu
               20                  25                  30
   Arg Gly Leu His His Leu Gly Lys Cys Leu Lys Arg Arg Gln Gln Asp
           35                  40                  45
   Ala Leu Phe Glu Ala Leu Gln Lys Leu Lys Glu Glu Leu Met Leu Leu
       50                  55                  60
   Gly Phe Ile Ser Leu Met Leu Thr Val Ser Gln Ala Ala Ile Arg His
   65                  70                  75                  80
   Ile Cys Val Pro Pro Ala Leu Val Asn Asn Met Phe Pro Cys Lys Lys
                   85                  90                  95
   Pro Leu Glu Glu His His Ala Pro Lys Ser Ser His Ser Ile Ile Asn
               100                 105                 110
   Asn Ala Arg His Leu Leu Ser Thr Gly Glu Ser Pro Asp His Cys Ala
           115                 120                 125
   Ala Lys Gly Gln Val Pro Leu Val Ser Val Glu Ala Leu His Gln Leu
       130                 135                 140
   His Ile Phe Ile Phe Val Leu Ala Val Phe His Val Ile Phe Cys Ala
   145                 150                 155                 160
   Ser Thr Met Val Leu Gly Gly Ala Arg Ile Gln Gln Trp Lys His Trp
                   165                 170                 175
   Glu Asp Trp Phe Lys Lys Arg Pro Ser Gln Lys Gly Thr Thr Arg Arg
               180                 185                 190
   Gly His His Ala His Ala His Glu Leu Phe Ser Ala Asn His Glu Phe
           195                 200                 205
   Phe Glu Met His Ala Gly Gly Phe Trp Arg Arg Ser Val Val Ile Ser
       210                 215                 220
   Trp Val Arg Ser Phe Phe Lys Gln Phe Tyr Gly Ser Val Thr Lys Ser
   225                 230                 235                 240
   Glu Tyr Ile Ala Leu Arg Gln Ala Phe Ile Met Ser His Cys Arg Thr
                   245                 250                 255
   Asn Pro Ser Phe Asp Phe His Lys Tyr Met Leu Arg Thr Leu Glu Ile
               260                 265                 270
   Asp Phe Lys Lys Val Val Ser Ile Ser Trp Tyr Leu Trp Leu Phe Val
           275                 280                 285
   Val Val Phe Leu Leu Leu Asn Val Gly Gly Trp Asn Thr Tyr Phe Trp
       290                 295                 300
   Leu Ser Phe Leu Pro Leu Ile Leu Leu Leu Met Val Gly Ala Lys Leu
   305                 310                 315                 320
   Glu Tyr Ile Ile Ser Ser Leu Ala Leu Asp Val Ser Glu Lys Arg Ser
                   325                 330                 335
   Arg Ala Glu Glu Ala Val Ile Thr Pro Ser Asp Glu Leu Phe Trp Phe
               340                 345                 350
   His Arg Pro Gly Ile Val Leu Gln Leu Ile His Phe Ile Leu Phe Gln
           355                 360                 365
   Asn Ser Phe Glu Ile Ala Phe Phe Phe Trp Ile Leu Phe Thr Tyr Gly
       370                 375                 380
   Ile His Ser Cys Ile Met Glu Lys Leu Gly Tyr Leu Ile Pro Arg Leu
   385                 390                 395                 400
   Val Met Gly Val Leu Val Gln Val Leu Cys Ser Tyr Ser Thr Leu Pro
                   405                 410                 415
   Leu Tyr Ala Leu Val Thr Gln Met Gly Ser Lys Phe Lys Lys Gly Ile
               420                 425                 430
   Phe Asp Asn Val Val Gln Ser Thr Leu Glu Gly Trp Leu Glu Asp Thr
           435                 440                 445
   Arg Asn Arg Gly Glu Ser Thr Ser Glu Ala His Arg Ile Glu Met Gln
       450                 455                 460
   Pro Thr Thr Pro Glu Ser Tyr Asn Val Gln Ser Glu Asn Pro
   465                 470                 475
<210> 45
   <211> 1733
   <212> DNA
   <213> Arabidopsis thaliana
<400> 45
   ttggtttctt gcacgcgaag aaagaaacat ctgaaaattt gttcataaag aattcggttg      60
   aagaaatgag agaagaaaca gaaccaagcg agagaacgtt gggtttaaca ccaacttggt 120
   cagttgctac agtattgact atctttgtat ttgtttcttt gatcgttgaa cgttccattc 180
   atcggcttag taactggttg caaaagacta agagaaaacc tttgtttgct gcattggaga 240
   aaatgaaaga agagctgatg ctgctcggat tcatatcgct tcttcttaca gctacctcta 300
   gcacaatagc taacatatgc gtctcttcaa gtttccacaa cgatagattt gtcccatgta 360
   cgccttctga gattaatgag gaacttgaaa gtactatttc tactgtcaag cggactcagt 420
   taacgagatc tctcttcttg cacactctga ggagaagatt gagtggtata ggagaggata 480
   cgtgcagcga ggggcatgag ccatttcttt catatgaagg catggaacaa ttgcatcgct 540
   tcatatttat aatggcagtc actcatgtaa cttacagctg cttgacaatg cttcttgcaa 600
   tcgtgaagat tcatagatgg aggatatggg aagacgaggt tcatatggat cgaaatgatt 660
   gcttaactgt ggttgcacgc gaaaagattt tccgaaggca aacaacattt gtccagtatc 720
   atacatctgc tcctctggtc aagaatagat tacttatatg ggtgatatgt ttcttcagac 780
   agtttggaca ttctgttgtt cgttctgact atcttacact ccgaaaggga ttcatcatga 840
   accatcactt aacattgaca tacgattttc atagctatat gatccgctct atggaagaag 900
   agttccaaaa gattgttggc gtcagtgggc cactttgggg ttttgtagtt ggtttcatgc 960
   ttttcaacat aaaaggatca aatctttatt tctggctagc gatcattcca atcactcttg 1020
   ttcttttggt tggtgcaaag ttgcaacatg tcatcgcaac tttagcattg gagaacgcta 1080
   gtataacaga atatgcttct ggtataaagc tgagacctcg cgatgaactt ttttggttca 1140
   agaaaccgga attactctta tcccttatcc acttcattca gtttcagaat gcttttgaac 1200
   tggcttcttt cttctggttt tggtggcaat ttggatacaa ttcttgcttc ctaaggaacc 1260
   atttacttgt ctacttgcga cttattctag ggttttctgg acaattttta tgtagctaca 1320
   gcacactgcc actctatgca cttgtcactc agatgggaac aaactacaag gcagcattgt 1380
   tacctcaaag ggtaagagag acaattaatg gttggggaaa agcaacaaga cggaaaagaa 1440
   gacatggttt atatggagat gattcaacta ttcgaacaga aacaagcaca attgcatctg 1500
   ttgatgaata caatgaccag gtgcttgatg tttccgaaac ctctccagtt caagataacg 1560
   agcttgagct tcagcttatt cgcggagctt gtggaaattc tagtagtgtt gaaacaccga 1620
   tcttgcggcc ttgtgcttca atatcttcaa cgactttctc gaggctacag acagaaacaa 1680
   cagactcact ctcaaggtca tcctcattgc caatgagaag agaatgttaa gat 1733
<210> 46
   <211> 554
   <212> PRT
   <213> Arabidopsis thaliana
<400> 46
   Met Arg Glu Glu Thr Glu Pro Ser Glu Arg Thr Leu Gly Leu Thr Pro
   1               5                   10                  15
   Thr Trp Ser Val Ala Thr Val Leu Thr Ile Phe Val Phe Val Ser Leu
   20 25 30
   Ile Val Glu Arg Ser Ile His Arg Leu Ser Asn Trp Leu Gln Lys Thr
   35 40 45
   Lys Arg Lys Pro Leu Phe Ala Ala Leu Glu Lys Met Lys Glu Glu Leu
   50 55 60
   Met Leu Leu Gly Phe Ile Ser Leu Leu Leu Thr Ala Thr Ser Ser Thr
   65 70 75 80
   Ile Ala Asn Ile Cys Val Ser Ser Ser Phe His Asn Asp Arg Phe Val
   85 90 95
   Pro Cys Thr Pro Ser Glu Ile Asn Glu Glu Leu Glu Ser Thr Ile Ser
   100 105 110
   Thr Val Lys Arg Thr Gln Leu Thr Arg Ser Leu Phe Leu His Thr Leu
   115 120 125
   Arg Arg Arg Leu Ser Gly Ile Gly Glu Asp Thr Cys Ser Glu Gly His
   130 135 140
   Glu Pro Phe Leu Ser Tyr Glu Gly Met Glu Gln Leu His Arg Phe Ile
   145 150 155 160
   Phe Ile Met Ala Val Thr His Val Thr Tyr Ser Cys Leu Thr Met Leu
   165 170 175
   Leu Ala Ile Val Lys Ile His Arg Trp Arg Ile Trp Glu Asp Glu Val
   180 185 190
   His Met Asp Arg Asn Asp Cys Leu Thr Val Val Ala Arg Glu Lys Ile
   195 200 205
   Phe Arg Arg Gln Thr Thr Phe Val Gln Tyr His Thr Ser Ala Pro Leu
   210 215 220
   Val Lys Asn Arg Leu Leu Ile Trp Val Ile Cys Phe Phe Arg Gln Phe
   225 230 235 240
   Gly His Ser Val Val Arg Ser Asp Tyr Leu Thr Leu Arg Lys Gly Phe
   245 250 255
   Ile Met Asn His His Leu Thr Leu Thr Tyr Asp Phe His Ser Tyr Met
   260 265 270
   Ile Arg Ser Met Glu Glu Glu Phe Gln Lys Ile Val Gly Val Ser Gly
   275 280 285
   Pro Leu Trp Gly Phe Val Val Gly Phe Met Leu Phe Asn Ile Lys Gly
   290 295 300
   Ser Asn Leu Tyr Phe Trp Leu Ala Ile Ile Pro Ile Thr Leu Val Leu
   305                 310                 315                 320
   Leu Val Gly Ala Lys Leu Gln His Val Ile Ala Thr Leu Ala Leu Glu
                   325                 330                 335
   Asn Ala Ser Ile Thr Glu Tyr Ala Ser Gly Ile Lys Leu Arg Pro Arg
               340                 345                 350
   Asp Glu Leu Phe Trp Phe Lys Lys Pro Glu Leu Leu Leu Ser Leu Ile
           355                 360                 365
   His Phe Ile Gln Phe Gln Asn Ala Phe Glu Leu Ala Ser Phe Phe Trp
       370                 375                 380
   Phe Trp Trp Gln Phe Gly Tyr Asn Ser Cys Phe Leu Arg Asn His Leu
   385                 390                 395                 400
   Leu Val Tyr Leu Arg Leu Ile Leu Gly Phe Ser Gly Gln Phe Leu Cys
                   405                 410                 415
   Ser Tyr Ser Thr Leu Pro Leu Tyr Ala Leu Val Thr Gln Met Gly Thr
               420                 425                 430
   Asn Tyr Lys Ala Ala Leu Leu Pro Gln Arg Val Arg Glu Thr Ile Asn
           435                 440                 445
   Gly Trp Gly Lys Ala Thr Arg Arg Lys Arg Arg His Gly Leu Tyr Gly
       450                 455                 460
   Asp Asp Ser Thr Ile Arg Thr Glu Thr Ser Thr Ile Ala Ser Val Asp
   465                 470                 475                 480
   Glu Tyr Asn Asp Gln Val Leu Asp Val Ser Glu Thr Ser Pro Val Gln
                   485                 490                 495
   Asp Asn Glu Leu Glu Leu Gln Leu Ile Arg Gly Ala Cys Gly Asn Ser
               500                 505                 510
   Ser Ser Val Glu Thr Pro Ile Leu Arg Pro Cys Ala Ser Ile Ser Ser
           515                 520                 525
   Thr Thr Phe Ser Arg Leu Gln Thr Glu Thr Thr Asp Ser Leu Ser Arg
       530                 535                 540
   Ser Ser Ser Leu Pro Met Arg Arg Glu Cys
   545                 550
<210> 47
   <211> 1639
   <212> DNA
   <213> Arabidopsis thaliana
<400> 47
   ccggagcaaa aaaatggcgg gaggaggaac gaccttagag tacacaccaa cttgggtggt     60
   tgctctcgtc tgctccgtca ttgtctctat ctcttttgcc gtcgagcgtc tcattcaccg    120
   tgccggaaag cactttaaga acaacgatca gaagcagctt tttggggcat tacaaaagat    180
   caaagaagaa ctcatgctag tagggttcat atcgttgcta ttatcggtcg gccagtctaa    240
   aatcgcaaag atttgcatat caaaggagtt aagtgaaaag tttctccctt gtacgaaacc    300
   tgcaggggct gagaagtccc ttaaagactc ctcccatttc cagttcagct tcaccggccg    360
   tcatctcctc gccggagatg cccccgccgg tgactactgc tccctaaagg gaaaagtacc    420
   aataatgtca ttatcagctc tgcacgagct tcatatattc atcttcgtat tagctgttgc    480
   ccacattatt ttctgcctct taaccattgt ttttggtacc atgaagataa agcaatggaa    540
   aaaatgggag gataaggttt tagagaagga cttcgacaca gaccaaatga tccagaagaa    600
   attcacacac gttcaagaac acgaattcat caggtcacga tttcttgggg ttggaaaagc    660
   tgatgcttcc ttgggatggg tgcaatcgtt tatgaaacag tttcttgcgt cagtcaatga    720
   atcagattac atcacaatga ggctaggctt tgtcacgact cattgcaaga ccaacccaaa    780
   attcaatttc cataagtatt taatgcgtgc ccttaattct gatttcaaga aagtcgtcgg    840
   tatcagttgg tacctttggg tatttgtggt tctcttcttg cttctaaaca ttgttgcatg    900
   gcatgtttac ttctggctag cttttattcc tttgatcctt ttacttgccg tggggacgaa    960
   gttagagcat atcatcacag atttggctca tgaagttgca gagaaacaca ttgctgtgga   1020
   aggagatttg gttgtccgac catcagatga tttgttctgg tttcaaagtc cccggctagt   1080
   tctcttcttg atccatttca ttcttttcca aaactccttc gaaatcgcct acttcttctt   1140
   tatccttttt caatttggtt gggattcatg catcatggat catgtcaagt tcgtaattcc   1200
   aagactcgtc atcggggtaa taattcagct tctctgcagt tacagtacct taccgctcta   1260
   tgcactcgta actcagatgg gaagttcatt caaaggtgca atattcaacg agcagacaca   1320
   ggaacatctt gttggatggg caaaaatggc taaaagggga gtgaagaaag gcgcaacaca   1380
   agtaggtacc agtcatgatg ctactagtcc aagaccgtct attcagttga actccttgct   1440
   aggaaaaggc tcctctcaac aaaatcagaa ccccaaggaa aaatcagaga ttgctcacca   1500
   tgattaacaa acaaaaactt gttctgacat tttttttgtt gttgtgattt ttgatttttt     1560
   gaacttgttc gactttgttt ggtatatata actgattgaa aggcgttgta acaaataaac   1620
   acagcaaaga tgctgaatc                                                 1639
<210> 48
   <211> 497
   <212> PRT
   <213> Arabidopsis thaliana
<400> 48
   Met Ala Gly Gly Gly Thr Thr Leu Glu Tyr Thr Pro Thr Trp Val Val
   1               5                   10                  15
   Ala Leu Val Cys Ser Val Ile Val Ser Ile Ser Phe Ala Val Glu Arg
   20 25 30
   Leu Ile His Arg Ala Gly Lys His Phe Lys Asn Asn Asp Gln Lys Gln
   35 40 45
   Leu Phe Gly Ala Leu Gln Lys Ile Lys Glu Glu Leu Met Leu Val Gly
   50 55 60
   Phe Ile Ser Leu Leu Leu Ser Val Gly Gln Ser Lys Ile Ala Lys Ile
   65 70 75 80
   Cys Ile Ser Lys Glu Leu Ser Glu Lys Phe Leu Pro Cys Thr Lys Pro
   85 90 95
   Ala Gly Ala Glu Lys Ser Leu Lys Asp Ser Ser His Phe Gln Phe Ser
   100 105 110
   Phe Thr Gly Arg His Leu Leu Ala Gly Asp Ala Pro Ala Gly Asp Tyr
   115 120 125
   Cys Ser Leu Lys Gly Lys Val Pro Ile Met Ser Leu Ser Ala Leu His
   130 135 140
   Glu Leu His Ile Phe Ile Phe Val Leu Ala Val Ala His Ile Ile Phe
   145 150 155 160
   Cys Leu Leu Thr Ile Val Phe Gly Thr Met Lys Ile Lys Gln Trp Lys
   165 170 175
   Lys Trp Glu Asp Lys Val Leu Glu Lys Asp Phe Asp Thr Asp Gln Met
   180 185 190
   Ile Gln Lys Lys Phe Thr His Val Gln Glu His Glu Phe Ile Arg Ser
   195 200 205
   Arg Phe Leu Gly Val Gly Lys Ala Asp Ala Ser Leu Gly Trp Val Gln
   210 215 220
   Ser Phe Met Lys Gln Phe Leu Ala Ser Val Asn Glu Ser Asp Tyr Ile
   225 230 235 240
   Thr Met Arg Leu Gly Phe Val Thr Thr His Cys Lys Thr Asn Pro Lys
   245 250 255
   Phe Asn Phe His Lys Tyr Leu Met Arg Ala Leu Asn Ser Asp Phe Lys
   260 265 270
   Lys Val Val Gly Ile Ser Trp Tyr Leu Trp Val Phe Val Val Leu Phe
   275 280 285
   Leu Leu Leu Asn Ile Val Ala Trp His Val Tyr Phe Trp Leu Ala Phe
       290                 295                 300
   Ile Pro Leu Ile Leu Leu Leu Ala Val Gly Thr Lys Leu Glu His Ile
   305                 310                 315                 320
   Ile Thr Asp Leu Ala His Glu Val Ala Glu Lys His Ile Ala Val Glu
                   325                 330                 335
   Gly Asp Leu Val Val Arg Pro Ser Asp Asp Leu Phe Trp Phe Gln Ser
               340                 345                 350
   Pro Arg Leu Val Leu Phe Leu Ile His Phe Ile Leu Phe Gln Asn Ser
           355                 360                 365
   Phe Glu Ile Ala Tyr Phe Phe Phe Ile Leu Phe Gln Phe Gly Trp Asp
       370                 375                 380
   Ser Cys Ile Met Asp His Val Lys Phe Val Ile Pro Arg Leu Val Ile
   385                 390                 395                 400
   Gly Val Ile Ile Gln Leu Leu Cys Ser Tyr Ser Thr Leu Pro Leu Tyr
                   405                 410                 415
   Ala Leu Val Thr Gln Met Gly Ser Ser Phe Lys Gly Ala Ile Phe Asn
               420                 425                 430
   Glu Gln Thr Gln Glu His Leu Val Gly Trp Ala Lys Met Ala Lys Arg
           435                 440                 445
   Gly Val Lys Lys Gly Ala Thr Gln Val Gly Thr Ser His Asp Ala Thr
       450                 455                 460
   Ser Pro Arg Pro Ser Ile Gln Leu Asn Ser Leu Leu Gly Lys Gly Ser
   465                 470                 475                 480
   Ser Gln Gln Asn Gln Asn Pro Lys Glu Lys Ser Glu Ile Ala His His
                   485                 490                 495
   Asp